# EUROPEAN PATENT APPLICATION

(11) **EP 2 030 635 A1**
(43) Date of publication of application: **04.03.2009**
(21) Application number: 07743870.3
(22) Date of filing: 22.05.2007
(51) Int. Cl.: A61K 51/00, C07D 213/61, C07D 233/64, C07D 277/24, C07D 307/42, C07D 333/28, C07D 333/36, C07D 409/06

(54) **COMPOSITION FOR DIAGNOSING AMYLOID-RELATED DISEASE**

(30) Priority: 24.05.2006 JP 2006144024; 27.03.2007 JP 2007081637
(71) Applicant: Nagasaki University, Nagasaki-Shi Nagasaki , 852-8521 (JP)
(72) Inventor: NAKAYAMA, Morio, Nagasaki-shi, Nagasaki 852-8521 (JP); HARATAKE, Mamoru, Nagasaki-shi, Nagasaki 852-8521 (JP); ONO, Masahiro, Nagasaki-shi, Nagasaki 852-8521 (JP)
(74) Representative: Spaltmann, Frank
(86) International application number: PCT/JP2007/060436
(87) International publication number: WO 2007/136059

(57) **Abstract**

There is provided a composition comprising a compound represented by general formula (I), wherein R¹ represents a 5-iodothiophen-2-yl group or the like, and R² represents a 4-dimethylaminophenyl group or the like. This composition is useful for diagnosis of an amyloid-related disease such as Alzheimer's disease because the compound has high binding specificity to amyloid β protein, high permeability through the blood-brain barrier, and a property of being rapidly eliminated from sites other than senile plaques in the brain.

## Description

### Technical Field

The present invention relates to a composition used for diagnosis of an amyloid-related disease such as Alzheimer's disease, a method for screening for a therapeutic or prophylactic agent for an amyloid-related disease using the above-mentioned composition, and a method for evaluating a therapeutic or prophylactic agent for an amyloid-related disease using the above-mentioned composition.

### Background Art

With the rapid aging of population in recent years, increases in the number of patients with diseases associated with dementia including Alzheimer's disease (AD) have become one of serious social problems. Currently, the Hasegawa Scale, ADAS, and MMSE are available as methods for clinical diagnosis of AD. All of these methods quantitatively evaluate the decreased cognitive function in an individual suspected of AD and are commonly used. In addition, image diagnosis methods (MRI, CT, etc.) are supplementarily used. However, these diagnostic methods are inadequate for definite diagnosis of AD, and development of senile plaques and neurofibrils needs to be confirmed in biopsy of the brain before death or histopathological examination of the brain after death to make definite diagnosis. Accordingly, it is difficult to diagnose AD at an early stage before occurrence of an extensive brain damage by the current diagnostic methods. Although some reports have shown biological diagnostic markers for AD so far, no clinically practical technique has yet been developed. Under such circumstances, social demand for early diagnosis of AD is high, and urgent development of a method therefor is eagerly anticipated.

A senile plaque is the most characteristic brain lesion of AD, and the major component thereof is amyloid β protein having a β-sheet structure. Imaging of a senile plaque from out of the body is considered to lead to the establishment of an effective diagnostic method of AD, and such imaging requires a probe compound that specifically binds to amyloid β protein. So far, several derivatives containing congo red or thioflavine T as a parent structure have been reported as probe compounds (Patent Documents 1 and 2 and Non-Patent Document 1), but these compounds suffer from many problems including low specificity to amyloid β protein, low permeability through the blood-brain barrier, and slow clearance due to their nonspecific binding in the brain. Therefore, these reported compounds have not yet been put into practical use for diagnosis of diseases associated with accumulation of amyloid at present.
Patent Document 1: Japanese Patent Laid-Open No. 2004-250407
Patent Document 2: Japanese Patent Laid-Open No. 2004-250411
Non-Patent Document 1: Klunk W.E. et al., Annals of Neurology Vol. 55, No. 3, March 2004, 306-319

### Disclosure of the Invention

### Problems to be Solved by the Invention

The present invention was accomplished against the technical background described above, and an object thereof is to provide a compound showing high binding specificity to amyloid β protein, high permeability through the blood-brain barrier, and rapid clearance from sites other than senile plaques in the brain in combination.

### Means for Solving the Problems

The inventors of the present invention conducted various researches to achieve the foregoing object. As a result, they found that a chalcone derivative having an aromatic heterocyclic ring has an excellent property as a probe compound for imaging of amyloid β protein, and accomplished the present invention based on this finding.

Specifically, the present invention provides the following (1) to (14).
(1) A composition for diagnosis of an amyloid-related disease, comprising a compound represented by general formula (I): 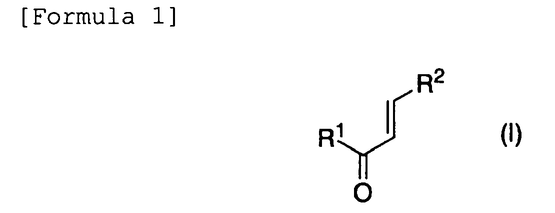 wherein R¹ represents an aromatic heterocyclic ring that may be substituted with one or more substituents selected from the following substituent group A, R² represents an aryl group that may be substituted with one or more substituents selected from the following substituent group A, or an aromatic heterocyclic ring that may be substituted with one or more substituents selected from the following substituent group A, and the substituent group A is a group consisting of a halogen atom, a hydroxyl group, a carboxyl group, a sulfone group, a dimethylamino group, a methylamino group, an amino group, a nitro group, an alkyl group having 1 to 4 carbon atoms that may be substituted with a halogen atom, an alkoxy group having 1 to 4 carbon atoms that may be substituted with a halogen atom, an alkoxy group having 2 to 8 carbon atoms that may be substituted with a halogen atom, an alkoxy group having 3 to 12 carbon atoms that may be substituted with a halogen atom, and a group represented by the formula: -(CH₂CH₂O)ₙ-F, wherein n is an integer of 4 to 10; or the compound labeled with a radionuclide, or a pharmaceutically acceptable salt thereof.
(2) The composition for diagnosis of an amyloid-related disease according to (1), wherein R¹ in the general formula (I) represents a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or a pyridin-2-yl group.
(3) The composition for diagnosis of an amyloid-related disease according to (1) or (2), wherein R² in the general formula (I) represents a 4-aminophenyl group, a 4-methylaminophenyl group, a 4-dimethylaminophenyl group, a 4-hydroxyphenyl group, a 4-methoxyphenyl group, a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or a pyridin-2-yl group.
(4) The composition for diagnosis of an amyloid-related disease according to (1), wherein R¹ in the general formula (I) represents a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group, and R² in the general formula (I) represents a 5-aminothiophen-2-yl group, a 5 methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 4-aminophenyl group, a 4-methylaminophenyl group, a 4-dimethylaminophenyl group, a 4-hydroxyphenyl group, a 4-methoxyphenyl group, a pyridin-4-yl group, a pyridin-3-yl group, or a pyridin-2-yl group.
(5) The composition for diagnosis of an amyloid-related disease according to (1), wherein R¹ in the general formula (I) represents a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a pyridin-4-yl group, a pyridin-3-yl group, or a pyridin-2-yl group, and R² in the general formula (I) represents a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group.
(6) The composition for diagnosis of an amyloid-related disease according to (1), wherein R¹ in the general formula (I) represents a 6-hydroxypyridin-3-yl group, a 5-hydroxythiophen-2-yl group, a 5-hydroxyfuran-2-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group, and R² in the general formula (I) represents a 5-methylaminothiophen-2-yl group, a 5-dimethylaminothiophen-2-yl group, a 5-methylaminofuran-2-yl group, a 5-dimethylaminofuran-2-yl group, a 4-methylaminophenyl group, or a 4-dimethylaminophenyl group.
(7) The composition for diagnosis of an amyloid-related disease according to (1), wherein R¹ in the general formula (I) represents a 5-methylaminothiophen-2-yl group, a 5-dimethylaminothiophen-2-yl group, a 5-methylaminofuran-2-yl group, or a 5-dimethylaminofuran-2-yl group, and R² in the general formula (I) represents a 6-hydroxypyridin-3-yl group, a 5-hydroxythiophen-2-yl group, or a 5-hydroxyfuran-2-yl group.
(8) The composition for diagnosis of an amyloid-related disease according to (1), wherein R¹ in the general formula (I) represents a thienyl group in which one hydrogen atom is substituted with a halogen atom, and R² in the general formula (I) represents a phenyl group in which one hydrogen atom is substituted with a dimethylamino group or a methylamino group.
(9) The composition for diagnosis of an amyloid-related disease according to (1), wherein R¹ in the general formula (I) represents a 5-iodothiophen-2-yl group, and R² in the general formula (I) represents a 4-dimethylaminophenyl group or a 4-methylaminophenyl group.
(10) The composition for diagnosis of an amyloid-related disease according to any one of (1) to (9), wherein the radionuclide is a positron-emitting radionuclide.
(11) The composition for diagnosis of an amyloid-related disease according to any one of (1) to (9), wherein the radionuclide is a γ-ray-emitting radionuclide.
(12) The composition for diagnosis of an amyloid-related disease according to any one of (1) to (11), wherein the amyloid-related disease is Alzheimer's disease.
(13) A method for screening for a therapeutic or prophylactic agent for an amyloid-related disease, comprising the steps of administering a test substance to an amyloid-related disease model animal, administering the composition for diagnosis of an amyloid-related disease according to any one of (1) to (12) to the model animal, and examining a distribution or a quantity of the compound represented by the general formula (I) contained in the brain of the model animal.
(14) A method for evaluating a therapeutic or prophylactic agent for an amyloid-related disease, comprising the steps of administering the therapeutic or prophylactic agent for an amyloid-related disease to an amyloid-related disease model animal, administering the composition for diagnosis of an amyloid-related disease according to any one of (1) to (12) to the model animal, and examining a distribution or a quantity of the compound represented by the general formula (I) contained in the brain of the model animal.

### Advantages of the Invention

The compound represented by the general formula (I) is useful for diagnosis of Alzheimer's disease because the compound has high binding specificity to amyloid β protein, permeability through the blood-brain barrier, and a property of being rapidly eliminated from sites other than senile plaques in the brain.

### Brief Description of the Drawings

Figure 1 shows a method for synthesizing aromatic heterocyclic ring-containing chalcone derivatives used in Example 1 (numbers in the figure correspond to the compound numbers in Example 1);
Figure 2 shows binding properties of Compounds 1 and 4 in Example 1 to the Aβ(1-42) aggregate (■, Compound 1; ▲, Compound 4);
Figure 3 shows a method for synthesizing chalcone derivatives used in Example 2 (numbers in the figure correspond to the compound numbers in Example 2);
Figure 4 shows a method for synthesizing chalcone derivatives used in Example 2 (numbers in the figure correspond to the compound numbers in Example 2);
Figure 5 shows a method for synthesizing chalcone derivatives used in Example 2 (numbers in the figure correspond to the compound numbers in Example 2);
Figure 6 shows a method for synthesizing chalcone derivatives used in Example 2 (numbers in the figure correspond to the compound numbers in Example 2);
Figure 7 shows a method for synthesizing chalcone derivatives used in Example 2 (numbers in the figure correspond to the compound numbers in Example 2);
Figure 8 shows a method for synthesizing chalcone derivatives used in Example 2 (numbers in the figure correspond to the compound numbers in Example 2);
Figure 9 shows a method for labeling chalcone derivatives used in Example 2 with a radioactive iodine;
Figure 10 shows inhibition curves of Compound 14 in Example 2, congo red, and thioflavine T of binding of [¹²⁵I]CL-NMe2 to the Aβ (1-42) aggregate (•, Compound 14 in Example 2; ■, congo red; ▲, thioflavine T);
Figure 11 shows results of a fluorescence staining experiment using brain tissue sections of Alzheimer's disease model mice (Compound 14 in Example 2, A and B; Compound 23 in Reference Example 2, C and D; Compound 3 in Reference Example 2, E and F; Compound 5 in Reference Example 2, G and H; Compound 7 in Reference Example 2, I and J; senile plaque amyloid, A, C, E, G, and I; vascular amyloid, B, D, F, H, and J);
Figure 12 shows a method for synthesizing chalcone derivatives used in Reference Example 1 (numbers in the figure correspond to the compound numbers in Reference Example 1);
Figure 13 shows a method for labeling chalcone derivatives used in Example Reference Example 1 with a radioactive iodine;
Figure 14 shows binding properties of Compound 7 (A) and Compound 12 (B) in Reference Example 1 to the Aβ aggregate (•, in the presence of the Aβ aggregate; ■, in the absence of the Aβ aggregate);
Figure 15 shows a method for synthesizing fluorine-containing chalcone derivatives used in Reference Example 2 (numbers in the figure correspond to the compound numbers in Reference Example 2);
Figure 16 shows a method for synthesizing fluorine-containing chalcone derivatives used in Reference Example 2 (numbers in the figure correspond to the compound numbers in Reference Example 2);
Figure 17 shows a method for synthesizing fluorine-containing chalcone derivatives used in Reference Example 2 (numbers in the figure correspond to the compound numbers in Reference Example 2); and
Figure 18 shows a method for labeling fluorine-containing chalcone derivatives used in Example Reference Example 2 with a radioactive carbon.

### Best Mode for Carrying Out the Invention

Hereafter, the present invention will be explained in detail.

In the present invention, "halogen atom" means, for example, a fluorine atom, a chlorine atom, a bromine atom, or an iodine atom.

In the present invention, "an alkyl group having 1 to 4 carbon atoms" means, for example, a methyl group, an ethyl group, a propyl group, an isopropyl group, a butyl group, an isobutyl group, a sec-butyl group, a tert-butyl group, or the like.

In the present invention, "an alkyl group having 1 to 4 carbon atoms that may be substituted with a halogen atom" means, for example, a fluoromethyl group, a 2-fluoroethyl group, a 3-fluoropropyl group, a 4-fluorobutyl group, or the like.

In the present invention, "an alkoxy group having 1 to 4 carbon atoms" means, for example, a methoxy group, an ethoxy group, a propoxy group, an isopropoxy group, a butoxy group, an isobutoxy group, a sec-butoxy group, a tert-butoxy group, or the like.

In the present invention, "an alkoxy group having 2 to 8 carbon atoms" means, for example, a 2-ethoxyethoxy group or the like.

In the present invention, "an alkoxy group having 3 to 12 carbon atoms" means, for example, a 2-(2-ethoxyethoxy)ethoxy group or the like.

In the present invention, "an alkoxy group having 1 to 4 carbon atoms that may be substituted with a halogen atom" means, for example, a fluoromethoxy group, a 2-fluoroethoxy group, a 3-fluoropropoxy group, a 4-fluorobutoxy group, or the like.

In the present invention, "an alkoxy group having 2 to 8 carbon atoms that may be substituted with a halogen atom" means, for example, a 2-(2-fluoroethoxy)ethoxy group or the like.

In the present invention, "an alkoxy group having 3 to 12 carbon atoms that may be substituted with a halogen atom" means, for example, a 2-[2-(2-fluoroethoxy)ethoxy]ethoxy group or the like.

In the present invention, "aryl group" means, for example, a phenyl group, a 1-naphthyl group, a 2-naphthyl group, or the like.

In the present invention, "an aromatic heterocyclic ring" means, for example, a pyridin-2-yl group, a pyridin-3-yl group, a pyridin-4-yl group, a pyrimidin-2-yl group, a pyrimidin-4-yl group, a pyrimidin-5-yl group, a thiophen-2-yl group, a thiophen-3-yl group, a furan-2-yl group, a furan-3-yl group, a thiazol-2-yl group, a 1H-imidazol-2-yl group, a 1H-imidazol-5-yl group, or the like.

In the present invention, "an aryl group that may be substituted with a substituent selected from the substituent group A" means, for example, a 2-dimethylaminophenyl group, a 2-methylaminophenyl group, a 2-aminophenyl group, a 2-methoxyphenyl group, a 2-hydroxyphenyl group, a 3-dimethylaminophenyl group, a 3-methylaminophenyl group, a 3-aminophenyl group, a 3-methoxyphenyl group, a 3-hydroxyphenyl group, a 4-dimethylaminophenyl group, a 4-methylaminophenyl group, a 4-aminophenyl group, a 4-methoxyphenyl group, a 4-hydroxyphenyl group, a 3-hydroxy-4-dimethylaminophenyl group, a 3-hydroxy-4-methylaminophenyl group, a 3-hydroxy-4-methoxyphenyl group, a 3,5-dihydroxy-4-dimethylaminophenyl group, a 3,5-dihydroxy-4-methylaminophenyl group, a 3,5-dihydroxy-4-methoxyphenyl group, or the like.

In the present invention, "an aromatic heterocyclic ring that may be substituted with a substituent selected from the substituent group A" means, for example, a 6-iodopyridin-3-yl group, a 6-fluoropyridin-3-yl group, a 6-hydroxypyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodopyridin-2-yl group, a 5-fluoropyridin-2-yl group, a 5-hydroxypyridin-2-yl group, a 5-(2-fluoroethyl)pyridin-2-yl group, a 5-(2-fluoroethoxy)pyridin-2-yl group, a 5-[2-(2-fluoroethoxy)ethoxy]pyridin-2-yl group, a 5-{2-[2-(2-fluoroethoxy)ethoxy]ethoxy}pyridin-2-yl group, a 5-iodopyridin-3-yl group, a 5-fluoropyridin-3-yl group, a 5-hydroxypyridin-3-yl group, a 5-(2-fluoroethyl)pyridin-3-yl group, a 5-(2-fluoroethoxy)pyridin-3-yl group, a 5-[2-(2-fluoroethoxy)ethoxy]pyridin-3-yl group, a 5-{2-[2-(2-fluoroethoxy)ethoxy]ethoxy}pyridin-3-yl group, a 4-iodopyridin-2-yl group, a 4-fluoropyridin-2-yl group, a 4-hydroxypyridin-2-yl group, a 4-(2-fluoroethyl)pyridin-2-yl group, a 4-(2-fluoroethoxy)pyridin-2-yl group, a 4-[2-(2-fluoroethoxy)ethoxy]pyridin-2-yl group, a 4-{2-[2-(2-fluoroethoxy)ethoxy]ethoxy}pyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 5-dimethylaminopyridin-2-yl group, a 5-methylaminopyridin-2-yl group, a 5-methoxypyridin-2-yl group, a 6-dimethylaminopyridin-3-yl group, a 6-methylaminopyridin-3-yl group, a 6-methoxypyridin-3-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, or the like.

In the general formula (I), R¹ is preferably a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or pyridin-2-yl group, more preferably, a 6-iodopyridin-3-yl group, a 5-iodothiophen-2-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethoxy)thiophen-2-yl group, 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethoxy)furan-2-yl group, 5-[2-(2-fluoroethoxy)ethoxy]furan-2-yl group, or a 5-{2-[2-(2-fluoroethoxy)ethoxy]ethoxy}furan-2-yl group.

In the general formula (I), R² is preferably a 4-aminophenyl group, 4-methylaminophenyl group, a 4-dimethylaminophenyl group, a 4-hydroxyphenyl group, a 4-methoxyphenyl group, a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or a pyridin-2-yl group, more preferably, a 4-methylaminophenyl group or a 4-dimethylaminophenyl group.

Furthermore, preferred examples of combinations of R¹ and R² include the following (A) to (F).
(A) R¹ is a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group, and R² is a 5-aminothiophen-2-yl group, a 5 methylaminothiophen-2-yl group, a 5-dimethylaminothiophen-2-yl group, a 5-aminofuran-2-yl group, a 5 methylaminofuran-2-yl group, a 5-dimethylaminofuran-2-yl group, a thiazol-2-yl group, a 1H-imidazol-5-yl group, a 1H-imidazol-2-yl group, a 4-aminophenyl group, a 4-methylaminophenyl group, a 4-dimethylaminophenyl group, a 4-hydroxyphenyl group, a 4-methoxyphenyl group, a pyridin-4-yl group, a pyridin-3-yl group, or a pyridin-2-yl group.
(B) R¹ is a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a pyridin-4-yl group, a pyridin-3-yl group, or a pyridin-2-yl group, and R² is a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group.
(C) R¹ is a 6-hydroxypyridin-3-yl group, a 5-hydroxythiophen-2-yl group, or a 5-hydroxyfuran-2-yl group, and R² is a 5-methylaminothiophen-2-yl group, a 5-dimethylaminothiophen-2-yl group, a 5-methylaminofuran-2-yl group, a 5-dimethylaminofuran-2-yl group, a 4-methylaminophenyl group, or a 4-dimethylaminophenyl group.
(D) R¹ is a 5-methylaminothiophen-2-yl group, a 5-dimethylaminothiophen-2-yl group, a 5-methylaminofuran-2-yl group, or a 5-dimethylaminofuran-2-yl group, and R² is a 6-hydroxypyridin-3-yl group, a 5-hydroxythiophen-2-yl group, or a 5-hydroxyfuran-2-yl group.
(E) R¹ is a thienyl group in which one hydrogen atom is substituted with a halogen atom, and R² is a phenyl group in which one hydrogen atom is substituted with a dimethylamino group or a methylamino group.
(F) R¹ is a 5-iodothiophen-2-yl group, and R² is a 4-dimethylaminophenyl group or a 4-methylaminophenyl group.

Common compounds represented by the general formula (I) are listed in Tables 1 to 11.

**[Table 1]**

| Number | R¹ | R² |
|---|---|---|
| I-1 | 6-Iodopyridin-3-yl group | 5-Aminothiophen-2-yl group |
| I-2 | 6-Iodopyridin-3-yl group | 5-Methylaminothiophen-2-yl group |
| I-3 | 6-Iodopyridin-3-yl group | 5-Dimethylaminothiophen-2-yl group |
| I-4 | 6-Iodopyridin-3-yl group | 5-Aminofuran-2-yl group |
| I-5 | 6-Iodopyridin-3-yl group | 5-Methylaminofuran-2-yl group |
| I-6 | 6-Iodopyridin-3-yl group | 5-Dimethylaminofuran-2-yl group |
| I-7 | 6-Iodopyridin-3-yl group | Thiazol-2-yl group |
| I-8 | 6-Iodopyridin-3-yl group | 1H-Imidazol-5-yl group |
| I-9 | 6-Iodopyridin-3-yl group | 1H-Imidazol-2-yl group |
| I-10 | 6-Iodopyridin-3-yl group | 4-Aminophenyl group |
| I-11 | 6-Iodopyridin-3-yl group | 4-Methylaminophenyl group |
| I-12 | 6-Iodopyridin-3-yl group | 4-Dimethylaminophenyl group |
| I-13 | 6-Iodopyridin-3-yl group | 4-Hydroxyphenyl group |
| I-14 | 6-Iodopyridin-3-yl group | 4-Methoxyphenyl group |
| I-15 | 6-Iodopyridin-3-yl group | Pyridin-4-yl group |
| I-16 | 6-Iodopyridin-3-yl group | Pyridin-3-yl group |
| I-17 | 6-Iodopyridin-3-yl group | Pyridin-2-yl group |
| I-18 | 6-Fluoropyridin-3-yl group | 5-Aminothiophen-2-yl group |
| I-19 | 6-Fluoropyridin-3-yl group | 5-Methylaminothiophen-2-yl group |
| I-20 | 6-Fluoropyridin-3-yl group | 5-Dimethylaminothiophen-2-yl group |
| I-21 | 6-Fluoropyridin-3-yl group | 5-Aminofuran-2-yl group |
| I-22 | 6-Fluoropyridin-3-yl group | 5-Methylaminofuran-2-yl group |
| I-23 | 6-Fluoropyridin-3-yl group | 5-Dimethylaminofuran-2-yl group |
| I-24 | 6-Fluoropyridin-3-yl group | Thiazol-2-yl group |
| I-25 | 6-Fluoropyridin-3-yl group | 1H-Imidazol-5-yl group |
| I-26 | 6-Fluoropyridin-3-yl group | 1H-Imidazol-2-yl group |
| I-27 | 6-Fluoropyridin-3-yl group | 4-Aminophenyl group |
| I-28 | 6-Fluoropyridin-3-yl group | 4-Methylaminophenyl group |
| I-29 | 6-Fluoropyridin-3-yl group | 4-Dimethylaminophenyl group |
| I-30 | 6-Fluoropyridin-3-yl group | 4-Hydroxyphenyl group |
| I-31 | 6-Fluoropyridin-3-yl group | 4-Methoxyphenyl group |
| I-32 | 6-Fluoropyridin-3-yl group | Pyridin-4-yl group |
| I-33 | 6-Fluoropyridin-3-yl group | Pyridin-3-yl group |
| I-34 | 6-Fluoropyridin-3-yl group | Pyridin-2-yl group |
| I-35 | 6-(2-Fluoroethyl)pyridin-3-yl group | 5-Aminothiophen-2-yl group |
| I-36 | 6-(2-Fluoroethyl)pyridin-3-yl group | 5-Methylaminothiophen-2-yl group |
| I-37 | 6-(2-Fluoroethyl)pyridin-3-yl group | 5-Dimethylaminothiophen-2-yl group |
| I-38 | 6-(2-Fluoroethyl)pyridin-3-yl group | 5-Aminofuran-2-yl group |
| I-39 | 6-(2-Fluoroethyl)pyridin-3-yl group | 5-Methylaminofuran-2-yl group |
| I-40 | 6-(2-Fluoroethyl)pyridin-3-yl group | 5-Dimethylaminofuran-2-yl group |
| I-41 | 6-(2-Fluoroethyl)pyridin-3-yl group | Thiazol-2-yl group |
| I-42 | 6-(2-Fluoroethyl)pyridin-3-yl group | 1H-Imidazol-5-yl group |
| I-43 | 6-(2-Fluoroethyl)pyridin-3-yl group | 1H-Imidazol-2-yl group |
| I-44 | 6-(2-Fluoroethyl)pyridin-3-yl group | 4-Aminophenyl group |
| I-45 | 6-(2-Fluoroethyl)pyridin-3-yl group | 4-Methylaminophenyl group |
| I-46 | 6-(2-Fluoroethyl)pyridin-3-yl group | 4-Dimethylaminophenyl group |
| I-47 | 6-(2-Fluoroethyl)pyridin-3-yl group | 4-Hydroxyphenyl group |
| I-48 | 6-(2-Fluoroethyl)pyridin-3-yl group | 4-Methoxyphenyl group |
| I-49 | 6-(2-Fluoroethyl)pyridin-3-yl group | Pyridin-4-yl group |
| I-50 | 6-(2-Fluoroethyl)pyridin-3-yl group | Pyridin-3-yl group |
| I-51 | 6-(2-Fluoroethyl)pyridin-3-yl group | Pyridin-2-yl group |

**[Table 2]**

| Number | R¹ | R² |
|---|---|---|
| I-52 | 6-(2-Fluoroethoxy)pyridin-3-yl group | 5-Aminothiophen-2-yl group |
| I-53 | 6-(2-Fluoroethoxy)pyridin-3-yl group | 5-Methylaminothiophen-2-yl group |
| I-54 | 6-(2-Fluoroethoxy)pyridin-3-yl group | 5-Dimethylaminothiophen-2-yl group |
| I-55 | 6-(2-Fluoroethoxy)pyridin-3-yl group | 5-Aminofuran-2-yl group |
| I-56 | 6-(2-Fluoroethoxy)pyridin-3-yl group | 5-Methylaminofuran-2-yl group |
| I-57 | 6-(2-Fluoroethoxy)pyridin-3-yl group | 5-Dimethylaminofuran-2-yl group |
| I-58 | 6-(2-Fluoroethoxy)pyridin-3-yl group | Thiazol-2-yl group |
| I-59 | 6-(2-Fluoroethoxy)pyridin-3-yl group | 1H-Imidazol-5-yl group |
| I-60 | 6-(2-Fluoroethoxy)pyridin-3-yl group | 1H-Imidazol-2-yl group |
| I-61 | 6-(2-Fluoroethoxy)pyridin-3-yl group | 4-Aminophenyl group |
| I-62 | 6-(2-Fluoroethoxy)pyridin-3-yl group | 4-Methylaminophenyl group |
| I-63 | 6-(2-Fluoroethoxy)pyridin-3-yl group | 4-Dimethylaminophenyl group |
| I-64 | 6-(2-Fluoroethoxy)pyridin-3-yl group | 4-Hydroxyphenyl group |
| I-65 | 6-(2-Fluoroethoxy)pyridin-3-yl group | 4-Methoxyphenyl group |
| I-66 | 6-(2-Fluoroethoxy)pyridin-3-yl group | Pyridin-4-yl group |
| I-67 | 6-(2-Fluoroethoxy)pyridin-3-yl group | Pyridin-3-yl group |
| I-68 | 6-(2-Fluoroethoxy)pyridin-3-yl group | Pyridin-2-yl group |
| I-69 | 5-Iodothiophen-2-yl group | 5-Aminothiophen-2-yl group |
| I-70 | 5-Iodothiophen-2-yl group | 5-Methylaminothiophen-2-yl group |
| I-71 | 5-Iodothiophen-2-yl group | 5-Dimethylaminothiophen-2-yl group |
| I-72 | 5-Iodothiophen-2-yl group | 5-Aminofuran-2-yl group |
| I-73 | 5-Iodothiophen-2-yl group | 5-Methylaminofuran-2-yl group |
| I-74 | 5-Iodothiophen-2-yl group | 5-Dimethylaminfuran-2-yl group |
| I-75 | 5-Iodothiophen-2-yl group | Thiazol-2-yl group |
| I-76 | 5-Iodothiophen-2-yl group | 1H-Imidazol-5-yl group |
| I-77 | 5-Iodothiophen-2-yl group | 1H-Imidazol-2-yl group |
| I-78 | 5-Iodothiophen-2-yl group | 4-Aminophenyl group |
| I-79 | 5-Iodothiophen-2-yl group | 4-Methylaminophenyl group |
| I-80 | 5-Iodothiophen-2-yl group | 4-Dimethylaminophenyl group |
| I-81 | 5-Iodothiophen-2-yl group | 4-Hydroxyphenyl group |
| I-82 | 5-Iodothiophen-2-yl group | 4-Methoxyphenyl group |
| I-83 | 5-Iodothiophen-2-yl group | Pyridin-4-yl group |
| I-84 | 5-Iodothiophen-2-yl group | Pyridin-3-yl group |
| I-85 | 5-Iodothiophen-2-yl group | Pyridin-2-yl group |
| I-86 | 5-Fluorothiophen-2-yl group | 5-Aminothiophen-2-yl group |
| I-87 | 5-Fluorothiophen-2-yl group | 5-Methylaminothiophen-2-yl group |
| I-88 | 5-Fluorothiophen-2-yl group | 5-Dimethylaminothiophen-2-yl group |
| I-89 | 5-Fluorothiophen-2-yl group | 5-Aminofuran-2-yl group |
| I-90 | 5-Fluorothiophen-2-yl group | 5-Mtethylaminofuran-2-yl group |
| I-91 | 5-Fluorothiophen-2-yl group | 5-Dimethylaminofuran-2-y group |
| I-92 | 5-Fluorothiophen-2-yl group | Thiazol-2-yl group |
| I-93 | 5-Fluorothiophen-2-yl group | 1H-Imidazol-5-yl group |
| I-94 | 5-Fluorothiophen-2-yl group | 1H-Imidazol-2-yl group |
| I-95 | 5-Fluorothiophen-2-yl group | 4-Aminophenyl group |
| I-96 | 5-Fluorothiophen-2-yl group | 4-Methylaminophenyl group |
| I-97 | 5-Fluorothiophen-2-yl group | 4-Dimthylaminophenyl group |
| I-98 | 5-Fluorothiophen-2-yl group | 4-Hydroxyphenyl group |
| I-99 | 5-Fluorothiophen-2-yl group | 4-Methoxyphenyl group |
| I-100 | 5-Fluorothiophen-2-yl group | Pyridin-4-yl group |
| I-101 | 5-Fluorothiophen-2-yl group | Pyridin-3-yl group |
| I-102 | 5-Fluorothiophen-2-yl group | Pyridin-2-yl group |

**[Table 3]**

| Number | R¹ | R² |
|---|---|---|
| I-103 | 5-(2-Fluoroethyl)thiophen-2-yl group | 5-Aminothiophen-2-yl group |
| I-104 | 5-(2-Fluoroethyl)thiophen-2-yl group | 5-Methylaminothiophen-2-yl group |
| I-105 | 5-(2-Fluoroethyl)thiophen-2-yl group | 5-Dimethylaminothiophen-2-yl group |
| I-106 | 5-(2-Fluoroethyl)thiophen-2-yl group | 5-Aminofuran-2-yl group |
| 1-107 | 5-(2-Fluoroethyl)thiophen-2-yl group | 5-Methylaminofuran-2-yl group |
| I-108 | 5-(2-Fluoroethyl)thiophen-2-yl group | 5-Dimethylaminofuran-2-yl group |
| I-109 | 5-(2-Fluoroethyl)thiophen-2-yl group | Thiazol-2-yl group |
| I-110 | 5-(2-Fluoroethyl)thiophen-2-yl group | 1H-Imidazol-5-yl group |
| I-111 | 5-(2-Fluoroethyl)thiophen-2-yl group | 1H-Imidazol-2-yl group |
| I-112 | 5-(2-Fluoroethyl)thiophen-2-yl group | 4-Aminophenyl group |
| I-113 | 5-(2-Fluoroethyl)thiophen-2-yl group | 4-Methylaminophenyl group |
| I-114 | 5-(2-Fluoroethyl)thiophen-2-yl group | 4-Dimethylaminophenyl group |
| I-115 | 5-(2-Fluoroethyl)thiophen-2-yl group | 4-Hydroxyphenyl group |
| I-116 | 5-(2-Fluoroethyl)thiophen-2-yl group | 4-Methoxyphenyl group |
| I-117 | 5-(2-Fluoroethyl)thiophen-2-yl group | Pyridin-4-yl group |
| I-118 | 5-(2-Fluoroethyl)thiophen-2-yl group | Pyridin-3-yl group |
| I-119 | 5-(2-Fluoroethyl)thiophen-2-yl group | Pyridin-2-yl group |
| I-120 | 5-(2-Fluoroethoxy)thiophen-2-yl group | 5-Aminothiophen-2-yl group |
| I-121 | 5-(2-Fluoroethoxy)thiophen-2-yl group | 5-Methylaminothiophen-2-yl group |
| I-122 | 5-(2-Fluoroethoxy)thiophen-2-yl group | 5-Dimethylamimthiophen-2-yl group |
| I-123 | 5-(2-Fluoroethoxy)thiophen-2-yl group | 5-Aminofuran-2-yl group |
| I-124 | 5-(2-Fluoroethoxy)thiophen-2-yl group | 5-Methylaminofuran-2-yl group |
| I-125 | 5-(2-Fluoroethoxy)thiophen-2-yl group | 5-Dimethylaminofuran-2-yl group |
| I-126 | 5-(2-Fluoroethoxy)thiophen-2-yl group | Thiazol-2-yl group |
| I-127 | 5-(2-Fluoroethoxy)thiophen-2-yl group | 1H-Imidazol-5-yl group |
| I-128 | 5-(2-Fluoroethoxy)thiophen-2-yl group | 1H-Imidazol-2-yl group |
| I-129 | 5-(2-Fluoroethoxy)thiophen-2-yl group | 4-Aminophenyl group |
| I-130 | 5-(2-Fluoroethoxy)thiophen-2-yl group | 4-Methylaminophenyl group |
| I-131 | 5-(2-Fluoroethoxy)thiophen-2-yl group | 4-Dimethylaminophenyl group |
| I-132 | 5-(2-Fluoroethoxy)thiophen-2-yl group | 4-Hydroxyphenyl group |
| I-133 | 5-(2-Fluoroethoxy)thiophen-2-yl group | 4-Methoxypheyl group |
| I-134 | 5-(2-Fluoroethoxy)thiophen-2-yl group | Pyridin-4-yl group |
| I-135 | 5-(2-Fluoroethoxy)thiophen-2-yl group | Pyridin-3-yl group |
| I-136 | 5-(2-Fluoroethoxy)thiophen-2-yl group | Pyridin-2-yl group |
| I-137 | 5-Iodofuran-2-yl group | 5-Aminothiophen-2-yl group |
| I-138 | 5-Iodofuran-2-yl group | 5-Methylaminothiophen-2-yl group |
| I-139 | 5-Iodofuran-2-yl group | 5-Dimethylaminothiophen-2-yl group |
| I-140 | 5-Iodofuran-2-yl group | 5-Aminofuran-2-yl group |
| I-141 | 5-Iodofuran-2-yl group | 5-Methylaminofuran-2-yl group |
| I-142 | 5-Iodofuran-2-yl group | 5-Dimethylaminofuran-2-yl group |
| I-143 | 5-Iodofuran-2-yl group | Thiazol-2-yl group |
| I-144 | 5-Iodofuran-2-yl group | 1H-Imidazol-5-yl group |
| I-145 | 5-Iodofuran-2-yl group | 1H-Imidazol-2-yl group |
| I-146 | 5-Iodofuran-2-yl group | 4-Aminophenyl group |
| I-147 | 5-Iodofuran-2-yl group | 4-Methylaminophenyl group |
| I-148 | 5-Iodofuran-2-yl group | 4-Dimethylaminophenyl group |
| I-149 | 5-Iodofuran-2-yl group | 4-Hydroxyphenyl group |
| I-150 | 5-Iodofuran-2-yl group | 4-Methoxyphenyl group |
| I-151 | 5-Iodofuran-2-yl group | Pyridin-4-yl group |
| I-152 | 5-Iodofuran-2-yl group | Pyridin-3-yl group |
| I-153 | 5-Iodofuran-2-yl group | Pyridin-2-yl group |

**[Table 4]**

| Number | R¹ | R² |
|---|---|---|
| I-154 | 5-Fluorofuran-2-yl group | 5-Aminothiophen-2-yl group |
| I-155 | 5-Fluorofuran-2-yl group | 5-Methylaminothiophen-2-yl group |
| I-156 | 5-Fluorofuran-2-yl group | 5-Dimethylaminothiophen-2-yl group |
| I-157 | 5-Fluorofuran-2-yl group | 5-Aminofuran-2-yl group |
| I-158 | 5-Fluorofuran-2-yl group | 5-Methylaminofuran-2-yl group |
| I-159 | 5-Fluorofuran-2-yl group | 5-Dimethylaminofuran-2-yl group |
| I-160 | 5-Fluorofuran-2-yl group | Thiazol-2-yl group |
| I-161 | 5-Fluorofuran-2-yl group | 1H-Imidazol-5-yl group |
| I-162 | 5-Fluorofuran-2-yl group | 1H-Imidazol-2-yl group |
| I-163 | 5-Fluorofuran-2-yl group | 4-Aminophenyl group |
| I-164 | 5-Fluorofuran-2-yl group | 4-Methylaminophenyl group |
| I-165 | 5-Fluorofuran-2-yl group | 4-Dimethylaminophenyl group |
| I-166 | 5-Fluorofuran-2-yl group | 4-Hydroxyphenyl group |
| I-167 | 5-Fluorofuran-2-yl group | 4-Methoxyphenyl group |
| I-168 | 5-Fluorofuran-2-yl group | Pyridin-4-yl group |
| I-169 | 5-Fluorofuran-2-yl group | Pyridin-3-yl group |
| I-170 | 5-Fluorofuran-2-yl group | Pyridin-2-yl group |
| I-171 | 5-(2-Fluoroethyl)furan-2-yl group | 5-Aminothiophen-2-yl group |
| I-172 | 5-(2-Fluoroethyl)furan-2-yl group | 5-Methylaminothiophen-2-yl group |
| I-173 | 5-(2-Fluoroethyl)furan-2-yl group | 5-Dimethylaminothiophen-2-yl group |
| I-174 | 5-(2-Fluoroethyl)furan-2-yl group | 5-Aminofuran-2-yl group |
| I-175 | 5-(2-Fluoroethyl)furan-2-yl group | 5-Methylaminofuran-2-yl group |
| I-176 | 5-(2-Fluoroethyl)furan-2-yl group | 5-Dimethylaminofuran-2-yl group |
| I-177 | 5-(2-Fluoroethyl)furan-2-yl group | Thiazol-2-yl group |
| I-178 | 5-(2-Fluoroethyl)furan-2-yl group | 1H-Imidazol-5-yl group |
| I-179 | 5-(2-Fluoroethyl)furan-2-yl group | 1H-Imidazol-2-yl group |
| I-180 | 5-(2-Fluoroethyl)furan-2-yl group | 4-Aminophenyl group |
| I-181 | 5-(2-Fluoroethyl)furan-2-yl group | 4-Methylaminophenyl group |
| I-182 | 5-(2-Fluoroethyl)fruan-2-yl group | 4-Dimethylaminophenyl group |
| I-183 | 5-(2-Fluoroethyl)furan-2-yl group | 4-Hydroxyphenyl group |
| I-184 | 5-(2-Fluoroethyl)furan-2-yl group | 4-Methoxyphenyl group |
| I-185 | 5-(2-Fluoroethyl)furan-2-yl group | Pyridin-4-yl group |
| I-186 | 5-(2-Fluoroethyl)furan-2-yl group | Pyridin-3-yl group |
| I-187 | 5-(2-Fluoroethyl)furan-2-yl group | Pyridin-2-yl group |
| I-188 | 5-(2-Fluoroethoxy)furan-2-yl group | 5-Aminothiophen-2-yl group |
| I-189 | 5-(2-Fluoroethoxy)furan-2-yl group | 5-Methylaminothiophen-2-yl group |
| I-190 | 5-(2-Fluoroethoxy)furan-2-yl group | 5-Dimethylaminothiophen-2-yl group |
| I-191 | 5-(2-Fluoroethoxy)furan-2-yl group | 5-Aminofuran-2-yl group |
| I-192 | 5-(2-Fluoroethoxy)furan-2-yl group | 5-Methylaminofuran-2-yl group |
| I-193 | 5-(2-Fluoroethoxy)furan-2-yl group | 5-Dimethylaminofuran-2-yl group |
| I-194 | 5-(2-Fluoroethoxy)furan-2-yl group | Thiazol-2-yl group |
| I-195 | 5-(2-Fluoroethoxy)furan-2-yl group | 1H-Imidazol-5-yl group |
| I-196 | 5-(2-Fluoroethoxy)furan-2-yl group | 1H-Imidazol-2-yl group |
| I-197 | 5-(2-Fluoroethoxy)furan-2-yl group | 4-Aminophenyl group |
| I-198 | 5-(2-Fluoroethoxy)furan-2-yl group | 4-Methylaminophenyl group |
| I-199 | 5-(2-Fluoroethoxy)furan-2-yl group | 4-Dimethylaminophenyl group |
| I-200 | 5-(2-Fluoroethoxy)furan-2-yl group | 4-Hydroxyphenyl group |
| I-201 | 5-(2-Fluoroethoxy)furan-2-yl group | 4-Methoxyphenyl group |
| I-202 | 5-(2-Fluoroethoxy)furan-2-yl group | Pyridin-4-yl group |
| I-203 | 5-(2-Fluoroethoxy)furan-2-yl group | Pyridin-3-yl group |
| I-204 | 5-(2-Fluoroethoxy)furan-2-yl group | Pyridin-2-yl group |

**[Table 5]**

| Number | R¹ | R² |
|---|---|---|
| I-205 | 5-Amimothiorphen-2-yl group | 6-Iodopyridin-3-yl group |
| I-206 | 5-Aminothiophen-2-yl group | 6-Fluoropyridin-3-yl group |
| I-207 | 5-Aminothiophen-2-yl group | 6-(2-Fluoroethyl)pyridin-3-yl group |
| I-208 | 5-Aminothiophen-2-yl group | 6-(2-Fluoroethoxy)pyridin-3-yl group |
| I-209 | 5-Aminothiophen-2-yl group | 5-Iodothiophen-2-yl group |
| I-210 | 5-Aminothiophen-2-yl group | 5-Fluorothiophen-2-yl group |
| I-211 | 5-Aminothiophen-2-yl group | 5-(2-Fluotnethyl)thiophen-2-yl group |
| I-212 | 5-Aminothiophen-2-yl group | 5-(2-Fluoroethoxy)thiophen-2-yl group |
| I-213 | 5-Aminothiophen-2-yl group | 5-Iodofuran-2-yl group |
| I-214 | 5-Aminothiophen-2-yl group | 5-Fiuorofuran-2-yl group |
| I-215 | 5-Aminothiophen-2-yl group | 5-(2-Fluoethyl)furan-2-yl group |
| I-216 | 5-Aminothiophen-2-yl group | 5-(2-Fluoroetroxy)furan-2-yl group |
| I-217 | 5-Methylaminothiophen-2-yl group | 6-Iodopyridin-3-yl group |
| I-218 | 5-Aminothiophen-2-yl group | 6-Fluompyridin-3-yl group |
| I-219 | 5-Methylaminothiophen-2-yl group | 6-(2-Fluoroethyl)pyridin-3-yl group |
| I-220 | 5-Methylaminothiophen-2-yl group | 6-(2-Fluoroethoxy)pyridin-3-yl group |
| I-221 | 5-Methylaminothiophen-2-yl group | 5-Iodothiophen-2-yl group |
| I-222 | 5-Methylaminothiophen-2-yl group | 5-Fluorothiophen-2-yl group |
| I-223 | 5-Methylaminothiophen-2-yl group | 5-(2-Fluoroethyl)thiophen-2-yl group |
| I-224 | 5-Methylaminothiophen-2-yl group | 5-(2-Fiuoroethoxy)thiophen-2-yl group |
| I-225 | 5-Methylaminothiophen-2-yl group | 5-Iodofuran-2-yl group |
| I-226 | 5-Methylaminothiophen-2-yl group | 5-Fluorofuran-2-yl group |
| I-227 | 5-Methylaminothiophen-2-yl group | 5-(2-Fluoroethyl)furan-2-yl group |
| I-228 | 5-Methylaminothiophen-2-yl group | 5-(2-Fluoroethoxy)furan-2-yl group |
| I-229 | 5-Dimethylaminothiophen-2-yl group | 6-Iodopyridin-3-yl group |
| I-230 | 5-Dimethylaminothiophen-2-ylgroup | 6-Fluoropyridin-3-yl group |
| I-231 | 5-Dimethylaminothiophen-2-yl group | 6-(2-Fluaroethyl)pyridin-3-yl group |
| I-232 | 5-Dimethylaminothiophen-2-yl group | 6-(2-Fluoroethoxy)pyridin-3-yl group |
| I-233 | 5-Dimethylaminothiophen-2-yl group | 5-Iodothiophen-2-yl group |
| I-234 | 5-Dimethylaminothiophen-2-yl group | 5-Fluorothiophen-2-yl group |
| I-235 | 5-Dimethylaminothiophen-2-yl group | 5-(2-Fluoroethyl)thiophen-2-yl group |
| I-236 | 5-Dimethylaminothiophen-2-yl group | 5-(2-Fluoroethooy)thiophar-2-yl group |
| I-237 | 5-Dimethylaminothiophen-2-yl group | 5-Iodofuran-2-yl group |
| I-238 | 5-Dimethylaminothiophen-2-yl group | 5-Fluorofuran-2-yl group |
| I-239 | 5-Dimethylaminothiophen-2-yl group | 5-(2-Fluoroethyl)furan-2-yl group |
| I-240 | 5-Dimethylaminothiophen-2-yl group | 5-(2-Fluoroetroxy)furan-2-yl group |
| I-241 | 5-Aminofuran-2-yl group | 6-Iodopyridin-3-yl group |
| I-242 | 5-Aminofuran-2-yl group | 6-Fluoropyridin-3-yl group |
| I-243 | 5-Aminofuran-2-yl group | 6-(2-Fluoroethyl)pyridin-3-yl group |
| I-244 | 5-Aminofuran-2-yl group | 6-(2-Fluoroethoxy)pyridin-3-yl group |
| I-245 | 5-Aminofuran-2-yl group | 5-Iodothiophen-2-yl group |
| I-246 | 5-Aminofuran-2-yl group | 5-Fluorothiophen-2-yl group |
| I-247 | 5-Aminofuran-2-yl group | 5-(2-Fluoroethyl)thiophen-2-yl group |
| I-248 | 5-Aminofuran-2-yl group | 5-(2-Fluoroethoxy)thiophen-2-yl group |
| I-249 | 5-Aminofuran-2-yl group | 5-Iodofuran-2-yl group |
| I-250 | 5-Aminofuran-2-yl group | 5-Fluorofiman-2-yl group |
| I-251 | 5-Aminofuran-2-yl group | 5-(2-Fluoroethyl)furan-2-yl group |
| I-252 | 5-Aminofuran-2-yl group | 5-(2-Fluoroethoxy)furan-2-yl group |

**[Table 6]**

| Number | R¹ | R² |
|---|---|---|
| I-253 | 5-Methylaminofuran-2-yl group | 6-Iodopyridin-3-yl group |
| I-254 | 5-Methylaminofuran-2-yl group | 6-Fluoropyridin-3-yl group |
| I-255 | 5-Methylaminofuran-2-yl group | 6-(2-Fluoroethyl)pyridin-3-yl group |
| I-256 | 5-Methylaminofuran-2-yl group | 6-(2-Fluoroethoxy)pyridin-3-yl group |
| I-257 | 5-Methylaminofuran-2-yl group | 5-Iodothiophen-2-yl group |
| I-258 | 5-Methylaminofuran-2-yl group | 5-Fluorothiophen-2-yl group |
| I-259 | 5-Methylaminofuran-2-yl group | 5-(2-Fluoroethyl)thiophen-2-yl group |
| I-260 | 5-Methylaminofuran-2-yl group | 5-(2-Fluoroethoxy)thiophen-2-yl group |
| I-261 | 5-Methylaminofuran-2-yl group | 5-Iodofuran-2-yl group |
| I-262 | 5-Methylaminofuran-2-yl group | 5-Fluorofuran-2-yl group |
| I-263 | 5-Methylaminofuran-2-yl group | 5-(2-Fluoroethyl)furan-2-yl group |
| I-264 | 5-Methylaminofuran-2-yl group | 5-(2-Fluoroethoxy)furan-2-yl group |
| I-265 | 5-Dimethylaminofuran-2-yl group | 6-Iodopyridin-3-yl group |
| I-266 | 5-Dimethylaminofuran-2-yl group | 6-Fluoropyridin-3-yl group |
| I-267 | 5-Dimethylaminofuran-2-yl group | 6-(2-Fluoroethyl)pyridin-3-yl group |
| I-268 | 5-Dimethylaminofuran-2-yl group | 6-(2-Fluoroethoxy)pyridin-3-yl group |
| I-269 | 5-Dimethylaminofuran-2-yl group | 5-Iodothiophen-2-yl group |
| I-270 | 5-Dimethylaminofuran-2-yl group | 5-Fluorothiophen-2-yl group |
| I-271 | 5-Dimethylaminofuran-2-yl group | 5-(2-Fluoroethyl)thiophen-2-yl group |
| I-272 | 5-Dimethylaminofuran-2-yl group | 5-(2-Fluoroethoxy)thiophen-2-yl group |
| I-273 | 5-Dimethylaminofuran-2-yl group | 5-Iodofuran-2-yl group |
| I-271 | 5-Dimethylaminofuran-2-yl group | 5-Fluorofuran-2-yl group |
| I-275 | 5-Dimethylaminofuran-2-yl group | 5-(2-Fluoroethyl)furan-2-yl group |
| I-276 | 5-Dimethylaminofuran-2-yl group | 5-(2-Fluoroethoxy)furan-2-yl group |
| I-277 | Thiazol-2-yl group | 6-Iodopyridin-3-yl group |
| I-278 | Thiazol-2-yl group | 6-Fluoropyridin-3-yl group |
| I-279 | Thiazol-2-yl group | 6-(2-Fluoroethyl)pyridin-3-yl group |
| I-280 | Thiazol-2-yl group | 6-(2-Fluoroethoxy)pyridin-3-yl group |
| I-281 | Thiazol-2-yl group | 5-Iodothiophen-2-yl group |
| I-282 | Thiazol-2-yl group | 5-Fluorothiophen-2-yl group |
| I-283 | Thiazol-2-yl group | 5-(2-Fluoroethyl)thiophen-2-yl group |
| I-284 | Thiazol-2-yl group | 5-(2-Fluoroethoxy)thiophen-2-yl group |
| I-285 | Thiazol-2-yl group | 5-Iodofuran-2-yl group |
| I-286 | Thiazol-2-yl group | 5-Fluorofuran-2-yl group |
| I-287 | Thiazol-2-yl group | 5-(2-Fluoroethyl)furan-2-yl group |
| I-288 | Thiazol-2-yl group | 5-(2-Fluoroethoxy)furan-2-yl group |
| I-289 | IH-Imidazol-5-yl group | 6-Iodopyridin-3-yl group |
| I-290 | IH-Imidazol-5-yl group | 6-Fluoropyridin-3-yl group |
| I-291 | IH-Imidazol-5-yl group | 6-(2-Fluoroethyl)pyridin-3-yl group |
| I-292 | IH-Imidazol-5-yl group | 6-(2-Fluoroethoxy)pyridin-3-yl group |
| I-293 | IH-Imidazol-5-yl group | 5-Iodothiophen-2-yl group |
| I-294 | IH-Imidazol-5-yl group | 5-Fluorothiophen-2-yl group |
| I-295 | IH-Imidazol-5-yl group | 5-(2-Fluoroethyl)thiophen-2-yl group |
| I-296 | IH-Imidazol-5-yl group | 5-(2-Fluoroethoxy)thiophen-2-yl group |
| I-297 | IH-Imidazol-5-yl group | 5-Iodofuran-2-yl group |
| I-198 I-298 | IH-Imidazol-5-yl group | 5-Fluorofuran-2-yl group |
| I-299 | IH-Imidazol-5-yl group | 5-(2+Fluoroethyl)furan-2-yl group |
| I-300 | IH-Imidazol-5-yl group | 5-(2-Fluoroethoxy)furan-2-yl group |

**[Table 7]**

| Number | R¹ | R² |
|---|---|---|
| I-301 | 1H-Imidazol-2-yl group | 6-Iodbpyridin-3-yl group |
| I-302 | 1H-Imidazol-2-yl group | 6-Fluoropyridin-3-yl group |
| I-303 | 1H-Imidazol-2-yl group | 6-(2-Fluoroethyl)pyridin-3-yl group |
| I-304 | 1H-Imidazol-2-yl group | 6-(2-Fluoroethoxy)pyridin-3-yl group |
| I-305 | 1H-Imidazol-2-yl group | 5-Iodothiophen-2-yl group |
| I-306 | 1H-Imidazol-2-yl group | 5-Fluorothiophen-2-yl group |
| I-307 | 1H-Imidazol-2-yl group | 5-(2-Fluoroethyl)thiophen-2-yl group |
| I-308 | 1H-Imidazol-2-yl group | 5-(2-Fluoroethoxy)thiophen-2-yl group |
| I-309 | 1H-Imidazol-2-yl group | 5-Icdofuran-2-yl group |
| I-310 | 1H-Imidazol-2-yl group | 5-Fluorofuran-2-yl group |
| I-311 | 1H-Imidazol-2-yl group | 5-(2-Fluoroethyl)furan-2-yl group |
| I-312 | 1H-Imidazol-2-yl group | 5-(2-Fluoroethoxy)fiuran-2-yl group |
| I-313 | Pyridin-4-yl group | 6-Iodopyridin-3-yl group |
| I-314 | Pyridin-4-yl group | 6-Fluoropyridin-3-yl group |
| I-315 | Pyridin-4-yl group | 6-(2-Fluoroethyl)pyridin-3-yl group |
| I-316 | Pyridin-4-yl group | 6-(2-Fluoroethoxy)pyridin-3-yl group |
| I-317 | Pyridin-4-yl group | 5-Iodothiophen-2-yl group |
| I-318 | Pyridin-4-yl group | 5-Fluorothiophen-2-yl group |
| I-319 | Pyridin-4-yl group | 5-(2-Fluoroethyl)thiophen-2-yl group |
| I-320 | Pyridin-4-yl group | 5-(2-Fluoroethoxy)thiophen-2-yl group |
| I-321 | Pyridin-4-yl group | 5-Iodofuran-2-yl group |
| I-322 | Pyridin-4-yl group | 5-Fluorofuran-2-yl group |
| I-323 | Pyridin-4-yl group | 5-(2-Fluotroethyl)furan-2-yl group |
| I-324 | Pyridin-4-yl group | 5-(2-Fluoroethoxy)furan-2-yl group |
| I-325 | Pyridin-3-yl group | 6-Iodopyridin-3-yl group |
| I-326 | Pyridin-3-yl group | 6-Fluoropyridin-3-yl group |
| I-327 | Pyridin-3-yl group | 6-(2-Fluoroethyl)pyridin-3-yl group |
| I-328 | Pyridin-3-yl group | 6-(2-Fluoroethoxy)pyridin-3-yl group |
| I-329 | Pyridin-3-yl group | 5-Iodothiophen-2-yl group |
| I-330 | Pyridin-3-yl group | 5-Fluorothiophen-2-yl group |
| I-331 | Pyridin-3-yl group | 5-(2-Fluoroethyl)thiophen-2-yl group |
| I-332 | Pyridin-3-yl group | 5-(2-Fluoroethoxy)thiophen-2-yl group |
| I-333 | Pyridin-3-yl group | 5-Iodofuran-2-yl group |
| I-334 | Pyridin-3-yl group | 5-Fluorofuran-2-yl group |
| I-335 | Pyridin-3-yl group | 5-(2-Fluoroethyl)furan-2-yl group |
| I-336 | Pyridin-3-yl group | 5-(2-Fluoroethoxy)furan-2-yl group |
| I-337 | Pyridin-2-yl group | 6-Iodopyridin-3-yl group |
| I-338 | Pyridin-2-yl group | 6-Fluoropyrdin3-yl group |
| I-339 | Pyridin-2-yl group | 6-(2-Fluoroethyl)pyridin-3-yl group |
| I-340 | Pyridin-2-yl group | 6-(2-Fluoruethoxy)pyridin-3-yl group |
| I-341 | Pyridin-2-yl group | 5-Iodothiophen-2-yl group |
| I-342 | Pyridin-2-yl group | 5-Fluorothiophen-2-yl group |
| I-343 | Pyridin-2-yl group | 5-(2-Fluoroethyl)thiophen-2-yl group |
| I-344 | Pyridin-2-yl group | 5-(2-Fluoroethoxy)thiophen-2-yl group |
| I-315 | Pyridin-2-yl group | 5-Iodofuran-2-yl group |
| I-346 | Pyridin-2-yl group | 5-Fluorofuran-2-yl group |
| I-347 | Pyridin-2-yl group | 5-(2-Fluoroethyl)Furan-2-yl group |
| I-348 | Pyridin-2-yl group | 5-(2-Fluoroethoxy)furan-2-yl group |

**[Table 8]**

| Number | R¹ | R² |
|---|---|---|
| I-349 | 6-Hydroxypyridin-3-yl group | 5-Methylaminothiophen-2-yl group |
| I-350 | 6-Hydroxypyridin-3-yl group | 5-Dimethylaminothiophen-2-yl group |
| I-351 | 6-Hydroxypyridin-3-yl group | 5-Methylaminofuran-2-yl group |
| I-352 | 6-Hydroxypyridin-3-yl group | 5-Dimethylaminofuran-2-yl group |
| I-353 | 6-Hydroxypyridin-3-yl group | 4-Methylaminophenyl group |
| I-354 | 6-Hydroxypyridin-3-yl group | 4-Dimethylaminophenyl group |
| I-355 | 5-Hydroxythiophen-2-yl group | 5-Methylaminothiophen-2-yl group |
| I-356 | 5-Hydroxythiophen-2-yl group | 5-Dimethylaminothiophen-2-yl group |
| I-357 | 5-Hydroxythiophen-2-yl group | 5-Methylaminofuran-2-yl group |
| I-358 | 5-Hydroxythiophen-2-yl group | 5-Dimethylaminofuram-2-yl group |
| I-359 | 5-Hydroxythiophen-2-yl group | 4-Methylaminophenyl group |
| I-360 | 5-Hydroxythiophen-2-yl group | 4-Dimethylaminophenyl group |
| I-361 | 5-Hydroxythiophen-2-yl group | 5-Methylaminothiophen-2-yl group |
| I-362 | 5-Hydroxythiophen-2-yl group | 5-Dimethylaminothiophen-2-yl group |
| I-363 | 5-Hydroxythiophen-2-yl group | 5-Methylaminofuran-2-yl group |
| I-364 | 5-Hydroxythiophen-2-yl group | 5-Dimethylaminofuran-2-yl group |
| I-365 | 5-Hydroxythiophen-2-yl group | 4-Methylaminophenyl group |
| I-366 | 5-Hydroxythiophen-2-yl group | 4-Dimethylaminophenyl group |
| I-367 | 5-Methylaminothiophen-2-yl group | 6-Hydroxypyridin-3-yl group |
| I-368 | 5-Methylaminothiophen-2-yl group | 5-Hydroxythiophen-2-yl group |
| I-369 | 5-Methylaminothiophen-2-yl group | 5-Hydroxyfuran-2-yl group |
| I-370 | 5-Methylaminothiophen-2-yl group | 6-Hydroxypyridin-3-yl group |
| I-371 | 5-Methylaminothiophen-2-yl group | 5-Hydroxythiophem-2-yl group |
| I-372 | 5-Methylaminothiophen-2-yl group | 5-Hydroxyfuran-2-yl group |
| I-373 | 5-Methylaminofuran-2-yl group | 6-Hydroxypyridin-3-yl group |
| I-374 | 5-Methylaminofuran-2-yl group | 5-Hydroxythiophen-2-yl group |
| I-375 | 5-Methylaminofuran-2-yl group | 5-Hydroxyfuran-2-yl group |
| I-376 | 5-Dimethylaminofuran-2-yl group | 6-Hydroxypyridin-3-yl group |
| I-377 | 5-Dimethylaminofuran-2-yl group | 5-Hydroxythiophen-2-yl group |
| I-378 | 5-Dimethylaminofuran-2-yl group | 5-Hydroxyfuran-2-yl group |

**[Table 9]**

| Number | R¹ | R² |
|---|---|---|
| I-379 | 6-[2-(2-Fluoroethoxy)ethoxy]pyridin-3-yl group | 5-Aminothiophen-2-yl group |
| I-380 | 6-[2-(2-Fluoroethoxy)ethoxy]pyridin-3-yl group | 5-Methylaminothiophen-2-yl group |
| I-381 | 6-[2-(2-Fluoroethoxy)ethoxy]pyridin-3-yl group | 5-Dimethylaminothiophen-2-yl group |
| I-382 | 6-[2-(2-Fluoroethoxy)ethoxy]pyridin-3-yl group | 5-Aminofuran-2-yl group |
| I-383 | 6-[2-(2-Fluoroethoxy)ethoxy]pyridin-3-yl group | 5-Methylaminofuran-2-yl group |
| I-384 | 6-[2-(2-Fluoroethoxy)ethoxy]pyridin-3-yl group | 5-Dimethylaminofuran-2-yl group |
| I-385 | 6-[2-(2-Fluoroethoxy)ethoxy]pyridin-3-yl group | Thiazol-2-yl group |
| I-386 | 6-[2-(2-Fluoroethoxy)ethoxy]pyridin-3-yl group | 1H-Imidazol-5-yl group |
| I-387 | 6-[2-(2-Fluoroethoxy)ethoxy]pyridin-3-yl group | 1H-Imidazol-2-yl group |
| I-388 | 6-[2-(2-Fluoroethoxy)ethoxy]pyridin-3-yl group | 4-Aminophenyl group |
| I-389 | 6-[2-(2-Fluoroethoxy)ethoxy]pyridin-3-yl group | 4-Methylaminophenyl group |
| I-390 | 6-[2-(2-Fluoroethoxy)ethoxy]pyridin-3-yl group | 4-Dimethylaminophenyl group |
| I-391 | 6-[2-(2-Fluoroethoxy)ethoxy]pyridin-3-yl group | 4-Hydroxyphenyl group |
| I-392 | 6-[2-(2-Fluoroethoxy)ethoxy]pyridin-3-yl group | 4-Methoxyphenyl group |
| I-393 | 6-[2-(2-Fluoroethoxy)ethoxy]pyridin-3-yl group | Pyridin-4-yl group |
| I-394 | 6-[2-(2-Fluoroethoxy)ethoxy]pyridin-3-yl group | Pyridin-3-yl group |
| I-395 | 6-[2-(2-Fluoroethoxy)ethoxy]pyridin-3-yl group | Pyridin-2-yl group |
| I-396 | 6-{2-[2-(2-Fluoroethoxy)ethoxy]ethoxy}pyridin-3-yl group | 5-Aminothiophen-2-yl group |
| I-397 | 6-{2-[2-(2-Fluoroethoxy)ethoxy]ethoxy}pyridin-3-yl group | 5-Methylaminothiophen-2-yl group |
| I-398 | 6-{2-[2-(2-Fluoroethoxy)ethoxy]ethoxy}pyridin-3-yl group | 5-Dimethylaminothiophen-2-yl group |
| I-399 | 6-{2-[2-(2-Fluoroethoxy)ethoxy]ethoxy}pyridin-3-yl group | 5-Aminofuran-2-yl group |
| I-400 | 6-{2-[2-(2-Fluoroethoxy)ethoxy]ethoxy}pyridin-3-yl group | 5-Methylaminofuran-2-yl group |
| I-401 | 6-{2-[2-(2-Fluoroethoxy)ethoxy]ethoxy}pyridin-3-yl group | 5-Dimethylaminofuran-2-yl group |
| I-402 | 6-{2-[2-(2-Fluoroethoxy)ethoxy]ethoxy}pyridin-3-yl group | Thiazol-2-yl group |
| I-403 | 6-{2-[2-(2-Fluoroethoxy)ethoxy]ethoxy}pyridin-3-yl group | 1H-Imidazol-5-yl group |
| I-404 | 6-{2-[2-(2-Fluoroethoxy)ethoxy]ethoxy}pyridin-3-yl group | 1H-Imidazol-2-yl group |
| I-405 | 6-{2-[2-(2-Fluoroethoxy)ethoxy]ethoxy}pyridin-3-yl group | 4-Aminophenyl group |
| I-406 | 6-{2-[2-(2-Fluoroethoxy)ethoxy]ethoxy}pyridin-3-yl group | 4-Methylaminophenyl group |
| I-407 | 6-{2-[2-(2-Fluoroethoxy)ethoxy]ethoxy}pyridin-3-yl group | 4-Dimethylaminophenyl group |
| I-408 | 6-{2-[2-(2-Fluoroethoxy)ethoxy]ethoxy}pyridin-3-yl group | 4-Hydroxyphenyl group |
| I-409 | 6-{2-[2-(2-Fluoroethoxy)ethoxy]ethoxy}pyridin-3-yl group | 4-Methoxyphenyl group |
| I-410 | 6-{2-[2-(2-Fluoroethoxy)ethoxy]ethoxy}pyridin-3-yl group | Pyridin-4-yl group |
| I-411 | 6-{2-[2-(2-Fluoroethoxy)ethoxy]ethoxy}pyridin-3-yl group | Pyridin-3-yl group |
| I-412 | 6-{2-[2-(2-Fluoroethoxy)ethoxy]ethoxy}pyridin-3-yl group | Pyridin-2-yl group |

**[Table 10]**

| Number | R¹ | R² |
|---|---|---|
| I-413 | 5-[2-(2-Fluodoethoxy)ethoxy]thiophen-2-yl goup | 5-Aminouhiophen-2-yl group |
| I-414 | 5-[2-(2-Fluoroethoxy)ethoxy]thiophen-2-yl group | 5-Methylaminothiophen-2-yl group |
| I-415 | 5-[2-(2-Fluoroethoxy)ethoxy]thiophen-2-yl group | 5-Dimethylaminothiophen-2-yl group |
| I-416 | 5-[2-(2-Fluoroethoxy)ethoxy]thiophen-2-yl group | 5-Aminofuran-2-yl group |
| I-417 | 5-[2-(2-Fluoroethoxy)ethoxy]thiophen-2-yl group | 5-Methylaminofuran-2-yl group |
| I-418 | 5-[2-(2-Fluoroethoxy)ethoxy]thiophen-2-yl group | 5-Dimethylaminofuran-2-yl group |
| I-419 | 5-[2-(2-Fluoroethoxy)etroxy]thiophen-2-yl group | Thiazol-2-yl group |
| I-120 | 5-[2-(2-Fluoroethoxy)ethoxy]thiophen-2-yl group | 1H-Imidazol-5-yl group |
| I-421 | 5-[2-(2-Fluoroethoxy)ethoxy]thiophen-2-yl group | 1H-Imidazol-2-yl group |
| I-422 | 5-[2-(2-Fluoroethoxy)ethoxy]thiophen-2-yl group | 4-Aminophenyl group |
| I-423 | 5-[2-(2-Fluoroethoxy)ethoxy]thiophen-2-yl group | 4-Methylaminophenyl group |
| I-424 | 5-[2-(2-Fluoroethoxy)ethoxy]thiophen-2-yl group | 4-Dimethylaminopenyl group |
| I-425 | 5-[2-(2-Fluoroethoxy)ethoxy]thiophen-2-yl group | 4-Hydroxyphenyl group |
| I-426 | 5-[2-(2-Fluoroethoxy)ethoxy]thiophen-2-yl group | 4-Methoxyphenyl group |
| I-127 | 5-[2-(2-Fluoroethoxy)ethoxy]thiophen-2-yl group | Pyridin-4-yl group |
| I-428 | 5-[2-(2-Fluoroethoxy)ethoxy]thiophen-2-yl group | Pyridin-3-yl group |
| I-429 | 5-[2-(2-Fluoroethoxy)ethoxy] thiopher-2-yl group | Pyridin-2-yl group |
| I-430 | 5-{2-[2-(2-Fluoroethoxy)ethoxy]ethoxy}thiophen-2-yl group | 5-Aminothiophen-2-yl group |
| I-431 | 5-{2-[2-(2-Fluoroethoxy)ethoxy]ethoxy}thiophen-2-yl group | 5-Methylaminothiophen-2-yl group |
| I-432 | 5-{2-[2-(2-Fluoroethoxy)ethoxy]ethoxy}thiophen-2-yl group | 5-Dimethylaminothiophen-2-yl group |
| I-433 | 5-{2-[2-(2-Fluoroethoxy)ethoxy]ethoxy}thiophen-2-yl group | 5-Aminofuran-2-yl group |
| I-434 | 5-{2-[2-(2-Fluoroethoxy)ethoxy]ethoxy}thiophen-2-yl group | 5-Methylaminofuran-2-yl group |
| I-435 | 5-{2-[2-(2-Fluoroethoxy)ethoxy]ethoxy}thiophen-2-yl group | 5-Dimethylaminofuran-2-yl group |
| I-436 | 5-{2-[2-(2-Fluoroethoxy)ethoxy]ethoxy}thiophen-2-yl group | Thiazol-2-yl group |
| I-437 | 5-{2-[2-(2-Fluoroethoxy)ethoxy]ethoxy}thiophen-2-yl group | 1H-Imidazol-5-yl group |
| I-438 | 5-{2-[2-(2-Fluoroethoxy)ethoxy]ethoxy}thiophen-2-yl group | 1H-Imidazol-2-yl group |
| I-439 | 5-{2-[2-(2-Fluoroethoxy)ethoxy]ethoxy}thiophen-2-yl group | 4-Aminopenyl group |
| I-440 | 5-{2-[2-(2-Fluoroethoxy)ethoxy]ethoxy}thiophen-2-yl group | 4-Methylaminophenyl group |
| I-441 | 5-{2-[2-(2-Fluoroethoxy)ethoxy]ethoxy}thiophen-2-yl group | 4-Dimethylaminophenyl group |
| I-442 | 5-{2-[2-(2-Fluoroethoxy)ethoxy]ethoxy}thiophen-2-yl group | 4-Hydroxyphenyl group |
| I-143 | 5-{2-[2-(2-Fluoroethoxy)ethoxy]ethoxy}thiophen-2-yl group | 4-Methoxyphenyl group |
| I-444 | 5-{2-[2-(2-Fluoroethoxy)ethoxy]ethoxy}thiophen-2-yl group | Pyridin-4-yl group |
| I-415 | 5-{2-[2-(2-Fluoroethoxy)ethoxy]ethoxy}thiophen-2-yl group, | Pyridin-3-yl group |
| I-146 | 5-{2-[2-(2-Fluoroethoxy)ethoxy]ethoxy}thiophen-2-yl group | Pyridin-2-yl group |

**[Table 11]**

| Number | R¹ | R² |
|---|---|---|
| I-447 | 5-[2-(2-Fluoroethoxy)ethoxyl]furan-2-yl group | 5-Aminothiophen-2-yl group |
| I-448 | 5-[2-(2-Fluoroethoxy)ethoxyl]furan-2-yl group | 5-Methylaminothiophen-2-yl group |
| I-449 | 5-[2-(2-Fluoroethoxy)ethoxyl]furan-2-yl group | 5-Dimethylaminothiophen-2-yl group |
| I-450 | 5-[2-(2-Fluoroethoxy)ethoxyl]furan-2-yl group | 5-Aminofuran-2-yl group |
| I-451 | 5-[2-(2-Fluoroethoxy)ethoxyl]furan-2-yl group | 5-Methylaminofuran-2-yl group |
| I-452 | 5-[2-(2-Fluoroethoxy)ethoxyl]furan-2-yl group | 5-Dimethylaminofuran-2-yl group |
| I-453 | 5-[2-(2-Fluoroethoxy)ethoxyl]furan-2-yl group | Thiazol-2-yl group |
| I-454 | 5-[2-(2-Fluoroethoxy)ethoxyl]furan-2-yl group | 1H-Imidazol-5-yl group |
| I-455 | 5-[2-(2-Fluoroethoxy)ethoxyl]furan-2-yl group | 1H-Imidzol-2-yl group |
| I-456 | 5-[2-(2-Fluoroethoxy)ethoxyl]furan-2-yl group | 4-Aminophenyl group |
| I-457 | 5-[2-(2-Fluoroethoxy)ethoxyl]furan-2-yl group | 4-Methylaminophenyl group |
| I-458 | 5-[2-(2-Fluoroethoxy)ethoxyl]furan-2-yl group | 4-Dimethylaminophenyl group |
| I-459 | 5-[2-(2-Fluoroethoxy)ethoxyl]furan-2-yl group | 4-Hydroxyphenyl group |
| I-460 | 5-[2-(2-Fluoroethoxy)ethoxyl]furan-2-yl group | 4-Methoxyphenyl group |
| I-461 | 5-[2-(2-Fluoroethoxy)ethoxyl]furan-2-yl group | Pyridin-4-yl group |
| I-462 | 5-[2-(2-Fluoroethoxy)ethoxyl]furan-2-yl group | Pyridin-3-yl group |
| I-463 | 5-[2-(2-Fluoroethoxy)ethoxyl]furan-2-yl group | Pynidin-2-yl group |
| I-464 | 5-{2-[2-(2-Fluoroethoxy)ethoxy]ethoxy}furan-2-yl group | 5-Aminothiophen-2-yl group |
| I-465 | 5-{2-[2-(2-Fluoroethoxy)ethoxy]ethoxy}furan-2-yl group | 5-Methylaminothiophen-2-yl group |
| I-466 | 5-{2-[2-(2-Fluoroethoxy)ethoxy]ethoxy}furan-2-yl group | 5-Dimethylaminothiophen-2-yl group |
| I-467 | 5-{2-[2-(2-Fluoroethoxy)ethoxy]ethoxy}furan-2-yl group | 5-Aminofuran-2-yl group |
| I-468 | 5-{2-[2-(2-Fluoroethoxy)ethoxy]ethoxy}furan-2-yl group | 5-Methylaminofuran-2-yl group |
| I-469 | 5-{2-[2-(2-Fluoroethoxy)ethoxy]ethoxy}furan-2-yl group | 5-Dimethylaminofuran-2-yl group |
| I-470 | 5-{2-[2-(2-Fluoroethoxy)ethoxy]ethoxy}furan-2-yl group | Thiazol-2-yl group |
| I-471 | 5-{2-[2-(2-Fluoroethoxy)ethoxy]ethoxy}furan-2-yl group | 1H-Imidazol-5-yl group |
| I-472 | 5-{2-[2-(2-Fluoroethoxy)ethoxy]ethoxy}furan-2-yl group | 1H-Imidazol-2-yl group |
| I-473 | 5-{2-[2-(2-Fluoroethoxy)ethoxy]ethoxy}furan-2-yl group | 4-Aminophenyl group |
| I-474 | 5-{2-[2-(2-Fluoroethoxy)ethoxy]ethoxy}furan-2-yl group | 4-Methylaminophenyl group |
| I-475 | 5-{2-[2-(2-Fluoroethoxy)ethoxy]ethoxy}furan-2-yl group | 4-Dimethylaminophenyl group |
| I-476 | 5-{2-[2-(2-Fluoroethoxy)ethoxy]ethoxy}furan-2-yl group | 4-Hydroxyphenyl group |
| I-477 | 5-{2-[2-(2-Fluoroethoxy)ethoxy]ethoxy}furan-2-yl group | 4-Methoxyphenyl group |
| I-478 | 5-{2-[2-(2-Fluoroethoxy)ethoxy]ethoxy}furan-2-yl group | Pyridin-4-yl group |
| I-479 | 5-{2-[2-(2-Fluoroethoxy)ethoxy]ethoxy}furan-2-yl group | Pyridin-3-yl group |
| I-480 | 5-{2-[2-(2-Fluoroethoxy)ethoxy]ethoxy}furan-2-yl group | Pyridin-2-yl group |

Among the above-mentioned compounds, 1-12 (Compound 4 of Example 1), 1-79 (Compound 13 of Example 2), and 1-80 (Compound 1 of Example 1) are preferred.

The compound represented by the general formula (I) can be synthesized according to the descriptions in the examples described later, Boeck P et al., Bioorganic & Medicinal Chemistry, 14, 1538-1545, 2006, Robinson PR et al., Bioorganic & Medicinal Chemistry Letters, 13, 4007-4013, 2005, and the like.

The compound represented by the general formula (I) is preferably labeled with a labeling substance. As the labeling substance, fluorescent substances, affinity-substances, and the like can be used, but radionuclides are preferably used. Types of radionuclides used for labeling are not particularly limited, and can be suitably selected depending on the use purpose. For example, when the compound represented by the general formula (I) is used for diagnosis by single photon emission computed tomography (SPECT), γ-ray-emitting radionuclides such as ^{99m}Tc, ¹¹¹In, ⁶⁷Ga, ²⁰¹TI, ¹²³I, and ¹³³Xe (preferably, ^{99m}Tc and ¹²³I) can be used as the radionuclides. Furthermore, when the compound represented by the general formula (I) is used for diagnosis by positron emission tomography (PET), positron-emitting radionuclides such as ¹¹C, ¹³N, ¹⁵O, ¹⁸F, ⁶²Cu, ⁶⁸Ga, and ⁷⁶Br (preferably, ¹¹C, ¹³N, ¹⁵O, and ¹⁸F) can be used. Furthermore, when the compound represented by the general formula (I) is administered to animals other than humans, radionuclides having a longer half life such as, for example, ¹²⁵I may be used. Radionuclides may be contained in the molecule of the compound represented by the general formula (I) or bound to the compound represented by the general formula (I).

It is sufficient to use a method commonly used for each radionuclide as the method for binding a radionuclide to the compound represented by the general formula (I). Furthermore, when a radionuclide is bound to the compound represented by the general formula (I), the radionuclide alone may be bound, but a radionuclide bound to another substance may be bound. Since the above-mentioned ^{99m}Tc is usually bound to a compound to be labeled in the form of a complex, a complex containing ⁹⁹Tc may also be bound to the compound represented by the general formula (I). Examples of the complex containing ⁹⁹Tc include a complex containing 2-hydrazinopyridine (Liu S et al., Bioconjug Chem. 1996 Jan-Feb; 7(1): 63-71), a complex containing N-(2-mercaptoethyl)-2-[(2-mercaptoethyl)amino]-acetamide (Zhen W et al., J Med Chem. 1999 Jul 29; 42(15): 2805-15), a complex containing 2,2'-(1,2-ethanediyldiimino)bis-ethanethiol (Oya S et al., Nucl Med Biol. 1998 Feb; 25(2): 135-40), a tricarbonyl complex (Schibli R et al., Bioconjug Chem. 2000 may-Jun; 11(3): 345-51), and so forth.

Instead of the compound represented by the general formula (I), a pharmaceutically acceptable salt thereof can be used. Examples of the pharmaceutically acceptable salt thereof include alkali metal salts (sodium salts, potassium salts, and lithium salts), alkaline earth metal salts (calcium salts and magnesium salts), sulfates, hydrochlorides, nitrates, phosphates, and so forth.

The composition of the present invention is used for diagnosis of an amyloid-related disease. Here, the "amyloid-related disease" means a disease caused by accumulation of amyloid β protein, primarily Alzheimer's disease, but examples thereof also include diseases such as Down's syndrome, hereditary cerebral hemorrhage with amyloidosis-Dutch type (HCHWA-D). Furthermore, a prodrome of a disease, which is not generally recognized as a "disease," is also included in the "amyloid-related disease" in the present invention. Examples of such a prodrome of the disease include mild cognitive impairment (MCI) observed before the onset of Alzheimer's disease and the like.

Usually, diagnosis of an amyloid-related disease using the composition of the present invention is made by administering the composition of the present invention to a patient for whom a diagnosis is to be made, a laboratory animal, or the like, then imaging the brain thereof, and making diagnosis based on the conditions of the compound represented by the general formula (I) (quantity, distribution, etc.) in the image. The administration method of the composition of the present invention is not particularly limited and can be suitably selected depending on the type of a compound, the type of a labeling substance, and the like. Usually, the composition of the present invention is administered by an injection, a drip infusion, or the like into the skin, the peritoneal cavity, a vein, an artery, or the spinal fluid. The dose of the composition of the present invention is not particularly limited and can be suitably selected depending on the type of a compound, the type of a labeling substance, and the like. For adults, 10⁻¹⁰ to 10⁻³ mg, more preferably 10⁻⁸ to 10⁻⁵ mg of the compound represented by the general formula (I) is preferably administered daily.

Since the composition of the present invention is usually administered by an injection or a drip infusion as described above, components usually contained in an injection solution or a drip infusion solution may be contained. Examples of such components include liquid carriers (for example, potassium phosphate buffer, physiological saline, Ringer's solution, distilled water, polyethylene glycol, vegetable oils and fats, ethanol, glycerine, dimethyl sulfoxide, propylene glycol, etc.), antibacterial agents, local anesthetics (for example, procaine hydrochloride, dibucaine hydrochloride, etc.), buffers (for example, tris-hydrochloride buffer, HEPES buffer, etc.), and osmotic modifiers (for example, glucose, sorbitol, sodium chloride, etc.).

The composition for diagnosis of an amyloid-related disease of the present invention can also be used for screening for a therapeutic or prophylactic agent for an amyloid-related disease. For example, a test substance is administered to a model animal of a "disease" such as Alzheimer's disease, the composition for diagnosis of an amyloid-related disease of the present invention is administered to the model animal, and then the distribution or the quantity of the compound represented by the general formula (I) contained in the brain of the model animal is examined. As a result, when a significant difference (for example, a reduced distribution site, a decreased quantity, etc.) is detected as compared with a control (model animal not receiving the test substance), the test substance can be a candidate therapeutic agent for an amyloid-related disease. Furthermore, after a test substance is administered to a model animal with a "prodrome of a disease" such as mild cognitive impairment, the composition for diagnosis of an amyloid-related disease of the present invention is administered to the model animal, and then the distribution or the quantity of the compound represented by the general formula (I) contained in the brain of the model animal is examined. As a result, when a significant difference (for example, a reduction or a slowed enlargement of a distribution site, a decrease or a slowed increase in quantity, etc.) is detected as compared with a control, the test substance can be a candidate prophylactic agent for an amyloid-related disease.

Furthermore, the composition for diagnosis of an amyloid-related disease of the present invention can also be used to evaluate a therapeutic or prophylactic agent for an amyloid-related disease whose effect has already been confirmed. Specifically, after the therapeutic or prophylactic agent for the disease is administered to a model animal of an amyloid-related disease, the composition for diagnosis of an amyloid-related disease of the present invention is administered to the model animal, and then the distribution or the quantity of the compound represented by the general formula (I) contained in the brain of the model animal is examined. This allows the above-mentioned therapeutic or prophylactic agent (specifically, effective dosage, effective administration method, etc. thereof) to be evaluated.

### Examples

Hereafter, the present invention will be explained more specifically with reference to the examples and the reference examples.

### Example 1

### Experimental methods

### Reagents and instrument

Amyloid β protein (Human, 1-42) [TFA form] was purchased from Peptide Institute, Inc., and special grade reagents were used as other reagents. ¹H-NMR was measured using Varian Gemini 300 and tetramethylsilane as an internal standard substance.

### (1) Synthesis of chalcone derivatives containing aromatic heterocyclic ring

### Synthesis of (E) -3-(4-(dimethylamino)phenyl)-1-(5-iodothiophen-2-yl)prop-2-en-1-one (1)

256 mg (1 mmol) of 5-acetyl-2-iodothiophene and 149 mg (1 mmol) of 4-dimethylaminobenzaldehyde were dissolved in ethanol (20 mL), and 10% aqueous potassium hydroxide (30 mL) was added to the mixture with ice cooling. A reaction was allowed at room temperature for 6 h, followed by addition of 30 mL of purified water, and the precipitated crystals were filtered by suction. The crystals were washed with 50% aqueous ethanol and dried to obtain target Compound 1. Yield 238 mg (yield rate 62.1%). ¹H NMR (300 MHz, CDCl₃) δ 3.06 (s, 6H), 6.69 (d, J = 9.0 Hz, 2H), 7.11 (d, J = 15.6 Hz, 1H), 7.32 (d, J = 3.9 Hz, 1H), 7.45 (d, J = 3.9 Hz, 1H), 7.54 (d, J = 9.0 Hz, 2H), 7.81 (d, J = 15.3 Hz, 2H).

### Synthesis of (E)-1-(6-bromopyridin-3-yl)-3-(4-(dimethylamino)phenyl)prop-2-en-1-one (2)

200 mg (1 mmol) of 5-acetyl-2-bromopyridine and 149 mg (1 mmol) of 4-dimethylaminobenzaldehyde were dissolved in ethanol (15 mL), and the mixture was added to 10% aqueous potassium hydroxide (10 mL) with ice cooling.
The mixture was stirred at room temperature for 1 h, the precipitate crystals were collected by suction filtration, washed with ethanol, and dried to obtain target Compound 2. Yield 248 mg (yield rate 74.9%). ¹H NMR (300 MHz, CDCl₃) δ 3.06 (s, 6H), 6.78 (d, J = 9.0 Hz, 2H), 7.46 (d, J = 15.3 Hz, 1H), 7.65 (d, J = 9.0 Hz, 2H), 7.77 (d, J = 8.4 Hz, 1H), 7.82 (d, J = 15.0 Hz, 1H), 8.29 (d, J = 8.4 Hz, 1H), 8.98 (d, J = 2.7 Hz, 1H).

### Synthesis of (E)-1-(6-tributylstannyl-3-yl)-3-(4-(dimethylamino)phenyl)prop-2-en-1-one (3)

To a solution of Compound 2 (100 mg, 0.30 mmol) in dry dioxane (5 mL) were added bis(tributyltin) (1 mL), tetrakis(triphenylphosphine)palladium (100 mg), and triethylamine (5 mL), and the mixture was heated to reflux for 8 h. The reaction solvent was evaporated under reduced pressure, and the residue was subjected to preparative thin layer chromatography using ethyl acetate/hexane (1/4) as an elution solvent to obtain target Compound 3. Yield 22 mg (yield rate 13.5%).

### Synthesis of (E)-3-(4-(dimethylamino)phenyl)-1-(6-iodinepyridin-3-yl)prop-2-en-1-one (4)

To a solution of Compound 3 (15 mg, 0.03 mmol) in ethyl acetate (5 mL) was added a solution of iodine in ethyl acetate (1 mL, 10 mg/mL) at room temperature. A reaction was allowed at room temperature for 30 min, and then saturated aqueous sodium hydrogen sulfite (15 mL) was added to terminate the reaction. The chloroform layer was separated and dried with sodium sulfate, the solvent was evaporated under reduced pressure, and then the residue was subjected to preparative thin layer chromatography using ethyl acetate/hexane (1/3) as an elution solvent to obtain target Compound 4. Yield 7 mg (yield rate 66.8%).

### (2) In-vitro binding inhibition experiment using Aβ(1-42) aggregate

The Aβ(1-42) aggregate was dissolved in a buffer containing 10 mM sodium phosphate and 1 mM EDTA (pH 7.4) at a concentration of 0.5 mg/mL, and the mixture was incubated at 37°C for 36 to 42 h. The binding inhibition experiment was performed using 12 x 75-mm borosilicate glass tubes. 850 µL of 10% ethanol solution, 50 µL of an Aβ-binding substance ([¹²⁵I]4-dimethylamino-4'-iodochalcone) (10% aqueous ethanol), 50 µL (57 nM) of Aβ(1-42) aggregate solution, and 50 µL of 10% aqueous ethanol each containing Compounds 1 and 4 at various concentrations (0.003 to 781 nM) were mixed, and the mixture was allowed to stand at room temperature for 3 h. Further, nonspecific binding was determined using 4-dimethylamino-4'-iodochalcone (400 nM), a nonradioactive compound. Aβ aggregate-bound chalcone derivatives and nonbound chalcone derivatives were separated with a Brandel M-24R cell harvester using a Whatman GF/B filter. The radioactivities of substances remaining on the filter after filtration were measured with a γ counter. The 50% inhibitory concentration was calculated using Graph Pad Prism (graph pad software), and the inhibition constant (Kᵢ value) was calculated by the Cheng-Prusoff equation, Kᵢ = IC50/(1+[L]/Kd). In this equation, the concentration of [¹²⁵I]4-dimethylamino-4'-iodochalcone used in the experiment was used as [L], and the dissociation constant of [¹²⁵I ]4-dimethylamino-4'-iodochalcone against the Aβ(1-42) aggregate was used as Kd.

### Experimental results

### (1) Synthesis of chalcone derivatives containing aromatic heterocyclic ring

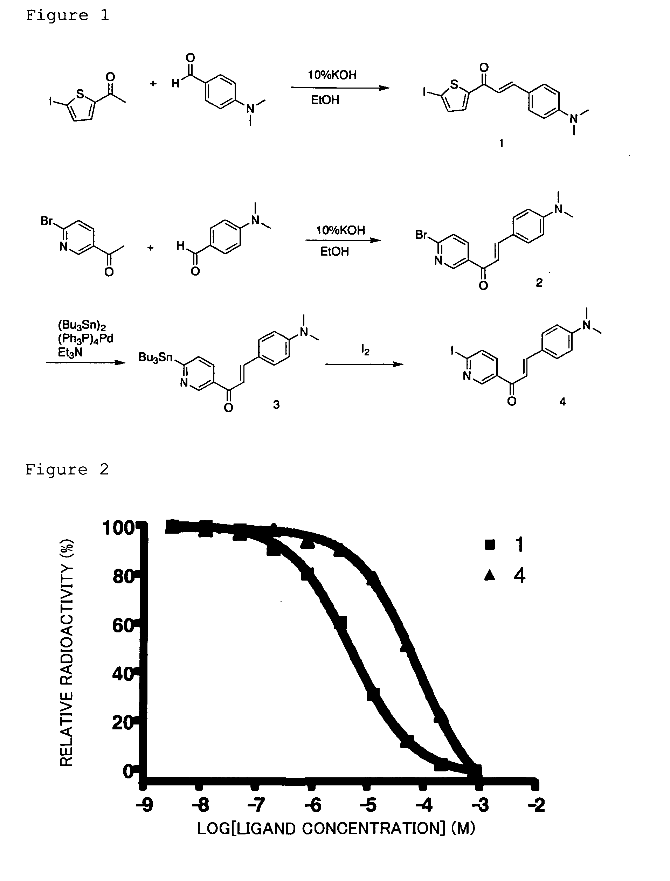

Figure 1 shows synthesis pathways of chalcone derivatives containing an aromatic heterocyclic ring. The chalcone skeleton was formed by an aldol condensation reaction of acetophenone and aldehyde corresponding to each compound. Compound 2 was converted to a tributyltin compound by a reaction with bis(tributyltin) using palladium as a catalyst. Compound 3, a tributyltin compound, was converted to Compound 4 by a reaction with iodine.

### (2) Binding experiment of chalcone derivatives containing aromatic heterocyclic ring to Aβ(1-42) aggregate

Figure 2 shows binding inhibition curves when Compounds 1 and 4 were reacted in the presence of [¹²⁵I]4-dimethylamino-4'-iodochalcone and the Aβ aggregate. Both Compounds 1 and 4 showed a concentration-dependent inhibition activity, and their property of binding to the Aβ aggregate was confirmed. A similar experiment was repeated three times. Table 12 shows the mean 50% inhibitory concentrations and standard deviations. Both Compounds 1 and 4 showed a strong binding property to an amyloid aggregate. In particular, Compound 1 showed a very strong binding property to the amyloid aggregate. Such results suggest that both of these compounds can sufficiently function as an imaging probe of amyloid β.

**[Table 12]**

| Inhibition constants (Ki) for binding of Compounds 1 and 4 to Aβ(1-42) aggregate | |
|---|---|
| Compound | K i (nM) |
| 1 | 5.2 ± 0.7 |
| 4 | 105 ± 24 |

### Example 2

### Experimental methods

### Reagents and instrument

As a radioactive iodine-125 (¹²⁵I), Iodine-125 (3.7 GBq/mL) manufactured by MP Biomedicals, Inc. was used. Reverse phase HPLC was performed at a flow rate of 1.0 mL/min using a Cosmosil 5C₁₈-AR-II column (4.6 x 150 mm) manufactured by Nacalai Tesque Inc. and ultrapure water:acetonitrile = 3:7 as an elution solvent. ¹HNMR was measured using Varian Gemini 300 and tetramethylsilane as an internal standard substance. Mass spectrometry was performed using JEOL IMS-DX300. Preparative TLC was performed using 12 PLC plates 20x20 cm-Silica gel 60 F₂₅₄, 2 mm manufactured by MERCK. Amyloid β protein (Human, 1-42) was purchased from Peptide Institute, Inc., and special grade reagents were used as other reagents.

### (1) Synthesis of chalcone derivatives

### Synthesis of 4-bromo-4'-nitrochalcone (1)

4-Nitroacetophenone (1.67 g, 10.1 mmol) and 4-bromobenzaldehyde (1.86 g, 10.0 mmol) were dissolved in ethanol (10 mL), and 10% aqueous potassium hydroxide (6 mL) was added dropwise with stirring. The mixture was stirred at room temperature for 30 min, then precipitates were collected by filtration and washed with cold ethanol to obtain Compound 1. Yield 1.98 g (yield rate 59.4%).
¹H NMR (300 MHz, CDCl₃) δ 7.48 (d, J = 15.6 Hz, 1H), 7.52 (d, J = 8.7 Hz, 2H), 7.59 (d, J = 8.7 Hz, 2H), 7.79 (d, J = 15.6 Hz, 1H), 8.15 (d, J = 9 Hz, 2H), 8.36 (d, J = 9 Hz, 2H).

### Synthesis of 4-bromo-4'-aminochalcone (2)

Compound 1 (372 mg, 1.12 mmol) was suspended in ethanol (5 mL), tin(II) chloride (1.05 g, 5.55 mmol) was slowly added with stirring, and the mixture was heated to reflux for 1 h. The solvent was evaporated under reduced pressure, followed by addition of 1 N aqueous sodium hydroxide, and the mixture was extracted with ethyl acetate. The mixture was dried with anhydrous sodium sulfate, and then the solvent was evaporated under reduced pressure to obtain Compound 2. Yield 190 mg (yield rate 56.1%). ¹H NMR (300 MHz, CDCl₃ δ 4.19 (s, 2H), 6.70 (d, J = 8.7 Hz, 2H), 7.47-7.55 (m, 5H), 7.71 (d, J = 15.6 Hz, 1H), 7.93 (d, J = 8.7 Hz, 2H).

### Synthesis of 4-tributylstannyl-4'-aminochalcone (3)

Compound 2 (200 mg, 0.66 mmol) was dissolved in 1,4-dioxane (10 mL), followed by addition of bis(tributyltin) (0.4 mL), tetra(triphenylphosphine)palladium (35 mg, 0.030 mmol), and triethylamine (6 mL), and the mixture was heated to reflux for 8 h. The solvent was evaporated under reduced pressure, and the residue was subjected to silica gel column chromatography using ethyl acetate/hexane (2/5) as an elution solvent to obtain Compound 3. Yield 165 mg (yield rate 48.7%). ¹H NMR (300 MHz, CDCl₃) δ 0.87-1.52 (m, 27H), 4.23 (s, 2H), 6.68 (d, J = 8.7 Hz, 2H), 7.50-7.58 (m, 5H), 7.76 (d, 15.3 Hz, 1H), 7.92 (d, J = 8.4 Hz, 2H). MS m/z 513 (MH⁺).

### Synthesis of 4-iodo-4'-aminochalcone (4)

Compound 3 (90 mg, 0.18 mmol) was dissolved in chloroform (5 mL), followed by addition of a solution of iodine in chloroform (2 mL, 0.25 M), and the mixture was stirred at room temperature. After 20 min of reaction, saturated aqueous sodium sulfite was added to terminate the reaction. The chloroform layer was separated and dried with anhydrous sodium sulfate, then the solvent was evaporated under reduced pressure, and the residue was purified by preparative TLC using ethyl acetate/hexane (1/1) as a developing solvent to obtain Compound 4. Yield 39 mg (yield rate 63.6%). ¹H NMR (300 MHz, CDCl₃) δ 4.17 (s, 2H), 6.70 (d, 8.4 Hz, 2H), 7,36 (d, J = 8.7 Hz, 2H), 7.54 (d, J = 15.6 Hz, 1H), 7.69 (d, J = 15.6 Hz, 1H), 7.74 (d, J = 8.4 Hz, 2H), 7.92 (d, J = 8.4 Hz, 2H). MS m/z 349 (M⁺).

### Synthesis of 4-bromo-4'-methylaminochalcone (5)

Compound 2 (230 mg, 0.76 mmol) was dissolved in DMSO (5 mL), followed by addition of potassium carbonate (526 mg, 3.81 mmol) and methyl iodide (0.2 mL), and the mixture was stirred at room temperature for 5 h. Purified water (50 mL) was added, the mixture was extracted with ethyl acetate (50 mL) and dried with anhydrous sodium sulfate, and then the solvent was evaporated under reduced pressure. The residue was subjected to silica gel column chromatography using ethyl acetate/hexane (1/3) as an elution solvent to obtain Compound 5. Yield 78 mg (yield rate 32.4%). ¹H NMR (300 MHz, CDCl₃) δ 2.89-2.94 (m, 3H), 4.36 (s, 1H), 6.61 (d, J = 8.7 Hz, 2H), 7.50-7.57 (m, 3H), 7.70 (d, J = 15.6 Hz, 1H), 7.84 (d, J = 8.4 Hz, 2H), 7.96 (d, J = 8.7 Hz, 2H).

### Synthesis of 4-tributylstannyl-4'-methylaminochalcone (6)

Compound 5 (110 mg, 0.35 mmol) was dissolved in 1,4-dioxane (9 mL), followed by addition of bis(tributyltin) (0.2 mL), tetra(triphenylphosphine)palladium (16 mg, 0.014 mmol), and triethylamine (5 mL), and the mixture was heated to reflux for 6 h. The reaction solvent was evaporated under reduced pressure, and the residue was subjected to silica gel column chromatography using ethyl acetate/hexane (2/7) as an elution solvent to obtain Compound 6. Yield 70 mg (yield rate 38.2%). ¹H NMR (300 MHz, CDCl) δ 0.87-1.59 (m, 27H), 2.93 (d, J = 5.1 Hz, 3H), 4.31 (s, 1H), 6.61 (d, J = 8.7 Hz, 2H), 7.50-7.60 (m, 5H), 7.77 (d, J = 15.6 Hz, 1H), 7.97 (d, J = 9 Hz, 2H). MS m/z 527 (MH⁺).

### Synthesis of 4-iodo-4'-methylaminochalcone (7)

Compound 6 (50 mg, 0.095 mmol) was dissolved in chloroform (5 mL), followed by addition of a solution of iodine in chloroform (1 mL, 0.25 M), and the mixture was stirred at room temperature for 15 min. Saturated aqueous sodium sulfite was added to terminate the reaction, the chloroform layer was separated and dried with anhydrous sodium sulfate, and then the solvent was evaporated under reduced pressure. The residue was purified by preparative TLC using ethyl acetate/hexane (2/3) as a developing solvent to obtain Compound 7.
Yield 16 mg (yield rate 46.7%). ¹H NMR (300 MHz, CDCl₃) δ 2.93 (d, J = 4.5 Hz, 3H), 4.35 (s, 1H), 6.61 (d, J = 9 Hz, 2H), 7.36 (d, J = 8.4 Hz, 2H), 7.56 (d, J = 15.6 Hz, 1H), 7.68 (d, J = 16.2 Hz, 1H), 7.74 (d, J = 8.1 Hz, 2H), 7.96 (d, J = 9 Hz, 2H). MS m/z 363 (M⁺).

### Synthesis of 4-bromo-4'-dimethylaminochalcone (8)

Compound 2 (300 mg, 0.99 mmol) and paraformaldehyde (315 mg, 10.5 mmol) were dissolved in acetic acid (15 mL), sodium cyanoborohydride (300 mg, 4.77 mmol) was slowly added, and the mixture was stirred at room temperature for 4 h. 1 N aqueous sodium hydroxide was added, the mixture was extracted with chloroform and dried with dried with anhydrous sodium sulfate, and then the solvent was evaporated under reduced pressure. The residue was subjected to silica gel column chromatography using ethyl acetate/hexane (1/4) as an elution solvent to obtain Compound 8. Yield 150 mg (yield rate 45.7%). ¹H NMR (300 MHz, CDCl₃) δ 3.09 (s, 6H), 6.71 (d, J = 9 Hz, 2H), 7.36 (d, J = 8.4 Hz, 2H), 7.59 (d, J = 15.3 Hz, 1H), 7.69 (d, J = 15.9 Hz, 1H), 7.74 (d, J = 8.4 Hz, 2H), 8.00 (d, J = 9 Hz, 2H). MS m/z 377 (M⁺).

### Synthesis of 4-tributylstannyl-4'-dimethylaminochalcone (9)

Compound 8 (70 mg, 0.21 mmol) was dissolved in 1,4-dioxane (5 mL), followed by addition of bis(tributyltin) (0.2 mL), tetra(triphenylphosphine)palladium (10 mg, 0.009 mmol), and triethylamine (2 mL), and the mixture was heated to reflux for 10 h. The reaction solvent was evaporated under reduced pressure, and the residue was purified by preparative TLC using ethyl acetate/hexane (1/3) as a developing solvent to obtain Compound 9. Yield 36 mg (yield rate 31.4 %). ¹H NMR (300 MHz, CDCl₃ δ 0.87-1.66 (m, 27H), 3.09 (s, 6H), 6.71 (d, J = 8.7 Hz, 2H), 7.34-7.62 (m, 5H), 7.77 (d, J = 15.9 Hz, 1H), 8.01 (d, J = 9 Hz, 2H). MS m/z 541 (MH⁺).

### Synthesis of 4-iodo-4'-dimethylaminochalcone (10)

Compound 9 (48 mg, 0.088 mmol) was dissolved in chloroform (3 mL), followed by addition of a solution of iodine in chloroform (0.8 mL, 0.25 M), and the mixture was stirred at room temperature for 20 min. Saturated aqueous sodium sulfite was added to terminate the reaction, the chloroform layer was separated and dried with anhydrous sodium sulfate, and then the solvent was evaporated under reduced pressure. Further, the residue was washed with hexane to obtain Compound 10. Yield 11 mg (yield rate 32.8%). ¹H NMR (300 MHz, CDCl₃) δ 3.09 (s, 6H), 6.71 (d, J = 9 Hz, 2H), 7.36 (d, J = 8.4 Hz, 2H), 7.56 (d, J = 15.6 Hz, 1H), 7.68 (d, J = 16.2 Hz, 1H), 7.74 (d, J = 8.1 Hz, 2H), 7, 96 (d, J = 9 Hz, 2H). MS m/z 363 (M⁺).

### Synthesis of 1-(5-iodo-2-thienyl)-3-(4-nitrophenyl)-prop-2-en-1-one (11)

2-Acetyl-5-iodothiophene (486 mg, 1.93 mmol) and 4-nitrobenzaldehyde (296 mg, 1.96 mmol) were dissolved in ethanol (10 mL), and 10% aqueous potassium hydroxide (6 mL) was added dropwise with stirring. The mixture was stirred at room temperature for 30 min, and the precipitates were collected by filtration and washed with 50% ethanol to obtain Compound 11. Yield 625 mg (yield rate 84.1%). ¹H NMR (300 MHz, CDCl₃) δ 7.38-7.43 (m, 2H), 7.51 (d, J = 4.2 Hz, 1H), 7.78 (d, J = 8.4 Hz, 2H), 7.84 (d, J = 15.6 Hz, 1H), 8.29 (d, J = 9 Hz, 2H).

### Synthesis of 1-(5-iodo-2-thienyl)-3-(4-aminophenyl)-prop-2-en-1-one (12)

Compound 11 (625 mg, 1.62 mmol) was suspended in ethanol (10 mL), tin(II) chloride (1.55 g, 8.17 mmol) was slowly added with stirring, and the mixture was heated to reflux for 1 h. The reaction solvent was evaporated under reduced pressure, followed by addition of 1 N sodium aqueous hydroxide, and the mixture was extracted with ethyl acetate. The mixture was dried with anhydrous sodium sulfate, and then the solvent was evaporated under reduced pressure to obtain Compound 12. Crude yield 586 mg. ¹H NMR (300 MHz, CDCl₃) δ 4.03 (s, 2H), 6.68 (d, J = 8.7 Hz, 2H), 7.13 (d, J = 15.3 Hz, 1H), 7.33 (d, J = 3.9 Hz, 1H), 7.44-7.49 (m, 3H), 7.78 (d, J = 15.3 Hz, 2H). MS m/z 355 (M⁺).

### Synthesis of 1-(5-iodo-2-thienyl)-3-(4-methylaminophenyl)-prop-2-en-1-one (13)

Compound 12 (190 mg, 0.54 mmol) was dissolved in DMSO (6 mL), followed by addition of potassium carbonate (374 mg, 2.71 mmol) and methyl iodide (0.1 mL), and the mixture was stirred for 20 min with heating at 50°C. Purified water (50 mL) was added, and the mixture was extracted with ethyl acetate (50 mL) and dried with anhydrous sodium sulfate, and then the solvent was evaporated under reduced pressure. The residue was subjected to silica gel column chromatography using ethyl acetate/hexane (1/4) as an elution solvent to obtain Compound 13. Yield 45 mg (yield rate 22.8%). ¹H NMR (300 MHz, CDCl₃) δ 2.90 (s, 3H), 4.22 (s, 1H), 6.59 (d, J = 8.4 Hz, 2H), 7.11 (d, J = 15.3 Hz, 1H), 7.32 (d, J = 4.2 Hz, 1H), 7.44 (d, J = 3.9 Hz, 1H), 7.50 (d, J = 8.7 Hz, 2H), 7.80 (d, J = 15.3 Hz, 1H). MS m/z 369 (M⁺).

### Synthesis of 1-(5-iodo-2-thienyl)-3-(4-dimethylaminophenyl)-prop-2-en-1-one (14)

2-Acetyl-5-iodothiophene (260 mg, 1.03 mmol) and 4-dimethylaminobenzaldehyde (159 mg, 1.07 mmol) were dissolved in ethanol (15 mL), and 10% aqueous potassium hydroxide (15 mL) was added dropwise with stirring. The mixture was stirred at room temperature for 3 h, and then the precipitates were collected by filtration and washed with 50% ethanol to obtain Compound 14. Yield 274 mg (yield rate 69.3%). ¹H NMR (300 MHz, CDCl₃) δ 3.05 (s, 6H), 6.79 (d, J = 8.7 Hz, 2H), 7.11 (d, J = 15.3 Hz, 1H), 7.32 (d, J = 3.9 Hz, 1H), 7.45 (d, J = 3.9 Hz, 1H), 7.54 (d, J = 8.7 Hz, 2H), 7.81 (d, J = 15 Hz, 1H). MS m/z 383 (M⁺).

### Synthesis of 1-(5-bromo-2-thienyl)-3-(4-nitrophenyl)-prop-2-en-1-one (15)

2-Acetyl-5-bromothiophene (623 mg, 3.04 mmol) and 4-nitrobenzaldehyde (464 mg, 3.07 mmol) was dissolved in ethanol (25 mL), and 10% aqueous potassium hydroxide (3 mL) was added dropwise with stirring. The mixture was stirred at room temperature for 30 min, and the precipitates were collected by filtration and washed with 50% ethanol to obtain Compound 15. Yield 686 mg (yield rate 66.8%). ¹H NMR (300 MHz, CDCl₃) δ 7.19 (d, J = 3.9 Hz, 1H), 7.48 (d, J = 15.6 Hz, 1H), 7.63 (d, J = 3.9 Hz, 1H), 7.78 (d, J = 8.4 Hz, 2H), 7.85 (d, J = 15.6 Hz, 1H), 8.29 (d, J = 8.7 Hz, 2H).

### Synthesis of 1-(5-bromo-2-thienyl)-3-(4-aminophenyl)-prop-2-en-1-one (16)

Compound 15 (678 mg, 2.20 mmol) was suspended in ethanol (20 mL), tin(II) chloride (2.04 g, 10.8 mmol) was slowly added with stirring, and the mixture was heated to reflux for 1 h. The reaction solvent was evaporated under reduced pressure, followed by addition of 1 N aqueous sodium hydroxide, and the mixture was extracted with ethyl acetate. The mixture was dried with anhydrous sodium sulfate, and then the solvent was evaporated under reduced pressure to obtain Compound 16. Crude yield 626 mg. ¹H NMR (300 MHz, CDCl₃) δ 4.03 (s, 2H), 6.68 (d, J = 9 Hz, 2H), 7.11-7.16 (m, 2H), 7.47 (d, J = 8.7 Hz, 2H), 7.56 (d, J = 3.9 Hz, 1H), 7.78 (d, J = 15.3 Hz, 1H). MS m/z 309 (MH⁺).

### Synthesis of 1-(5-bromo-2-thienyl)-3-(4-methylaminophenyl)-prop-2-en-1-one (17)

Compound 16 (212 mg, 0,69 mmol) was dissolved in DMSO (6 mL), followed by addition of potassium carbonate (483 mg, 3.49 mmol) and methyl iodide (0.1 mL), and the mixture was stirred at room temperature for 2 h. Purified water (100 mL) was added, the mixture was extracted with ethyl acetate (100 mL) and dried with anhydrous sodium sulfate, and then the solvent was evaporated under reduced pressure. The residue was subjected to silica gel column chromatography using ethyl acetate/hexane (1/5) as an elution solvent to obtain Compound 17. Yield 40 mg (yield rate 18.0%). ¹H NMR (300 MHz, CDCl₃) δ 2.91 (s, 3H), 4.19 (s, 1H), 6.60 (d, J = 8.7 Hz, 2H), 7.09-7.14 (m, 2H), 7.50 (d, J = 8.4 Hz, 2H), 7.56 (d, J = 4.2 Hz, 1H), 7.80 (d, J = 15.6 Hz, 1H). MS m/z 323 (MH⁺).

### Synthesis of 1-(5-tributylstannyl-2-thienyl)-3-(4-methylaminophenyl)-prop-2-en-1-one (18)

Compound 17 (40 mg, 0.12 mmol) was dissolved in 1,4-dioxane (5 mL), followed by addition of bis(tributyltin) (0.1 mL), tetra(triphenylphosphine)palladium (10 mg, 0.009 mmol), and triethylamine (3 mL), and the mixture was heated to reflux for 2 h. The reaction solvent was evaporated under reduced pressure, and the residue was purified by preparative TLC using ethyl acetate/hexane (1/5) as a developing solvent to obtain Compound 18. Yield 10 mg (yield rate 15.1%). ¹H NMR (300 MHz, CDCl₃) δ 0.88-1.63 (m, 27H), 2.90 (d, J = 5.1 Hz, 3H), 4.14 (s, 1H), 6.60 (d, J = 8.4 Hz, 2H), 7.21-7.26 (m, 2H), 7.52 (d, J = 8.4 Hz, 2H), 7.80 (d, J = 15.6 Hz, 1H), 7.19 (d, J = 3,6 Hz, 1H). MS m/z 533 (MH⁺).

### Synthesis of 1-(5-bromo 2-thienyl)-3-(4-dimethylaminophenyl)-prop-2-en-1-one (19)

2-Acetyl-5-bromothiophene (416 mg, 2.03 mmol) and 4-dimethylaminobenzaldehyde (318 mg, 2.13 mmol) were dissolved in ethanol (5 mL), and 10% aqueous potassium hydroxide (5 mL) was added dropwise with stirring. The mixture was stirred at room temperature for 3 h, and then the precipitates were collected by filtration and washed with 50% ethanol to obtain Compound 19. Yield 565 mg (yield rate 82.8%). ¹H NMR (300 MHz, CDCl₃ ) δ 3.05 (s, 6H), 6.69 (d, J = 8.7 Hz, 2H), 7.12 (d, J = 15.3 Hz, 1H), 7.13 (d, J = 3.9 Hz, 1H), 7.55-7.57 (m, 3H), 7.18 (d, J = 15.3 Hz, 1H). MS m/z 337 (MH⁺).

### Synthesis of 1-(5-tributylstannyl-2-thienyl)-3-(4-dimethylaminophenyl)-prop-2-en-1-one (20)

Compound 19 (102 mg, 0.30 mmol) was dissolved in 1,4-dioxane (8 mL), followed by addition of bis(tributyltin) (0.2 mL), tetra(triphenylphosphine)palladium (16 mg, 0.014 mmol), and triethylamine (4 mL), and the mixture was heated to reflux for 2 h. The reaction solvent was evaporated under reduced pressure, and the residue was subjected to silica gel column chromatography using ethyl acetate/hexane (1/9) as an elution solvent to obtain Compound 20. Yield 10 mg (yield rate 15.1%). ¹H NMR (300 MHz, CDCl₃) δ 0.88-1.63 (m, 27H), 3.04 (s, 6H), 6.70 (d, J = 9 Hz, 2H), 7.21-7.27 (m, 2H), 7.55 (d, J = 9 Hz, 2H), 7.81 (d, J = 15.3 Hz, 1H), 7.92 (d, J = 3.6 Hz, 1H). MS m/z 547 (MH⁺).

### Synthesis of 4'-iodochalcone (21)

4-Iodoacetophenone (247 mg, 1.00 mmol) and benzaldehyde (0.11 mL, 1.08 mmol) were dissolved in ethanol (5 mL), and 10% aqueous potassium hydroxide (1 mL) was added dropwise with stirring. The mixture was stirred at room temperature for 2 h, and then the precipitates were collected by filtration and washed with 50% ethanol to obtain Compound 21. Yield 293 mg (yield rate 87.4%). ¹H NMR (300 MHz, CDCl₃) δ 7.42-7.44 (m, 3H), 7.47 (d, J = 15.6 Hz, 1H), 7.63-7.66 (m, 2H), 7.74 (d, J = 8.4 Hz, 2H), 7.82 (d, J = 15.9 Hz, 1H), 7.88 (d, J = 8.7 Hz, 2H). MS m/z 334 (M⁺).

### Synthesis of 3-(2-furanyl)-1-(4-iodophenyl)-prop-2-en-1-one (22)

4-Iodoacetophenone (246 mg, 1.0 mmol) and 2-furaldehyde (96 mg, 1.0 mmol) were dissolved in ethanol (5 mL), and 10% aqueous potassium hydroxide (5 mL) was added dropwise with stirring. The mixture was stirred at room temperature for 30 min, and then the precipitates were collected by filtration and washed with 50% ethanol to obtain Compound 22. Yield 212 mg (yield rate 65.4%). ¹H NMR (300 MHz, CDCl₃) δ 6.52-6.54 (m, 1H), 6.74 (d, J = 3.6 Hz, 1H), 7.39 (d, J = 15.0 Hz, 1H), 7.54 (d, J = 1.8 Hz, 1H), 7.60 (d, J = 15.6 Hz, 1H), 7.74 (d, J = 8.4 Hz, 2H), 7.86 (d, J = 8.4 Hz, 2H). MS m/z 324 (M⁺).

### Synthesis of 3-(3-furanyl)-1-(4-iodophenyl)-prop-2-en-1-one (23)

4-Iodoacetophenone (246 mg, 1.0 mmol) and 3-furaldehyde (96 mg, 1.0 mmol) were dissolved in ethanol (5 mL), and 10% aqueous potassium hydroxide (1 mL) was added dropwise with stirring. The mixture was stirred at room temperature for 30 min, and then the precipitates were collected by filtration and washed with 50% ethanol to obtain Compound 23. Yield 279 mg (yield rate 86.1%). ¹H NMR (300 MHz, CDCl₃) δ 6.70 (d, J = 1.8 Hz, 1H), 7.17 (d, J = 15.3 Hz, 1H), 7.48 (s, 1H), 7.69-7.75 (m, 4H), 7.86 (d, J = 9.0 Hz, 2H). MS m/z 324 (M⁺).

### Synthesis of 1-(4-iodophenyl)-3-(2-thienyl)-prop-2-en-1-one (24)

4-Iodoacetophenone (500 mg, 2.03 mmol) and 2-thiophenecarboxyaldehyde (228 mg, 2.03 mmol) were dissolved in ethanol (10 mL), and 10% aqueous potassium hydroxide (10 mL) was added dropwise with stirring. The mixture was stirred at room temperature for 30 min, and then the precipitates were collected by filtration and washed with 50% ethanol to obtain Compound 24. Yield 546 mg (yield rate 79.1%). ¹H NMR (300 MHz, CDCl₃) δ 7.09-7.12 (m, 1H), 7.26 (d, J = 15.0 Hz, 1H), 7.38 (d, J = 3.6 Hz, 1H), 7.44 (d, J = 5.4 Hz, 1H), 7.18 (d, J = 8.7 Hz, 2H), 7.87 (d, J = 8.4 Hz, 2H), 7.95 (d, J = 15.3 Hz, 1H). MS m/z 340 (M⁺).

### Synthesis of 1-(4-iodophenyl)-3-(3-thienyl)-prop-2-en-1-one (25)

4-Iodoacetophenone (246 mg, 1.00 mmol) and thiophene-3-carboaldehyde (0.1 mL, 1.10 mmol) were dissolved in ethanol (3 mL), and 10% aqueous potassium hydroxide (1 mL) was added dropwise with stirring. The mixture was stirred at room temperature for 30 min, and then the precipitates were collected by filtration and washed with 50% ethanol to obtain Compound 25. Yield 295 mg (yield rate 86.7%). ¹H NMR (300 MHz, CDCl₃) δ 7.26 (d, J = 15.6 Hz, 1H), 7.38-7.43 (m, 2H), 7.62-7.65 (m, 1H), 7.71 (d, J = 8.7 Hz, 2H), 7.80 (d, J = 15.6 Hz, 1H), 7.87 (d, J = 8.4 Hz, 1H). MS m/z 340 (M⁺).

### Synthesis of 1-(4-iodophenyl)-3-(1H-imidazol-2-yl)-prop-2-en-1-one (26)

4-Iodoacetophenone (246 mg, 1.00 mmol) and 2-imidazolecarboxyaldehyde (96 mg, 1.00 mmol) were dissolved in ethanol (10 mL), and 10% aqueous potassium hydroxide (10 mL) was added dropwise with stirring. The mixture was reacted at room temperature for 1 h and then extracted with ethyl acetate (50 mL). The mixture was dried with anhydrous sodium sulfate, then the solvent was evaporated under reduced pressure, and the residue was subjected to silica gel chromatography using ethyl acetate/hexane (1/1) as an elution solvent to obtain Compound 26. Yield 142 mg (yield rate 43.8%). ¹H NMR (300 MHz, CDCl₃) δ 7.31-7.40 (m, 2H), (s,) 7.64 (d, J = 15.6 Hz, 1H), 7.78 (d, J = 8.1 Hz, 2H), 7.82 (d, J = 15.6 Hz, 1H), 7.87 (d, J = 8.4 Hz, 2H). MS m/z 324 (M⁺).

### Synthesis of 1-(4-iodophenyl)-3-(thiazol-2-yl)-prop-2-en-1-one (27)

4-Iodoacetophenone (246 mg, 1.00 mmol) and 2-thiazolecarboxyaldehyde (113 mg, 1.00 mmol) were dissolved in ethanol (5 mL), and 10% aqueous potassium hydroxide (5 mL) was added dropwise with stirring. The mixture was stirred at room temperature for 30 min, and then the precipitates were collected by filtration and washed with 50% ethanol to obtain Compound 27. Yield 210 mg (yield rate 61.6%). ¹H NMR (300 MHz, CDCl₃) δ 7.52 (d, J = 3.0 Hz, 1H), 7.75-7.79 (m, 3H), 7.85-7.92 (m, 3H), 7.99 (d, J = 3.6 Hz, 1H). MS m/z 341 (M⁺).

### Synthesis of 5-dimethylamino-2-furaldehyde (28)

5-Bromo-2-furaldehyde (3.00 g, 17.1 mmol) was dissolved in purified water (6 mL), followed by addition of dimethylamine (5.3 mL, 50.9 mmol), and the mixture was heated to reflux for 40 min. Chloroform was added to extract a target compound in the chloroform layer, the chloroform layer was dried with anhydrous sodium sulfate, and the solvent was evaporated under reduced pressure. The residue was subjected to silica gel column chromatography using ethyl acetate/hexane (3/2) as an elution solvent to obtain Compound 28. Yield 845 mg (yield rate 35.4%). ¹H NMR (300 MHz, CDCl₃ ) δ 3.07 (s, 6H), 5.24 (s, 1H), 7.19 (s, 1H), 8.97 (s, 1H).

### Synthesis of 2-acetyl-5-bromofuran (29)

2-Acetylfuran (2.2 g, 20 mmol) was dissolved in DMF (20 mL), followed by addition of N-bromosuccinimide (3.91 g, 22 mmol). The mixture was reacted at room temperature for 30 min and then added into distilled water (50 mL), and the layers were separated with ethyl acetate (50 mL x 2). The ethyl acetate layer was dried with anhydrous sodium sulfate, then the solvent was evaporated under reduced pressure, and the residue was subjected to medium-pressure preparative chromatography using ethyl acetate/hexane (1/15) as an elution solvent to obtain Compound 29. Yield 1.68 g (yield rate 44.5%).

### Synthesis of 5-dimethylamino-2-thiophenecarboxyaldehyde (30)

5-Bromo-2-thiophenecarboxyaldehyde (1 g, 5.23 mmol) and dimethylamine (1.64 mL, 15.69 mmol) were mixed, followed by addition of purified water (3 mL). The mixture was reacted at 100°C for 12 h, and then the layers were separated with chloroform (50 mL x 2) and purified water (50 mL), the chloroform layer was dried with anhydrous sodium sulfate, and then the solvent was evaporated under reduced pressure. The residue was subjected to medium-pressure preparative chromatography using ethyl acetate/hexane (1/1) as an elution solvent to obtain Compound 30. Yield 670 mg (yield rate 82.5%). ¹H NMR (300 MHz, CDCl₃) δ 3.10 (s, 6H), 5.96 (s, 1H), 7.45 (s, 1H), 9.44 (s, 1H).

### Synthesis of 1-(4-iodophenyl)-3-(5-dimethylamino-2-furanyl)-prop-2-en-1-one (31)

4-Iodoacetophenone (246 mg, 1.00 mmol) and Compound 28 (139 mg, 1.00 mmol) were dissolved in ethanol (5 mL), followed by addition of 10% aqueous potassium hydroxide (7.5 mL). The mixture was reacted at room temperature for 3 h, then the layers were separated with ethyl acetate (50 mL x 2), and the ethyl acetate layer was dried with anhydrous sodium sulfate. The solvent was evaporated under reduced pressure, and the residue was subjected to medium-pressure preparative chromatography using ethyl acetate/hexane (1/2) as an elution solvent to obtain Compound 31. Yield 25 mg (yield rate 6.8%). ¹H NMR (300 MHz, CDCl₃) δ 3.04 (s, 6H), 5.22 (d, J = 3.9 Hz, 1H), 6.80 (d, J = 3.6 Hz, 1H), 6.89 (d, J = 15.0 Hz, 1H), 7.46 (d, J = 14.7 Hz, 1H), 7.70 (d, J = 8.1 Hz, 2H), 7.81 (d, J = 8.7 Hz, 2H). MS m/z 367 (M⁺).

### Synthesis of 1-(5-bromo-2-furanyl)-3-(4-dimethylaminophenyl)-prop-2-en-1-one (32)

Compound 29 (500 mg, 2.65 mmol) and 4-dimethylaminobenzaldehyde (394 mg, 2.65 mmol) were dissolved in ethanol (5 mL), followed by addition of 10% aqueous potassium hydroxide (10 mL). The mixture was stirred at room temperature for 1 h, and then the precipitated crystals were collected by filtration and washed with 50% aqueous ethanol to obtain Compound 32. Yield 470 mg (yield rate 55.4%). ¹H NMR (300 MHz, CDCl₃) δ 3.04 (s, 6H), 5.22 (d, J = 3.9 Hz, 1H), 6.80 (d, J = 3.6 Hz, 1H), 6.89 (d, J = 15.0 Hz, 1H), 7.46 (d, J = 14.7 Hz, 1H), 7.70 (d, J = 8.1 Hz, 2H), 7.81 (d, J = 8.7 Hz, 2H). MS m/z 321 (MH⁺).

### Synthesis of 3-(5-dimethylamino-2-thienyl)-1-(4-iodophenyl)-prop-2-en-1-one (33)

4-Iodoacetophenone (246 mg, 1.00 mmol) and Compound 30 (155 mg, 1.00 mmol) were dissolved in ethanol (5 mL), followed by addition of 10% aqueous potassium hydroxide (7.5 mL). The mixture was reacted at room temperature for 3 h, the layers were separated with ethyl acetate (50 mL x 2), and the ethyl acetate layer was dried with anhydrous sodium sulfate. The solvent was evaporated under reduced pressure, and the residue was subjected to medium-pressure preparative chromatography using ethyl acetate/hexane (1/4) as an elution solvent to obtain Compound 33. Yield 18 mg (yield rate 4.7%). ¹H NMR (300 MHz, CDCl₃) δ 3.04 (s, 6H), 5.22 (d, J = 3.9 Hz, 1H), 6.80 (d, J = 3.6 Hz, 1H), 6.89 (d, J = 15.0 Hz, 1H), 7.46 (d, J = 14.7 Hz, 1H), 7.70 (d, J = 8.1 Hz, 2H), 7.81 (d, J = 8.7 Hz, 2H). MS m/z 383 (M⁺).

### Synthesis of 1-(5-bromo-2-furyl)-3-(5-dimethylamino-2-furyl)-prop-2-en-1-one (34)

Compound 29 (100 mg, 0.53 mmol) and Compound 28 (78 mg, 0.56 mmol) were dissolved in ethanol (3 mL), and 10% aqueous potassium hydroxide (1 mL) was added dropwise with stirring. The mixture was stirred at room temperature for 40 min, the solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was subjected to silica gel column chromatography using ethyl acetate/hexane (1/4) as an elution solvent to obtain compound 34. Yield 29 mg (yield rate 17.7%) . ¹H NMR (300 MHz, CDCl₃) δ 3.05 (s, 6H), 5.25 (d, J = 3.6 Hz, 1H), 6.48 (d, J = 3.3 Hz, 1H), 6.78-6.83 (m, 2H), 7.14 (d, J = 3.3 Hz, 1H), 7.49 (d, J = 14.7 Hz, 1H). MS m/z 311 (MH⁺).

### Synthesis of 1-(5-bromo-2-furyl)-3-(5-dimethylamino-2-thienyl)-prop-2-en-1-one (35)

Compound 29 (97 mg, 0.51 mmol) and Compound 30 (75 mg, 0.48 mmol) were dissolved in ethanol (1.5 mL), and 10% aqueous potassium hydroxide (0.5 mL) was added dropwise with stirring. The mixture was stirred at room temperature for 3 h, then the solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was subjected to silica gel column chromatography using ethyl acetate/hexane (2/7) as an elution solvent to obtain Compound 35. Yield 18 mg (yield rate 11.4%). ¹H NMR (300 MHz, CDCl₃) δ 3.08 (s, 6H), 5.85 (d, J = 3.9 Hz, 1H), 6.48 (d, J = 3.6 Hz, 1H), 6.72 (d, J = 15 Hz, 1H), 7.14-7.16 (m, 2H), 7.91 (d, J = 15 Hz, 1H). MS m/z 327 (MH⁺).

### Synthesis of 3-(5-dimethylamino-2-furyl)-1-(5-iodo-2-thienyl)-prop-2-en-1-one (36)

2-Acetyl-5-iodothiophene (123 mg, 0.49 mmol) and Compound 28 (71 mg, 0.51 mmol) were dissolved in ethanol (5 mL), and 10% aqueous potassium hydroxide (2 mL) was added dropwise with stirring. The mixture was stirred at room temperature for 90 min, then the solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was subjected to silica gel column chromatography using ethyl acetate/hexane (1/3) as an elution solvent to obtain Compound 36. Yield 21 mg (yield rate 11.5%). ¹H NMR (300 MHz, CDCl₃) δ 3.04 (s, 6H), 5.22 (d, J = 3.6 Hz, 1H), 6.73 (d, J = 14.4 Hz, 1H), 6.79 (d, J = 3.9 Hz, 1H), 7.29 (d, J = 3.9 Hz, 1H), 7.50 (d, J = 3.9 Hz, 1H), 7.45 (d, J = 14.7 Hz, 1H). MS m/z 373 (M⁺).

### Synthesis of 3-(5-dimethylamino-2-thienyl)-1-(5-iodo-2-thienyl)-prop-2-en-1-one (37)

2-Acetyl-5-iodothiophene (123 mg, 0.49 mmol) and Compound 30 (76 mg, 0.49 mmol) were dissolved in ethanol (3 mL), and 10% aqueous potassium hydroxide (1 mL) was added dropwise with stirring. The mixture was stirred at room temperature for 3 h, then the solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was subjected to silica gel column chromatography using ethyl acetate/hexane (1/4) as an elution solvent to obtain compound 37. Yield 15 mg (yield rate 7.9%). ¹H NMR (300 MHz, CDCl₃) δ 3.07 (s, 6H), 5.85 (d, J = 4.2 Hz, 1H), 6.63 (d, J = 14.7 Hz, 1H), 7.15 (d, J = 4.5 Hz, 1H), 7.29 (d, J = 3.9 Hz, 1H), 7.38 (d, J = 4.2 Hz, 1H), 7.87 (d, J = 14.7 Hz, 1H). MS m/z 389 (M⁺).

### Synthesis of 2,4-dinitro-4'-iodochalcone (38)

4-Iodoacetophenone (246 mg, 1.00 mmol) and 2,4-dinitrobenzaldehyde (202 mg, 1.03 mmol) were dissolved in ethanol (5 mL), and 10% aqueous potassium hydroxide (1 mL) was added dropwise with stirring. The mixture was stirred at room temperature for 6 h, and then the precipitates were collected by filtration and washed with cold ethanol to obtain Compound 38. Yield 232 mg (yield rate 54.7%). ¹H NMR (300 MHz, CDCl₃) δ 7.37 (d, J = 15.9 Hz, 1H), 7.74 (d, J = 8.4 Hz, 2H), 7.90-7.96 (m, 3H), 8.14 (d, J = 16.2 Hz, 1H), 8.53 (d, d, J₁ = 8.4 Hz, J₂ = 2.1 Hz, 1H), 8.95 (d, J = 2.4 Hz, 1H).

### Synthesis of 2,4-diamino-4'-iodochalcone (39)

Compound 38 (132 mg, 0.31 mmol) was suspended in ethanol (5 mL), tin(II) chloride (603 mg, 3.18 mmol) was slowly added with stirring, and the mixture was heated to reflux for 3 h. The solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was subjected to silica gel column chromatography using ethyl acetate as an elution solvent to obtain Compound 39. Yield 39 mg (yield rate 34.4%). ¹H NMR (300 MHz, CDCl₃) δ 4.36 (s, 4H), 7.03 (d, d, J₁ = 8.7 Hz, J₂ = 2.4 Hz, 1H), 7.18 (d, J = 8.7 Hz, 1H), 7.59-7.67 (m, 2H), 7.72 (d, J = 8.4 Hz, 2H), 7.85 (d, J = 8.4 Hz, 2H), 7.95 (m, 1H).

### Synthesis of 4-hydroxy-4'-iodo-3-methoxychalcone (40)

4-Iodoacetophenone (246 mg, 1.0 mmol) and vanillin (152 mg, 1.0 mmol) were dissolved in ethanol (10 mL), and 10% aqueous potassium hydroxide (5 mL) was added dropwise with stirring. The mixture was reacted at room temperature for 3 h and heated to reflux for 2 h, then ethanol was evaporated under reduced pressure, and the mixture was extracted with ethyl acetate (30 mL). The mixture was dried with anhydrous sodium sulfate, the solvent was evaporated under reduced pressure, and the residue was purified by preparative TLC using ethyl acetate/hexane (1/4) as a developing solvent to obtain Compound 40. Yield 20 mg (yield rate 5.3%). ¹H NMR (300 MHz, CDCl₃) δ 3.05 (s, 3H), 5.68 (s, 1H), 6.88 (d, J = 8.7 Hz, 1H), 7.14 (d, d, J₁ = 8.1 Hz, J₂ = 2.1 Hz, 1H), 7.28 (d, J = 2.4 Hz, 1H), 7.33 (d, J = 15.6 Hz, 1H), 7.72 (d, J = 8.7 Hz, 2H), 7.74 (d, J = 15.6 Hz, 1H), 7.86 (d, J = 8.7 Hz, 2H). MS m/z 380 (M⁺).

### Synthesis of 3-hydroxy-4'-iodo-4-methoxychalcone (41)

4-Iodoacetophenone (251 mg, 1.02 mmol) and isovanillin (160 mg, 1.05 mmol) were dissolved in ethanol (5 mL), and 10% aqueous potassium hydroxide (3 mL) was added dropwise with stirring. The mixture was stirred at room temperature for 2 days, then the solvent was evaporated under reduced pressure, followed by addition of 1 N aqueous sodium hydroxide, and the mixture was extracted with ethyl acetate. The mixture was dried with anhydrous sodium sulfate, then the solvent was evaporated under reduced pressure, and the residue was loaded on a medium-pressure preparative column using ethyl acetate/hexane (1/5) as an elution solvent to obtain Compound 41. Yield 143 mg (yield rate 36.9%). ¹H NMR (300 MHz, CDCl₃) δ 3.95 (s, 3H), 5.68 (s, 1H), 6.88 (d, J = 8.7 Hz, 1H), 7.14 (d, d, J₁ = 8.1 Hz, J₂ = 2.1 Hz, 1H), 7.28 (d, J = 2.4 Hz, 1H), 7.33 (d, J = 15.6 Hz, 1H), 7.72 (d, J = 8.7 Hz, 2H), 7.74 (d, J = 15.6 Hz, 1H), 7.86 (d, J = 8.7 Hz, 2H). MS m/z 380 (M⁺).

### Synthesis of 3,4-dimethoxy-4'-iodochalcone (42)

4-Iodoacetophenone (246 mg, 1.0 mmol) and 3,4-dimethoxybenzaldehyde (166 mg, 1.0 mmol) were dissolved in ethanol (10 mL), and 10% aqueous potassium hydroxide (5 mL) was added dropwise with stirring. The mixture was stirred at room temperature for 1 h, and then the precipitated crystals were collected by filtration to obtain Compound 42. Yield 164 mg (yield rate 47.6%). ¹H NMR (300 MHz, CDCl₃) δ 3.95 (s, 3H), 3.96 (s, 3H), 5.68 (s, 1H), 6.91 (d, J = 8.4 Hz, 1H), 7.15 (d, J = 1.8 Hz, 1H), 7.24 (d, d, J₁ = 8.1 Hz, J₂ = 2.1 Hz, 1H), 7.32 (d, J = 15.6 Hz, 1H), 7.73 (d, J = 8.4 Hz, 2H), 7.77 (d, J = 15.6 Hz, 1H), 7.87 (d, J = 8.4 Hz, 2H). MS m/z 394 (M⁺).

### Synthesis of 3-(5-bromo-2-thienyl)-1-(4-nitrophenyl)-prop-2-en-1-one (43)

4-Nitroacetophenone (500 mg, 3.03 mmol) and 5-bromothiophene-2-carboaldehyde (0.33 mL, 3.06 mmol) were dissolved in ethanol (20 mL), and 10% aqueous potassium hydroxide (6 mL) was added dropwise with stirring. The mixture was stirred at room temperature for 3 h, and then the precipitates were collected by filtration and washed with 50% ethanol to obtain Compound 43. Yield 422 mg (yield rate 41.2%). ¹H NMR (300 MHz, CDCl₃) δ 7.09 (d, J = 3.9 Hz, 1H), 7.14-7.20 (m, 2H), 7.86 (d, J = 15.6 Hz, 1H), 8.12 (d, J = 8.7 Hz, 2H), 8.35 (d, J = 8.7 Hz, 2H).

### Synthesis of 3-(5-bromo-2-thienyl)-1-(4-aminophenyl)-prop-2-en-1-one (44)

Compound 43 (410 mg, 1.21 mmol) was suspended in ethanol (20 mL), tin(II) chloride (1.14 g, 6.01 mmol) was slowly added with stirring, and the mixture was heated to reflux for 1 h. The solvent was evaporated under reduced pressure, followed by addition of 1 N aqueous sodium hydroxide, and the mixture was extracted with ethyl acetate. The mixture was dried with anhydrous sodium sulfate, and then the solvent was evaporated under reduced pressure to obtain Compound 44. Yield 453 mg. ¹H NMR (300 MHz, CDCl₃) δ 4.16 (s, 2H), 6.80 (d, J = 8.4 Hz, 2H), 7.03-7.07 (m, 2H), 7.23 (d, J = 15.6 Hz, 1H), 7.78 (d, J = 15 Hz, 1H), 7.89 (d, J = 8.7 Hz, 2H). MS m/z 309 (MH⁺).

### Synthesis of 3-(5-bromo-2-thienyl)-1-(4-methylaminophenyl)-prop-2-en-1-one (45) and 3-(5-bromo-2-thienyl)-1-(4-dimethylaminophenyl)-prop-2-en-1-one (46)

Compound 44 (857 mg, 2.78 mmol) was dissolved in DMSO (15 mL), followed by addition of potassium carbonate (1.94 g, 14.0 mmol) and methyl iodide (0.5 mL), and the mixture was stirred at room temperature for 9 h. Purified water was added, the mixture was extracted with ethyl acetate and dried with anhydrous sodium sulfate, and then the solvent was evaporated under reduced pressure. The residue was subjected to silica gel column chromatography using ethyl acetate/hexane (1/4) as an elution solvent to obtain Compounds 45 and 46. Compound 45: yield 228 mg (25.4%). ¹H NMR (300 MHz, CDCl₃) δ 2.92-2.94 (m, 3H), 4.33 (s, 1H), 6.61 (d, J = 8.7 Hz, 2H), 7.02-7.06 (m, 2H), 7.26 (d, J = 15.3 Hz, 1H), 7.78 (d, J = 15.6 Hz, 1H), 7.93 (d, J = 9 Hz, 2H). MS m/z 323 (MH⁺). Compound 46: yield 53 mg (5.7%). ¹H NMR (300 MHz, CDCl₃) δ 3.09 (s, 6H), 6.70 (d, J = 9 Hz, 2H), 7.02-7.06 (m, 2H), 7.28 (d, J = 15.3 Hz, 1H), 7.78 (d, J = 15 Hz, 1H), 7.96 (d, J = 9 Hz, 2H). MS m/z 337 (MH⁺).

### (2) Labeling of chalcone derivatives with ¹²⁵I

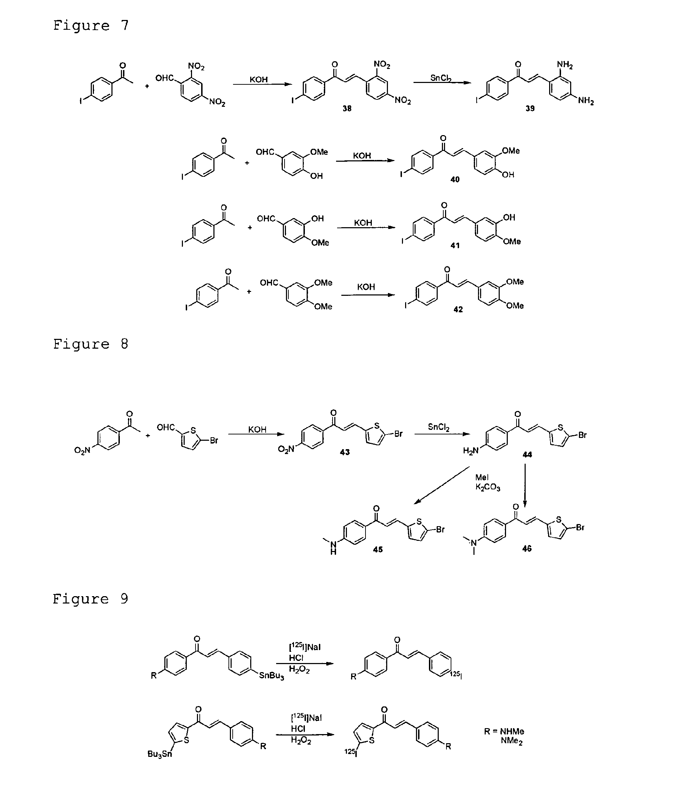

[¹²⁵I]NaI (1 to 5 mCi) and 1 N hydrochloric acid (50 mL) were added to solutions of various tributyltin compounds (1 mg/mL) in ethanol (50 mL), and 3% (w/v) aqueous hydrogen peroxide (50 mL) was added finally (Figure 9). The mixture was allowed to stand for 2 min at room temperature, then saturated aqueous sodium hydrogensulfite (100 mL) was added to terminate the reaction, and saturated aqueous sodium hydrogencarbonate (100 mL) was added to neutralize the reaction solution. The mixture was extracted with ethyl acetate, dehydrated by passing through a Pasteur pipette containing sodium sulfate, and then purified by reverse phase HPLC (water:acetonitrile = 4:6). Nonradioactive compounds were analyzed for absorbance at 254 nm by HPLC as preparations, target compounds having matching absorbance were separated and extracted with ethyl acetate, and ethyl acetate was evaporated under a nitrogen gas flow.

### (3) Preparation of Aβ(1-42) aggregate

Aβ(1-42) was dissolved in 10 mM phosphate buffer containing 1 mM EDTA (pH 7.4) at a concentration of 0.25 mg/mL, and the mixture was incubated at 37°C for 42 h to prepare an Aβ(1-42) aggregate.

### (4) Calculation of inhibition constant (Kᵢ values) by binding inhibition experiment using Aβ(1-42) aggregate

850 mL of 10% EtOH solution, 50 mL of a solution of [¹²⁵I]4-dimethylamino-4'-iodochalcone ([¹²⁵ICL-NMe₂) in 10% ethanol, and 50 mL of a sample solution at various concentrations (0 to 20 mM) were mixed, 50 mL of an Aβ(1-42) aggregate solution was finally added, and the mixture was allowed to stand at room temperature for 3 h. Aβ(1-42) aggregate-bound compounds and nonbound compounds were separated with a Brandel M-24R cell harvester using a Whatman GF/B filter. Radioactivities remaining on the filter after filtration were measured with a γ-counter, and inhibition curves were created using Graph Pad Prism 4.0 to calculate inhibition constants (Kᵢ values).

### (5) Examination of binding site by binding inhibition experiment using Aβ(1-42) aggregate

850 mL of 10% EtOH solution, 50 mL of [¹²⁵I)CL-NMe₂ solution, 50 m L of a solution of congo red, thioflavine T, or compound 14 at various concentrations (0 to 20 mM) were mixed, 50 mL of an Aβ(1-42) aggregate solution was finally added, and the mixture was allowed to stand at room temperature for 3 h. Aβ(1-42) aggregate-bound compounds and nonbound compounds were separated with a Brandel M-24R cell harvester using a Whatman GF/B filter. Radioactivities remaining on the filter after filtration were measured with a γ-counter, and inhibition curves were created using Graph Pad Prism 4.0 to calculate inhibition constants.

### (6) In-vivo radioactivity distribution experiment using normal mice

Radioactive iodine labeling compounds were diluted with physiological saline containing 10% EtOH. To each of four or five 5-week-old male ddY mice (20 to 25 g) per group, 100 mL of a labeled compound, [¹²⁵I]Compound 7, 10, 13, or 14 (0.2 to 0.4 mCi), was administered from the caudal vein. Animals were decapitated at 2, 10, 30, or 60 min, blood was collected, the major organs were isolated, and then weights and radioactivities thereof were measured.

### (7) Fluorescence staining experiment using brain tissue section of Alzheimer's disease model mouse

As an Alzheimer's disease model mouse, APP/PS1 double transgenic mouse was used. The brain was removed, immobilized with 10% formaldehyde, dehydrated with alcohol and xylene, and then paraffin-embedded. The paraffin-embedded brain tissue was cut into a 5-µm section, and the section was treated with xylene, alcohol, and water and deparaffinized. The section was incubated in PBS for 30 min and then in solutions of various chalcone derivatives (100 µM) in 50% aqueous ethanol for 10 min. The section was washed with 50% ethanol and PBS and then examined with a fluorescence microscope (Leica TCS SP2).

### Experimental results

With a focus on senile plaques, which are said to start developing at the earliest stage among pathological changes characteristic to AD, development of an imaging probe specifically binding to Aβ, the major component of senile plaque, was planned. So far, it has been suggested that chalcone derivatives with iodine introduced at the 4'th position and various substituents at the 4th position are useful as amyloid imaging probes. Accordingly, in this example, derivatives with a substituent introduced at the 4'th position and iodine at the 4th position in the chalcone skeleton, derivatives with various aromatic heterocyclic rings introduced into Rings A and B, and derivatives with two introduced substituents were synthesized, and usefulness as amyloid imaging probes was evaluated.

### (1) Synthesis of chalcone derivatives

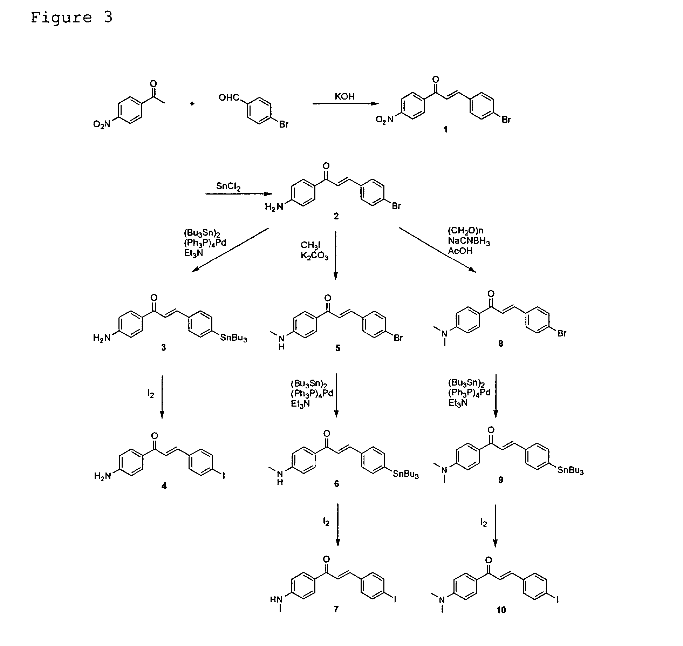

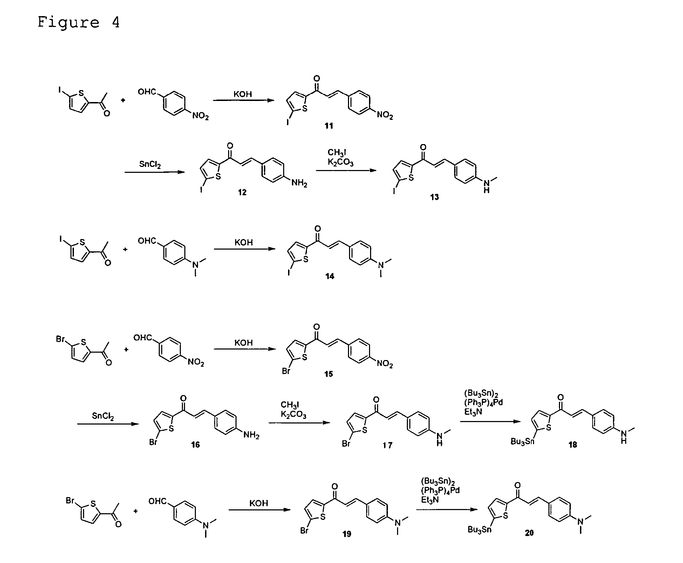

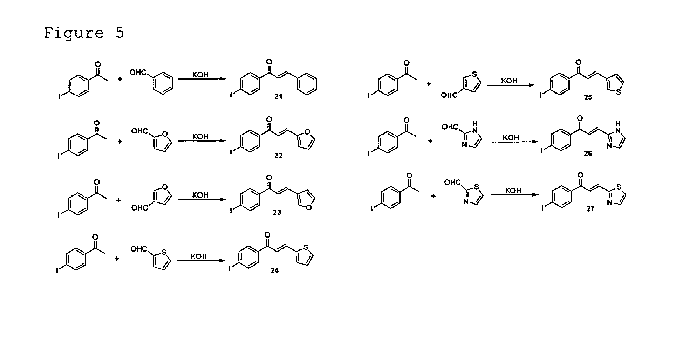

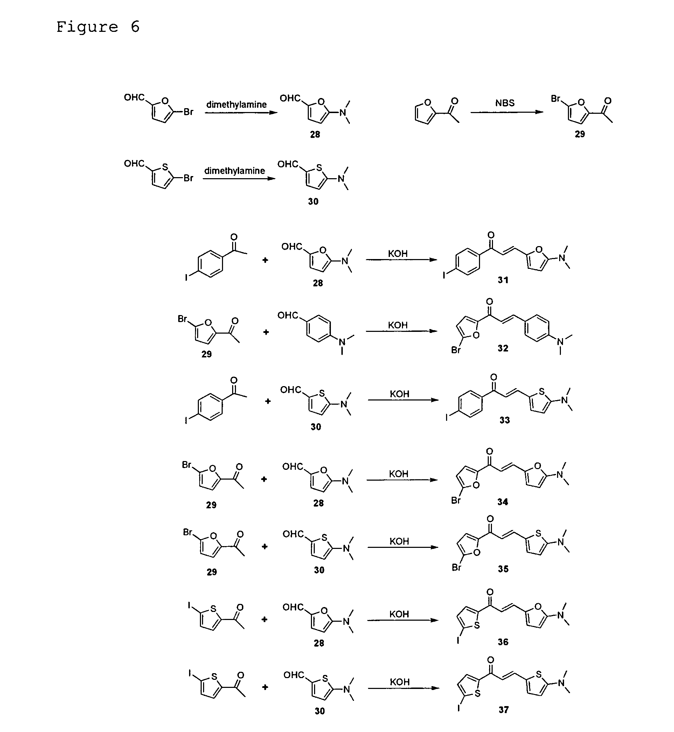

Figures 3 to 8 show synthesis pathways of chalcone derivatives. The chalcone skeleton was formed by an aldol condensation reaction. A nitro group of a chalcone derivative was reduced with tin(II) chloride. An amino group was monomethylated with methyl iodide under a basic condition. Further, an amino group was dimethylated according to a previously reported method. Each bromo compound was converted to a tributyltin compound by a reaction with bis(tributyltin) using palladium as a catalyst. These tributyltin compounds were converted to iodine compounds by a reaction with iodine.

### (2) ¹²⁵I labeling experiment

Figure 9 shows ¹²⁵I labeling pathways of chalcone derivatives. ¹²⁵I labeling was performed using hydrogen peroxide as an oxidizing agent, and a target ¹²⁵I-labeled compound was obtained by a tin-iodine exchange reaction. Absorbance of nonradioactive compounds at 254 nm was analyzed by reverse phase HPLC beforehand, and compounds having matching retention time were isolated and purified to obtain target ¹²⁵I-labeled compounds with radiochemical purity of 98% or higher.

### (3) Examination of binding affinity of chalcone derivatives for Aβ(1-42) aggregate

Various synthesized chalcone derivatives were subjected to a binding inhibition experiment using [¹²⁵I]CL-NMe₂ as a radioactive ligand to calculate inhibition constants [Kᵢ values]. Table 13 shows Kᵢ values obtained as a result of the experiment.

**[Table 13]**

| Ki values calculated by inhibition experiment | | | | | |
|---|---|---|---|---|---|
| compound | *K*ᵢ (nM)^{a} | compound | *Kᵢ* (nM)^{a} | compound | *Kᵢ* (nM)^{a} |
| 4^{b} | 248.1 ± 55.8 | 25 | 92.7 ± 11.0 | 39 | - |
| 7 | 23.9 ± 3.6 | 26 | 796.6 ± 316.4 | 40 | 1160.0 ± 153.5 |
| 10 | 13.3 ± 1.9 | 27 | >10000 | 41 | 157.3 ± 80.8 |
| | | | | 42 | 222.8 ± 76.1 |
| 12 | 120.6 ± 40.3 | 31 | 1131.8 ± 344.2 | | |
| 13 | 14.1 ± 0.6 | 32 | 126.3 ± 12.5 | 44 | 476.4 ± 47.9 |
| 14 | 3.9 ± 0.4 | 33 | 112.5 ± 10.1 | 45 | 198.4 ± 48.6 |
| | | | | 46 | 106.3 ± 7.1 |
| 21 | 151.4 ± 15.8 | 34 | >10000 | | |
| 22 | 907.6 ± 212.3 | 35 | 2648.3 ± 221.5 | CL-NH2 | 104.8 ± 12.0 |
| 23 | 124.7 ± 9.2 | 36 | 1608.3 ± 84.5 | CL-NHMe | 6.3 ± 1.6 |
| 24 | 102.2 ± 15.9 | 37 | 137.1 ± 3.4 | CL-NMe2 | 2.9 ± 0.3 |

| | | | | | |
|---|---|---|---|---|---|
| a: Values were expressed with mean ± standard error of 3 experiments. b: Values were expressed with mean ± standard error of 6 experiments. | | | | | |

It was demonstrated that the binding affinity of chalcone derivatives for the Aβ(1-42) aggregate improved depending on the type of the substituent, specifically, in the order of NH₂ < NHMe < NMe₂. Furthermore, it was shown that the derivatives in which a substituent was bound at the 4'th position (Compounds 4, 7, and 10) had a decreased binding affinity for the Aβ(1-42) aggregate as compared with the derivatives in which a substituent was bound at the 4th position (CL-NH₂, CL-NHMe, and CL-NMe₂). It was shown that, among the derivatives in which the same substituent (-NMe₂) was bound, the derivative in which thiophene was introduced into Ring B (Compound 14) had a lower Kᵢ value than the derivatives in which thiophene was introduced into Ring A (Compounds 33 and 46), suggesting that binding affinity for the Aβ(1-42) aggregate is affected by the types of aromatic heterocyclic rings introduced into Rings A and B.

When the binding affinity for the Aβ(1-42) aggregate was compared among the compounds having a dimethylamino group in the side chain thereof (CL-NMe₂, Compounds 14, 31, 32, 33, 34, 35, 36, and 37), the binding affinity for the Aβ(1-42) aggregate was increased in the order of 4-dimethylaminofuran (Compounds 31, 34, and 36) < 4-dimethylaminothiophene (Compounds 33, 35, and 37), and < 4-dimethylaminobenzene (CL-NMe₂ and Compounds 14 and 32) in Ring A and in the order of 2-bromofuran (Compounds 32, 34, and 35) < 2-iodothiophene (14,36,37) ≈ 4-iodobenzene (CL-NMe₂ and Compounds 31 and 33) in Ring B.

Among the derivatives in which two substituents were bound to Ring A (Compounds 39, 40, 41, and 42), no compound showed a high binding affinity for the Aβ(1-42) aggregate.

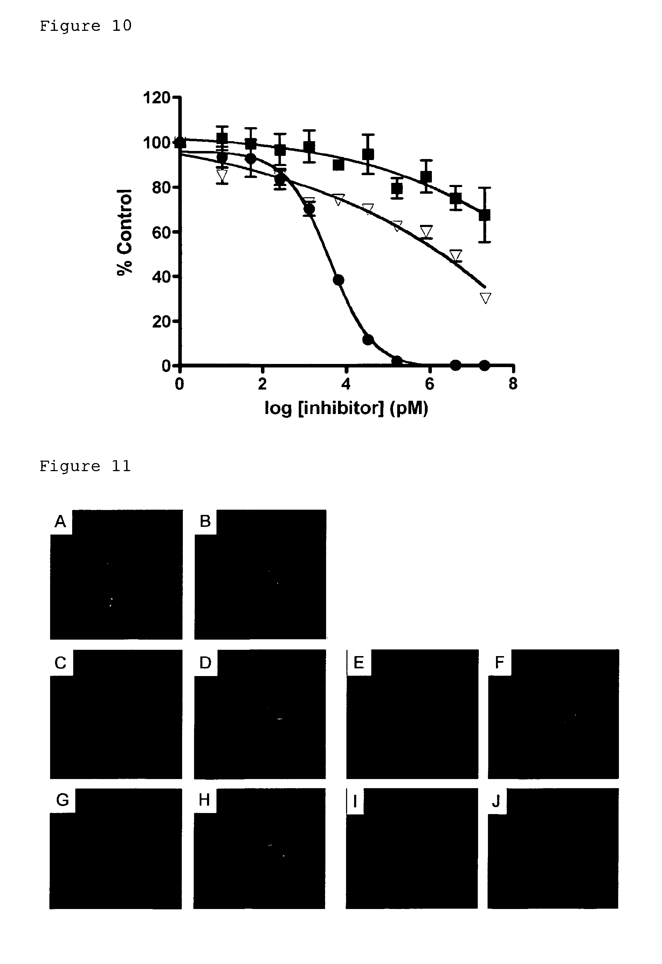

Furthermore, to examine the binding site of chalcone derivatives in the Aβ(1-42) aggregate, a binding inhibition experiment was performed using [¹²⁵I]CL-NMe₂ as a radioactive ligand and congo red, thioflavine T, or Compound 14 as an inhibitor (Figure 10). As a result, Compound 14 inhibited the binding of [¹²⁵I] CL-NMe₂ to the Aβ(1-42) aggregate with Kᵢ of 4 nM, whereas congo red or thioflavine T did not inhibit binding of [¹²⁵I]CL-NMe₂ to the Aβ(1-42) aggregate to a large extent, suggesting that binding sites of chalcone derivatives in the Aβ(1-42) aggregate do not match those of congo red and thioflavine T.

### (4) In-vivo radioactivity distribution experiment of ¹²⁵I-labeled chalcone derivatives in normal mice

An in-vivo radioactivity distribution experiment of ¹²⁵I-labeled chalcone derivatives was performed using 4 or 5-week-old normal mice. Table 14 shows the results of in-vivo radioactivity distributions after intravenous injection of [¹²⁵I]Compounds 7, 10, 13, and 14 to the tail of mice.

**[Table 14]**

| In-vivo radioactivity distribution experiment in normal mice | | | | | | | | | |
|---|---|---|---|---|---|---|---|---|---|
| [¹²⁵I]7 | | | | | [¹²⁵I]10 | | | | |
| | 2 min | 10 min | 30 min | 90 min | | 2 min | 10 min | 30 min | 60 min |
| blood | 1.76 (0.27) | 1.43 (0.30) | 1.15 (0.57 | 1.51 (0.26) | blood | 1.87 (0.23) | 1.89 (0.61) | 1.51 (0.40) | 1.38 (0.54) |
| liver | 10.82 (2.61) | 9.42 (1.92) | 6.77 (1.21) | 6.01 (1.81) | liver | 13.99 (1.57) | 10.29 (2.06) | 6.69 (1.21) | 7.66 (2.45) |
| kidney | 6.20 (0.93) | 4.58 (0.87) | 3.92 (1.05) | 4.04 (1.10) | kidney | 8.30 (0.85) | 4.51 (1.29) | 4.10 (1.15) | 4.59 (1.57) |
| intestine | 2.52 (0.81) | 7.72 (1.31) | 17.52 (1.74) | 18.47 (2.31) | intestine | 2.69 (0.13) | 9.27 (1.36) | 12.50 (2.49) | 20.09 (5.48) |
| spleen | 2.21 (0.48) | 1.47 (0.27) | 0.94 (0.46) | 0.71 (0.19) | spleen | 2.35 (0.35) | 1.52 (0.19) | 0.62 (0.16) | 0.89 (0.12) |
| lung | 11.10 (3.36) | 4.94 (1.73) | 2.22 (0.58) | 1.57 (0.23) | lung | 6.02 (0.60) | 3.92 (0.76) | 1.95 (0.25) | 1.71 (0.29) |
| stomach ^{a} | 1.06 (0.67) | 0.87 (0.38) | 0.76 (0.11) | 1.14 (0.48) | stomach ^{a} | 0.76 (0.13) | 0.89 (0.28) | 0.73 (0.23) | 2.11 (1.24) |
| pancreas | 4.93 (1.01) | 2.99 (1.76) | 1.04 (0.35) | 0.78 (0.16) | pancreas | 3.85 (1.97) | 2.12 (0.31) | 0.85 (0.10) | 0.84 (0.22) |
| heart | 5.03 (0.79) | 1.99 (0.26) | 0.98 (0.20) | 0.82 (0.14) | heart | 5.23 (0.61) | 2.05 (0.37) | 1.10 (0.16) | 0.84 (0.26) |
| brain | 3.07 (0.57) | 2.03 (0.41) | 0.69 (0.09) | 0.40 (0.08) | brain | 2.77 (0.37) | 2.04 (0.26) | 0.63 (0.06) | 0.42 (0.11) |
| | | | | | | | | | |

| [¹²⁵I]13 | | | | | [¹²⁵I]14 | | | | |
|---|---|---|---|---|---|---|---|---|---|
| | 2 min | 10 min | 30 min | 60 min | | 2 min | 10 min | 30 min | 60 min |
| blood | 3.00 (0.83) | 3.68 (0.70) | 2.29 (0.32) | 2.99 (0.49) | blood | 4.91 (0.71) | 3.58 (0.35) | 2.92 (0.60) | 2.63 (0.45) |
| liver | 8.41 (1.24) | 13.29 (1.65) | 5.10 (1.12) | 4.32 (0.75) | liver | 9.57 (2.00) | 7.10 (1.08) | 5.95 (1.19) | 4.96 (1.27) |
| kidney | 4.50 (1.00) | 6.59 (1.34) | 3.18 (0.80) | 2.52 (0.48) | kidney | 7.41 (1.54) | 4.66 (0.88) | 4.31 (1.01) | 2.41 (0.35) |
| intestine | 1.60 (0.32) | 3.86 (0.20) | 4.36 (1.49) | 6.286 (1.78) | intestine | 1.73 (0.21) | 2.57 (0.53) | 4.05 (0.99) | 4.69 (1.64) |
| spleen | 2.06 (0.49) | 4.00 (1.55) | 2.06 (0.28) | 2.52 (0.36) | spleen | 4.05 (0.83) | 5.48 (1.82) | 5.01 (1.72) | 3.03 (0.31) |
| lung | 5.61 (1.33) | 6.33 (1.42) | 3.17 (0.52) | 3.11 (0.66) | lung | 8.29 (0.99) | 5.17 (0.69) | 3.53 (0.35) | 2.87 (0.46) |
| stomach ^{a} | 1.48 (0.39) | 5.13 (1.67) | 9.94 (1.69) | 14.32 (1.81) | stomach | 1.21 (0.27) | 5.29 (1.24) | 9.81 (6.47) | 8.73 (4.21) |
| pancreas | 3.73 (0.67) | 2.53 (0.72) | 1.48 (0.34) | 1.76 (0.10) | pancreas | 3.82 (0.57) | 1.94 (0.86) | 1.95 (0.32) | 1.86 (0.27) |
| heart | 3.55 (0.57) | 2.48 (0.36) | 1.45 (0.17) | 1.43 (0.29) | heart | 6.05 (0.95) | 3.02 (0.60) | 1.48 (0.78) | 1.65 (0.24) |
| brain | 2.56 (0.44) | 1.13 (0.16) | 0.28 (0.06) | 0.15 (0.03) | brain | 2.46 (0.30) | 0.7 (0.31) | 0.31 (0.04) | 0.21 (0.02) |

| | | | | | | | | | |
|---|---|---|---|---|---|---|---|---|---|
| a: Expressed with a percent of the dose. Each value is the mean of 3 to 5 mice. Other values were expressed with a percent of the dose/g. | | | | | | | | | |

All of [¹²⁵I] Compounds 7, 10, 13, and 14 showed high penetration into the brain at 2 min after administration (2.5 to 3.1% ID/g). Further, since radioactivity that remained in the brain at 30 min after administration was only 0.3 to 0.7% ID/g, these four types of chalcone derivatives were found to show rapid radioactivity clearance from the normal brain.

The ratios of radioactivity in the brain at 2 min after administration to that at 30 min after administration were 17.9% for [¹²⁵I]CL-NHMe, 13.6% for [¹²⁵I]CL-NMe₂, 22.5% for [¹²⁵I] Compound 7, 22.7% for [¹²⁵I]Compound 10, 10.9% for [¹²⁵I] Compound 13, and 12.6% for [¹²⁵I] Compound 14, suggesting that [¹²⁵I] Compounds 7 and 10 had delayed radioactivity clearance from the brain as compared with [¹²⁵I] CL-NHMe and [¹²⁵I] CL-NMe₂. On the other hand, [¹²⁵I] Compounds 13 and 14 showed rapid radioactivity clearance as compared with CL-NHMe and CL-NMe₂ as well as [¹²⁵I]Compounds 7 and 10. Above all, [¹²⁵I]Compound 13 showed the most rapid radioactivity clearance among the chalcone derivatives synthesized so far. However, [¹²⁵I]Compounds 13 and 14 showed instability in the organism such as radioactivity accumulation in the stomach, retention in blood, and deiodination in the organism.

### (5) Fluorescence staining experiment using brain tissue sections of Alzheimer's disease model mice

All the chalcone derivatives showed a binding property to senile plaque amyloid and vascular amyloid on the brain tissue sections of the Alzheimer's disease model mice (Figure 11). Binding of Compound 14 to amyloid is stronger than that of fluorine-containing chalcone derivatives, which reflected the results of the *in-vitro* inhibition experiment (Tables 13 and 17).

### [Reference Example 1]

### Experimental methods

### Reagents and instrument

As a radioactive iodine-125 (¹²⁵I), IODINE-125 (74 MBq) manufactured by Amersham Biosciences was used. Reverse phase HPLC was performed at a flow rate of 1.0 mL/min using Cosmosil 5C₁₈-AR Column (4.6 x 150 mm) manufactured by Nacalai Tesque Inc. and ultrapure water (A) and acetonitrile (B) (A:B = 40:60) as an elution solvent. ¹H-NMR was measured using Varian Gemini 300 and tetramethylsilane as an internal standard substance. Mass spectrometry was performed using JEOL IMS-DX300. Amyloid β protein (Human, 1-40) [HCl form] and Amyloid β protein (Human, 1-42) [TFA form] were purchased from Peptide Institute, Inc., and special grade reagents were used as other reagents.

### (1) Synthesis of chalcone derivatives

### Synthesis of (E)-1-(3-bromophenyl)-3-(4-hydroxy-3-methoxyphenyl)prop-2-en-1-one

1.99 g (10 mmol) of 3-bromoacetophenone was dissolved in ethanol (10 mL), and the mixture was added to 10% aqueous potassium hydroxide (30 mL) with ice cooling. The mixture was stirred for 15 min, 1.52 g (10 mmol) of o-vanillin was added as a solid, and the mixture was further stirred for 15 min with ice cooling. The temperature was returned to room temperature, the mixture was stirred for 4 h, then the precipitated crystals were filtered by suction, the filtrate was evaporated under reduced pressure, and the residue was subjected to silica gel chromatography using ethyl acetate/hexane (1/4) as an elution solvent to obtain target Compound 5. Yield 470 mg (yield rate 14.1%). ¹H NMR (300 MHz, CDCl₃) δ 8.14 (s, 1H), 8.04 (d, J = 15.9 Hz, 1H), 7.90-7.98 (m, 1H), 7.66-7.71 (m, 2H), 7.32-7.40 (m, 1H), 7.15-7.20 (m, 1H), 6.89-6.91 (m, 2H), 6.31 (s, 1H), 3.94 (s, 3H).

### Synthesis of (E)-1-(3-(tributylstannyl)phenyl)-3-(4-hydroxy-3-methoxyphenyl)prop-2-en-1-one

To a solution of Compound 5 (370 mg, 1.11 mmol) in dioxane (10 mL) were added bis(tributyltin) (1 mL), tetrakis(triphenylphosphine)palladium (75 mg), and triethylamine (5 mL), and the mixture was heated to reflux for 12 h. The reaction solvent was evaporated under reduced pressure, and the residue was subjected to silica gel column chromatography using ethyl acetate/hexane (1/9) as an elution solvent to obtain target Compound 6. Yield 161 mg (yield rate 26.7%). ¹H NMR (300 MHz, CDCl₃) δ 8.10 (s, 1H), 8.03 (d, J = 15.9 Hz, 1H), 7.90-7.95 (m, 1H), 7.73 (d, J = 15.9 Hz, 1H), 7.62-7.68 (m, 1H), 7.41-7.49 (m, 1H), 7.15-7.20 (m, 1H), 6.87-6.89 (m, 2H), 6.24 (s, 1H), 3,94 (s, 3H), 0.86-1.65 (m, 27H).

### Synthesis of (E)-3-(4-hydroxy-3-methoxyphenyl)-1-(3-iodophenyl)prop-2-en-1-one

A solution of iodine in chloroform (2 mL, 1.1 mM solution) was added to a solution of Compound 6 (150 mg, 0.28 mmol) in chloroform (15 mL) at room temperature. The mixture was reacted at room temperature for 30 min, and then saturated aqueous sodium hydrogen sulfite (20 mL) was added to terminate the reaction. The chloroform layer was separated and dried with sodium sulfate, the solvent was evaporated under reduced pressure, and then the residue was subjected to silica gel chromatography using ethyl acetate/hexane (1/5) as an elution solvent to obtain target Compound 7. Yield 47 mg (yield rate 44.8%).

### Synthesis of (E)-1-(4-bromophenyl)-3-(4-nitrophenyl)prop-2-en-1-one

1.99 g (10 mmol) of 4-bromoacetophenone was dissolved in ethanol (10 mL), and the mixture was added to 10% aqueous potassium hydroxide (30 mL) with ice cooling. The mixture was stirred for 15 min, then 1.51 g (10 mmol) of 4-nitrobenzaldehyde was added as a solid, and the mixture was further stirred for 15 min with ice cooling. The temperature was returned at room temperature, the mixture was stirred for 4 h, followed by addition of ethyl acetate (50 mL), the precipitated crystals were filtered by suction and thoroughly washed with ethyl acetate to obtain target Compound 8. Yield 1.27 g (yield rate 38.2%).

### Synthesis of (E)-3-(4-aminophenyl)-1-(4-bromophenyl)prop-2-en-1-one

Tin(II) chloride (5.0 g, 26.4 mmol) was slowly added to a solution of Compound 8 (1.0 g, 3.01 mmol) in ethanol (15 mL) with stirring, and the mixture was heated to reflux for 2 h. After termination of the reaction, 1 N aqueous sodium hydroxide (150 mL) was added to the reaction solution, and the mixture was extracted with 50 mL (150 mL) of ethyl acetate. The mixture was dried with anhydrous sodium sulfate, then the solvent was evaporated under reduced pressure, and the residue was subjected to silica gel chromatography using ethyl acetate/hexane (1/3) as an elution solvent to obtain target Compound 9. Yield 556 mg (yield rate 61.1%).

### Synthesis of (E)-1-(4-bromophenyl)-3-(4-(dimethylamino)phenyl)prop-2-en-1-one

Sodium cyanoborohydride (250 mg, 3.98 mmol) was slowly added to a solution of Compound 9 (250 mg, 0.83 mmol) and paraformaldehyde (400 mg, 13.4 mmol) in acetic acid (15 mL) with stirring, and the mixture was stirred at room temperature for 3 h. After termination of the reaction, 50 mL of 1 N aqueous sodium hydroxide was added to the reaction solution, and the mixture was extracted with 50 mL (25 mL x 2) of chloroform. The mixture was dried with anhydrous sodium sulfate, then the solvent was evaporated under reduced pressure, and the residue was subjected to silica gel chromatography using ethyl acetate/hexane (1/12) as an elution solvent to obtain target Compound 10. Yield 236 mg (yield rate 86.1%).

### Synthesis of (E)-1-(4-(tributylstannyl)phenyl)-3-(4-(dimethylamino)phenyl)prop-2-en-1-one

To a solution of Compound 10 (220 mg, 0.67 mmol) in dioxane (10 mL) were added bis(tributyltin) (1 mL), tetrakis(triphenylphosphine)palladium (84 mg), and triethylamine (5 mL), and the mixture was heated to reflux for 2 h. The reaction solvent was evaporated under reduced pressure, and the residue was subjected to silica gel column chromatography using ethyl acetate/hexane (1/18) as an elution solvent to obtain target Compound 11. Yield 43 mg (yield rate 11.9%). ¹H NMR (300 MHz, CDCl₃) δ 7.91 (d, J = 9.0 Hz, 2H), 7.80 (d, J = 15.9 Hz, 1H), 7.51-7.65 (m, 3H), 7.35 (d, J = 15.9 Hz, 1H), 6.69 (d, J = 9.0 Hz, 2H), 3.05 (s, 6H), 0.88-1.59 (m, 27H).

### Synthesis of (E)-3-(4-dimethylamino)phenyl)-1-(4-iodophenyl)prop-2-en-1-one

A solution of iodine in chloroform (2 mL, 1.1 mM solution) was added to a solution of Compound 11 (4 mg, 0.07 mmol) in chloroform (5 mL) at room temperature. The mixture was reacted at room temperature for 30 min, and saturated aqueous sodium hydrogen sulfite (20 mL) was added to terminate the reaction. The chloroform layer was separated and dried with sodium sulfate, the solvent was evaporated under reduced pressure, and then the residue was subjected to silica gel chromatography using ethyl acetate/hexane (1/9) as an elution solvent to obtain target Compound 12. Yield 13 mg (yield rate 46.6%). ¹H NMR (300 MHz, CDCl₃) δ 7.82-7.88 (m, 3H), 7.71-7.78 (m, 2H), 7.53 (d, J = 9.0 Hz, 2H), 7.20-7.30 (m, 2H), 6.68 (d, J = 9.0 Hz, 2H), 3.05 (s, 6H).

### (2) Iodine labeling experiment

50 µL of a solution of a tributyltin precursor (1 mg/mL) in ethanol, 50 µL of 1 N hydrochloric acid, and Na[¹²⁵I]Compound 1 (1 to 5 µCi) were placed in a glass vial, and 50 µL (3% (w/v)) of aqueous hydrogen peroxide was added finally. The mixture was allowed to stand at room temperature for 10 min, and then 100 µL of saturated aqueous sodium hydrogen sulfite was added to terminate the reaction. The mixture was neutralized with saturated aqueous sodium hydrogencarbonate and then extracted with ethyl acetate (1 mL x 2). The mixture was dehydrated by passing through a Pasteur pipette containing sodium sulfate, ethyl acetate was evaporated with nitrogen, and then the reside was dissolved in ethanol and purified by reverse phase HPLC (water:acetonitrile = 40:60). Nonradioactive compounds as preparations were analyzed by HPLC for absorbance at 254 nm, target compounds having matching absorbance were separated, and acetonitrile was evaporated. Radioactivities were measured, specific radioactivities of the target compounds were calculated from specific radioactivity (2200 Ci/mmol) of ¹²⁵I.

### (3) In-vitro binding experiment of chalcone derivatives using Aβ aggregate

The Aβ aggregate was dissolved in a buffer containing 10 mM sodium phosphate and 1 mM EDTA (pH 7.4) at a concentration of 0.5 mg/mL, and the mixture was incubated at 37°C for 36 to 42 h. The binding experiment was performed using 12 x 75 mm borosilicate glass tubes. 900 µL of 10% ethanol solution, 50 µL (57 nM) of an Aβ(1-40) aggregate solution, and 50 µL of [¹²⁵I]Compound 7 or 12 having various concentrations were mixed, and the mixture was allowed to stand at room temperature for 3 h. Aβ aggregate-bound chalcone derivatives and nonbound chalcone derivatives were separated with a Brandel M-24R cell harvester using Whatman GF/B filters. The radioactivities of substances remaining in the filter used for filtration were measured with a γ counter.

### (4) In-vivo radioactivity distribution experiment in mice

A radioactive iodine-labeled compound (Compound 7) was diluted with physiological saline containing 5 to 10% ethanol. Three to five 5-week-old male ddY mice (25 to 30 g) per group were decapitated at 2, 10, 30, or 60 min after intravenous administration of 100 µL (0.5 to 1 µCi) of the labeled compound, blood was collected, organs were removed, and weights and radioactivities thereof were measured.

### Experimental results

### (1) Synthesis of chalcone derivatives

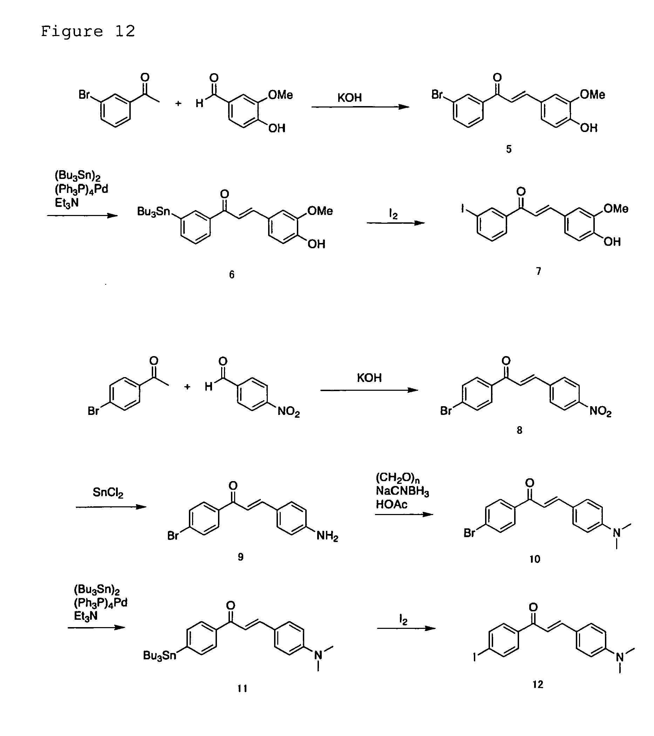

Figure 12 shows synthesis pathways of chalcone derivatives. For chalcone derivatives, the basic chalcone skeleton was formed by a condensation reaction of an acetophenone compound and an aldehyde compound in the presence of potassium. The nitro group of Compound 8 was reduced using tin(II) chloride as a reducing agent. The generated amino group was dimethylated by a usual method. Bromo compounds (Compounds 5 and 10) were converted to tributyltin compounds by a reaction with bis(tributyltin) using palladium as a catalyst. These tributyltin compounds were converted to Compounds 7 and 12 by a reaction with iodine. It is noted that Compounds 7 and 12 are not included in the compounds represented by the general formula (I) because the group corresponding to R¹ is a phenyl group, not an aromatic heterocyclic ring.

### (2) Iodine labeling experiment

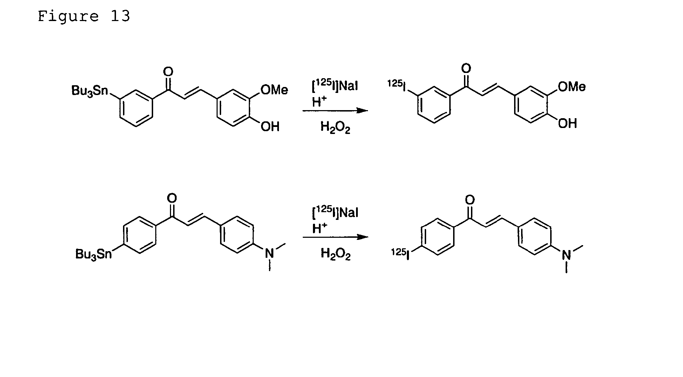

As shown in Figure 13, radioactive iodine labeling was performed by a tin-iodine exchange reaction using hydrogen peroxide as an oxidizing agent, and a target radioactive iodine-125 labeled compound was obtained at a radiochemical yield of 50-80%. After the labeling reaction, the compound was separated and purified by reverse phase HPLC to obtain a carrier-free labeled compound with a radiochemical yield of 98% or higher.

### (3) In-vitro binding experiment using Aβ aggregate

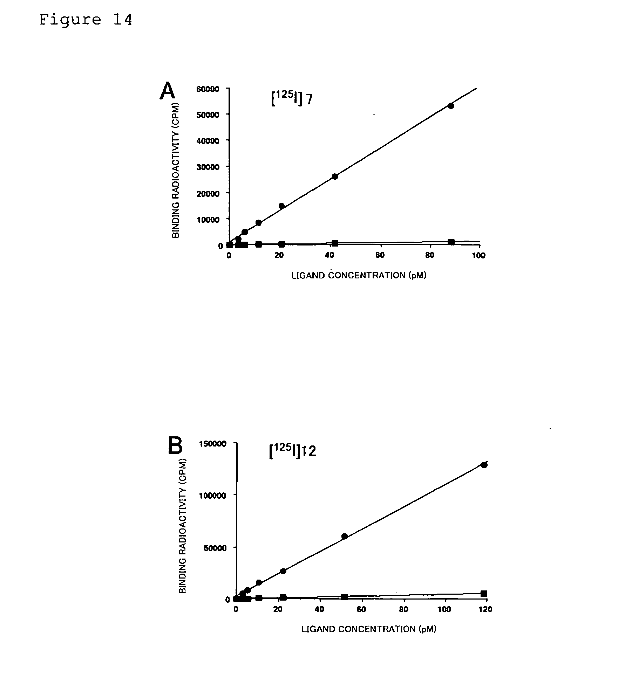

Figure 14 shows the results obtained when [¹²⁵I]Compounds 7 and 12 having various concentrations were reacted in the presence of or in the absence of the Aβ aggregate, the reaction mixture was filtered through a cell harvester, and the radioactivity remaining on the filter paper was measured. In the absence of the Aβ aggregate, the radioactivity remaining on the filter paper was low, but, after the reaction in the presence of the Aβ aggregate, [¹²⁵I]Compounds 7 and 12 showed markedly high values. These results revealed that both [¹²⁵I]Compounds 7 and 12, chalcone derivatives, had a strong binding property to the Aβ aggregate.

### (4) In-vivo radioactivity distribution experiment in mice

Table 15 shows the results of in-vivo radioactivity distributions after administration of chalcone derivatives to normal mice.

**[Table 15]**

| In-vivo distributions of radioactivity of [¹²⁵I]Compound 7 after intravenous administration^{(a)} | | | | |
|---|---|---|---|---|
| | Time after administration (min) | | | |
| Tissue | 2 | 10 | 30 | 60 |
| | [¹²⁵I] Compound 7 | | | |
| Blood | 4.90 (0.52) | 3.80 (0.91) | 1.84 (0.31) | 1.15 (0.44) |
| Liver | 15.71 (1.27) | 18.55 (2.68) | 7.67 (1.68) | 4.05 (0.61) |
| Kidneys | 11.99 (3.60) | 9.16 (2.76) | 5.00 (2.27) | 4.31 (0.81) |
| Intestines | 2.50 (0.31) | 10.52 (1.82) | 20.60 (6.13) | 29.31 (3.26) |
| Spleen | 2.57 (0.42) | 1.80 (0.30) | 1.03 (0.04) | 0.88 (0.15) |
| Heart | 5.58 (0.99) | 2.88 (1.02) | 1.44 (0.34) | 1.38 (0.34) |
| Brain | 2.52 (0.20) | 1.28 (0.17) | 0.40 (0.11) | 0.35 (0.05) |

| | | | | |
|---|---|---|---|---|
| ^{(a)} Expressed with percents of the dose/g. Each value is the mean value of 3 to 5 mice. The value in parentheses is standard deviation. | | | | |

As shown in Table 15, penetration of the chalcone derivative ([¹²⁵I]Compound 7) into the brain after administration was confirmed, and it was shown that the chalcone derivative was washed out immediately thereafter.

The above reference example results suggest that Compounds 7 and 12 can be used as an imaging probe of amyloid β. A compound in which a benzene ring closer to the carbonyl group in Compound 7 or 12 is substituted with a pyridine ring is included in the compound represented by the general formula (I). Ono et al. reported that, when a benzene ring in a stilbene derivative binding to amyloid β protein is substituted with a pyridine ring, lipid solubility is decreased, and clearance from the brain is improved with the binding property to amyloid β protein being maintained (M. Ono et al., Nuclear Medicine and Biology 32 (2005) 329-335). This report of Ono et al. suggests that the above-mentioned pyridine-substituted compounds derived from Compounds 7 and 12 similarly have a binding property to amyloid β protein as with Compounds 7 and 12, and more rapid clearance from the brain than these compounds.

### [Reference Example 2]

### Experimental methods

### Reagents and instrument

Amyloid β protein (Human, 1-42) [TFA form] was purchased from Peptide Institute, Inc., and special grade reagents were used as other reagents. ¹H-NMR was measured using Varian Gemini 300 and tetramethylsilane as an internal standard substance.

### (1) Synthesis of fluorine-containing chalcone derivatives

### Synthesis of (E)-3-(4-(dimethylamino)phenyl)-1-(4-hydroxyphenyl)prop-2-en-1-one (1)

4-Hydroxyacetophenone (1.36 g, 10 mmol) and 4-dimethylaminobenzaldehyde (1.0 g, 6.7 mmol) was dissolved in 15 mL of ethanol. 10% KOH (30 mL) was added, and the mixture was reacted at 100°C for 24 h. The mixture was extracted with ethyl acetate, and then the solvent was evaporated under reduced pressure. The residue was washed with ethyl acetate:n-hexane = 1:2 and filtered, and the resulting filtrate was evaporated under reduced pressure to obtain 1.50 g (yield rate 84%) of Compound 1. ¹H-NMR (CD₃OD) δ: 3.04 (s, 6H), 6.76 (d, J = 8.7 Hz, 2H), 6.88 (d, J = 8.7 Hz, 2H), 7.50 (d, J = 15.3 Hz, 1H), 7.59 (d, J = 9 Hz, 2H), 7.72 (d, J = 15.3 Hz, 1H), 7.98 (d, J = 8.7 Hz, 2H), (DMSO-d₆) δ: 2.99 (s, 6H), 6.74 (d, J = 8.7 Hz, 2H), 6.88 (d, J = 8.4 Hz, 2H), 7.62 (s, 2H), 7.68 (d, J = 8.7 Hz, 2H), 8.03 (d, J = 8.7 Hz, 2H), 10.30 (s, 1H). EI-MS: m/z 267 (M⁺)

### Synthesis of (E-)1-(4-(2-hydroxyethoxy)phenyl)-3-(4-(dimethylamino)phenyl)prop-2-en-1-one (2)

Compound 1 (500 mg, 1.87 mmol) and ethylene chlorohydrin (124.6 µL, 1.87 mmol) were dissolved in N,N-dimethylformamide (DMF) (5 mL), followed by addition of K₂CO₃ (775.3 mg, 5.61 mmol), and the mixture was reacted at 100°C for 18 h. Purified water was added, the mixture was extracted with chloroform and dehydrated by adding Na₂SO₄, and then the solvent was evaporated under reduced pressure. The residue was purified by silica gel column chromatography using ethyl acetate:n-hexane = 1:1 as a developing solvent to obtain 422 mg (yield rate 73%) of Compound 2. ¹H-NMR (CDCl₃ δ: 3.04 (s, 6H), 4.00-4.01 (m, 2H), 4.17 (t, J = 4.8 Hz, 2H), 6.69 (d, J = 9.0 Hz, 2H), 6.99 (d, J = 6.9 Hz, 2H), 7.35 (d, J = 15.3 Hz, 1H), 7.55 (d, J = 9.0 Hz, 2H), 7.79 (d, J = 15.3 Hz, 1H), 8.02 (d, J = 9.3 Hz, 2H).

### Synthesis of (E)-1-(4-(2-fluoroethoxy)phenyl)-3-(4-(dimethylamino)phenyl)prop-2-en-1-one (3)

Compound 2 (100 mg, 0.32 mmol) was dissolved in ethylene glycol dimethyl ether (5 mL), and the mixture was stirred in the acetone bath with cooling. Diethylaminosulfur trifluoride (DAST) (84.55 µL, 0.64 mmol) was slowly added while stirring with cooling. Aqueous potassium carbonate was slowly added to terminate the reaction, the mixture was extracted with chloroform and dehydrated by adding Na₂SO₄, and then the solvent was evaporated under reduced pressure. The residue was purified by preparative TLC using ethyl acetate:n-hexane = 1:3 as a developing solvent to obtain 39 mg (yield rate 39%) of Compound 3. ¹H-NMR (CDCl₃) δ: 3.09 (s, 6H), 4.30 (d, t, J₁ = 27.6 Hz, J₂ = 4.2 Hz, 2H), 4.79 (d, t, J₁ = 47.4 Hz, J₂ = 4.2 Hz, 2H), 6.70 (d, J = 8.7 Hz, 2H), 7.00 (d, J = 9.0 Hz, 2H), 7.35 (d, J = 15.6 Hz, 1H), 7.55 (d, J = 9.0 Hz, 2H), 7.79 (d, J = 15.3 Hz, 1H), 8.03 (d, J = 9.0 Hz, 2H). EI-MS: m/z 313 (M⁺)

### Synthesis of (E)-1-(4-(2-(2-hydroxyethoxy)ethoxy)phenyl)-3-(4-(dimethylamino)phenyl)prop-2-en-1-one (4)

Compound 1 (500 mg, 1.87 mmol) and ethylene glycol mono-2-chloroethyl ether (237 µL, 2.24 mmol) were dissolved in DMF (5 ml), followed by addition of K₂CO₃ (775.3 mg, 5.61 mmol), and the mixture was reacted at 100°C for 18 h. Purified water was added, the mixture was extracted with chloroform and dehydrated by adding Na₂SO₄, and then the solvent was evaporated under reduced pressure. The residue was purified by silica gel column chromatography using ethyl acetate:n-hexane = 2:1 as an elution solvent to obtain Compound 4. ¹H-NMR (CDCl₃) δ: 3.05 (s, 6H), 3.69 (t, J = 4.8 Hz, 2H), 3.78 (s, 2H), 3.91 (t, J = 4.8 Hz, 2H), 4.23 (t, J = 4.8 Hz, 2H), 6.70 (d, J = 9.0 Hz, 2H), 6.99 (d, J = 9.0 Hz, 2H), 7.35 (d, J = 15.3 Hz, 1H), 7.55 (d, J = 8.7 Hz, 2H), 7.79 (d, J = 15.6 Hz, 1H), 8.02 (d, J = 9.0 Hz, 2H).

### Synthesis of (E)-1-(4-(2-(2-fluoroethoxy)ethoxy)phenyl)-3-(4-(dimethylamino)phenyl)prop-2-en-1-one (5)

Compound 4 (100 mg, 0.28 mmol) was dissolved in ethylene glycol dimethyl ether (4 mL), and the mixture was stirred with ice cooling. DAST (74.6 µL, 0.56 mmol) was slowly added while stirring with cooling, and the temperature was returned to room temperature after 10 min. Aqueous potassium carbonate was slowly added to terminate the reaction, the mixture was extracted with chloroform and dehydrated by adding Na₂SO₄, and then the solvent was evaporated under reduced pressure. The residue was purified by preparative TLC using ethyl acetate:n-hexane = 1:1 as a developing solvent to obtain 28 mg (yield rate 28%) of Compound 5. ¹H-NMR (CDCl₃) δ: 3.04 (s, 6H), 3.77-3.94 (m, 4H) 4.21-4.24 (m, 3H) 4.61 (d, t, J₁ = 47.4 Hz, J₂ = 4.2 Hz, 1H), 6.69 (d, J = 9.3 Hz, 2H), 6.99 (d, J = 8.7 Hz, 2H), 7.35 (d, J = 15.3 Hz, 2H), 7.55 (d, J = 9.0 Hz, 2H), 7.78 (d, J = 15.6 Hz, 2H), 8.02 (d, J = 9.0 Hz, 2H). EI-MS: m/z 357 (M⁺)

### Synthesis of (E)-1-(4-(2-(2-(2-hydroxyethoxy)ethoxy)ethoxy)phenyl)-3-(4-dimethylamino)phenyl)prop-2-en-1-one (6)

Compound 1 (355 mg, 1.33 mmol) and 2-[2-(2-chloroethoxy)ethoxy]ethanol (232 (µL, 1.60 mmol) were dissolved in DMF (4 mL), followed by addition of K₂CO₃ (551.4 mg, 3.99 mmol), and the mixture was reacted at 100°C for 6 h. Purified water was added, the mixture was extracted with chloroform and dehydrated with Na₂SO₄, and then the solvent was evaporated under reduced pressure. The residue was purified by silica gel column chromatography using ethyl acetate:n-hexane = 6:1 as an elution solvent to obtain 429 mg (yield rate 82.6%) of Compound 6. ¹H-NMR (CDCl₃) δ: 3.04 (s, 6H), 3.62 (t, J = 5.1 Hz, 2H), 3.73-3.75 (m, 6H), 3.90 (t, J = 4.8 Hz, 2H), 4.22 (t, J = 4.8 Hz, 2H), 6.70 (d, J = 9.0 Hz, 2H), 6.99 (d, J = 8.7 Hz, 2H), 7.35 (d, J = 15.3 Hz, 1H), 7.55 (d, J = 9.0 Hz, 2H), 7.78 (d, J = 15.3 Hz, 1H), 8.02 (d, J = 9.0 Hz, 2H).

### Synthesis of (E)-1-(4-(2-(2-(2-fluoroethoxy)ethoxy)ethoxy)phenyl)-3-(4-(dimethylamino)phenyl)prop-2-en-1-one (7)

Compound 6 (200 mg, 0.50 mmol) was dissolved in ethylene glycol dimethyl ether (4 mL), and the mixture was stirred in an acetone bath with cooling. DAST (133 µL, 1.0 mmol) was slowly added while stirring with cooling, the temperature was returned to room temperature after 10 min, aqueous potassium carbonate was slowly added to terminate the reaction, the mixture was extracted with chloroform and dehydrated by adding Na₂SO₄, and then the solvent was evaporated under reduced pressure. The residue was purified by preparative TLC using ethyl acetate:n-hexane = 1:1 as a developing solvent to obtain 29 mg (yield rate 14.4%) of Compound 7. ¹H-NMR (CDCl₃) δ: 3.04 (s, 6H), 3.73-3.81 (m, 6H), 3.90 (t, J = 5.1 Hz, 2H), 4.21 (t, J = 5.1 Hz, 2H), 4.49 (t, J = 4.5 Hz, 1H), 4.65 (t, J = 4.5 Hz, 1H), 6.70 (d, J = 8.7 Hz, 2H), 6.98 (d, J = 9.0 Hz, 2H), 7.35 (d, J = 15.3 Hz, 1H), 7.55 (d, J = 8.7 Hz, 2H), 7.78 (d, J = 15.3 Hz, 1H), 8.02 (d, J = 9.0 Hz, 2H). EI-MS: m/z 401 (M⁺)

### Synthesis of 1-(4-(2-hydroxyethoxy)phenyl)ethanone (8)

4-Hydroxyacetophenone (1.36 g, 10 mmol) and ethylene chlorohydrin (660 µL, 10 mmol) were dissolved in DMF (5 mL), followed by addition of K₂CO₃ (4.14 g, 30 mmol), and the mixture was reacted at 100°C for 50 h. After 12 h, ethylene chlorohydrin (333 µL, 5 mmol) was further added. Purified water was added, the mixture was extracted with chloroform and dehydrated by adding N₂SO₄, and then the solvent was evaporated under reduced pressure. The residue was purified by silica gel column chromatography using ethyl acetate:n-hexane = 3:2 as an elution solvent to obtain 1.79 g (yield rate 99.4%) of Compound 8. ¹H-NMR (CDCl₃) δ: 2.75 (s, 3H), 4.20 (s, 2H), 4.35 (t, J = 5.1 Hz, 2H), 7.15 (d, J = 9 Hz, 2H), 8.13 (d, J = 9 Hz, 2H).

### Synthesis of 1-(4-(2-fluoroethoxy)phenyl)ethanone (9)

Compound 8 (1.62 g, 8.9 mmol) was dissolved in ethylene glycol dimethyl ether (4 mL), and the mixture was stirred in an acetone bath with cooling. DAST (2.3 mL, 17.8 mmol) was slowly added while stirring with cooling. Aqueous potassium carbonate was slowly added to terminate the reaction, the mixture was extracted with chloroform and dehydrated by adding Na₂SO₄ and then the solvent was evaporated under reduced pressure. The residue was purified by silica gel column chromatography using ethyl acetate:n-hexane = 1:1 as a developing solvent to obtain 1.02 g (yield rate 63.3%) of Compound 9. ¹H-NMR (CDCl₃) δ: 4.24 (d, t, J₁ = 28.2 Hz, J₂ = 4.2 Hz, 2H), 4.75 (d, t, J₁ = 47.1 Hz, J₂ = 3.9 Hz, 2H), 6.92 (d, J = 9 Hz, 2H), 7.89 (d, J = 9.3 Hz, 2H).

### Synthesis of (E)-1-(4-(2-fluoroethoxy)phenyl)-3-(4-nitrophenyl)prop-2-en-1-one (10)

Compound 9 (860 mg, 4.8 mmol) and 4-nitrobenzaldehyde (720 mg, 4.8 mmol) were dissolved in ethanol (7 mL). A small amount of 10% KOH was gradually added with stirring, and the mixture was stirred at room temperature for 1 h. The precipitated crystals were collected by suction filtration to obtain 856 mg (yield rate 56.6%) of Compound 1.
¹H-NMR (CDCl₃) δ: 4.32 (d, t, J₁ = 27.6 Hz, J₂ = 4.2 Hz, 2H), 4.81 (d, t, J₁ = 47.4 Hz, J₂ = 4.2 Hz, 2H), 7.04 (d, J = 8.7 Hz, 2H), 7.65 (d, J = 15.6 Hz, 1H), 7.79 (d, J = 8.7 Hz, 2H), 7.82 (d, J = 12.6 Hz, 1H), 8.06 (d, J = 9.0 Hz, 2H), 8.28 (d, J = 8.7 Hz, 2H).

### Synthesis of (E)-1-(4-(2-fluoroethoxy)phenyl)-3-(4-aminophenyl)prop-2-en-1-one (11)

Compound 10 (856 mg, 2.7 mmol) and SnCl₂ (2.55 g, 13.5 mmol) were dissolved in ethanol (10 mL), and the mixture was stirred at 100°C for 2.5 h. 1 N NaOH was added, the mixture was extracted with ethyl acetate and dehydrated by adding Na₂SO₄, and then the solvent was evaporated under reduced pressure. The residue was purified by silica gel column chromatography using chloroform as an elution solvent to obtain 333 mg (yield rate 43.0%) of Compound 11. ¹H-NMR (CDCl₃) δ: 4.02 (s, broad, 2H), 4.30 (d, t, J₁ = 27.6 Hz, J₂ = 4.2 Hz, 2H), 4.79 (d, t, J₁ = 47.4 Hz, J₂ = 4.2 Hz, 2H), 6.68 (d, J = 8.7 Hz, 2H), 7.00. (d, J = 8.7 Hz, 2H), 7.36 (d, J = 15.3 Hz, 1H), 7.48 (d, J = 8.4 Hz, 2H), 7.75 (d, J = 15.3 Hz, 1H), 8.03 (d, J = 6.9 Hz, 2H). EI-MS: m/z 285 (M⁺)

### Synthesis of (E)-1-(4-(2-fluoroethoxy)phenyl)-3-(4-aminophenyl)prop-2-en-1-one (12)

Compound 11 (290 mg, 1.02 mmol) was dissolved in DMSO (6 mL). K₂CO₃ (691 mg, 5.08 mmol) was added, the mixture was stirred, and CH₃I (0.18 mL, 3.05 mmol) was slowly added dropwise. The mixture was stirred at room temperature for 3 h, followed by addition of purified water, and the mixture was extracted with ethyl acetate. The mixture was dehydrated by adding Na₂SO₄, then the solvent was evaporated under reduced pressure, and the residue was purified by silica gel column chromatography using ethyl acetate:n-hexane = 1:2 as an elution solvent to obtain 90 mg (yield rate 29.5%) of Compound 12. ¹H-NMR (CDCl₃) δ: 2.89 (s, 3H), 4.23 (d, t, J₁ = 27.9 Hz, J₂ = 4.2 Hz, 2H), 4.79 (d, t, J₁ = 47.4 Hz, J₂ = 4.2 Hz, 2H), 6.59 (d, J = 8.7 Hz, 2H), 6.99 (d, J = 9.0 Hz, 2H), 7.34 (d, J = 15.3 Hz, 1H), 7.51 (d, J = 8.4 Hz, 2H), 7.78 (d, J = 15.3 Hz, 1H), 8.02 (d, J = 9.3 Hz, 2H). EI-MS: m/z 299 (M⁺)

### Synthesis of 1-(4-(2-(2-hydroxyethoxy)ethoxy)phenyl)ethanone (13)

4-Hydroxyacetophenone (1.36 g, 10 mmol) and ethylene glycol mono-2-chloroethyl ether (1.06 mL, 10 mmol) were dissolved in DMF (5 mL), followed by addition of K₂CO₃ (4.14 g, 30 mmol), and the mixture was reacted at 100°C for 18 h. Purified water was added, the mixture was extracted with chloroform and dehydrated by adding Na₂SO₄, and then the solvent was evaporated under reduced pressure to obtain Compound 13. ¹H-NMR (CDCl₃) δ: 2.56 (s, 3H), 3.68 (t, J = 4.8 Hz, 2H), 3.75-3.79 (m, 2H) 3.90 (t, J = 5.1 Hz, 2H), 4.21 (t, J = 4.8 Hz, 2H), 6.96 (d, J = 8.7 Hz, 2H), 7.94 (d, J = 8.7 Hz).

### Synthesis of 1-(4-(2-(2-fluoroethoxy)ethoxy)phenyl)ethanone (14)

The whole amount of the above-obtained Compound 13 was dissolved in ethylene glycol dimethyl ether (2 mL), and the mixture was stirred with ice cooling. DAST (1 mL, 7.5 mmol) was slowly added while stirring with cooling. DAST (1 mL, 7.5 mmol) was further added, aqueous potassium carbonate was slowly added to terminate the reaction after 18 h, the mixture was extracted with chloroform and dehydrated by adding Na₂SO₄, and then the solvent was evaporated under reduced pressure. The residue was purified by silica gel column chromatography using ethyl acetate:n-hexane = 1:1 as an elution solvent to obtain 568 mg (yield rate 25%) of Compound 14. ¹H-NMR (CDCl₃) δ: 2.56 (s, 3H), 3.78 (t, J = 3.3 Hz, 1H), 3.86-3.94 (m, 3H), 4.22 (t, J = 5.1 Hz, 2H), 4.51 (t, J = 3.0 Hz, 1H), 4.67 (t, J = 3.0. Hz, 1H), 6.96 (d, J = 8.7 Hz, 2H), 7.93 (d, J = 8.7 Hz, 2H). EI-MS: m/z 226 (M⁺)

### Synthesis of (E)-1-(4-(2-(2-fluoroethoxy)ethoxy)phenyl)-3-(4-nitrophenyl)prop-2-en-1-one (15)

Compound 14 (568 mg, 2.5 mmol) and 4-nitrobenzaldehyde (380 mg, 2.5 mmol) were dissolved in ethanol (5 mL). A small amount of 10% KOH was gradually added with stirring, and the mixture was stirred at room temperature for 1 h. The precipitated crystals were collected by suction filtration to obtain 128 mg (yield rate 14.2%) of Compound 15. ¹H-NMR (CDCl₃) δ: 3.79 (t, J = 4.2 Hz, 1H), 3.88-4.27 (m, 3H), 4.8 (t, J = 4.8 Hz, 2H), 4.53 (t, J = 4.2 Hz, 1H), 4.69 (t, J = 4.2 Hz, 1H), 7.03 (d, J = 8.7 Hz, 2H), 7.66 (d, J = 15.6 Hz, 1H), 7.79 (d, J = 9.0 Hz, 2H), 7.81 (d, J = 15.6 Hz, 1H), 8.05 (d, J = 8.7 Hz, 2H), 8.28 (d, J = 9.0 Hz, 2H).

### Synthesis of (E)-1-(4-(2-(2-fluoroethoxy)ethoxy)phenyl)-3-(4-aminophenyl)prop-2-en-1-one (16)

Compound 15 (128 mg, 0.36 mmol) and SnCl₂ (0.3 g, 1.78 mmol) were dissolved in ethanol (5 mL), and the mixture was stirred at 100°C for 30 min. 1 N NaOH was added, the mixture was extracted with ethyl acetate and dehydrated by adding Na₂SO₄ and then the solvent was evaporated under reduced pressure. The residue was purified by silica gel column chromatography using chloroform as an elution solvent to obtain 85 mg (yield rate 72.4%) of Compound 16. ¹H-NMR (CDCl₃) δ: 3.77-3.94 (m, 4H), 4.00 (s, broad, 2H), 4.23 (t, J = 4.5 Hz, 2H), 4.53 (t, J = 4.2 Hz, 1H), 4.69 (t, J = 4.2 Hz, 1H), 6.68 (d, J = 8.4 Hz, 2H), 6.99 (d, J = 8.7 Hz, 2H), 7.74 (d, J = 15.6 Hz, 1H), 7.48 (d, J = 8.4 Hz, 1H), 7.36 (d, J = 15.3 Hz, 1H), 8.01 (d, J = 9.0 Hz, 2H). EI-MS: m/z 329 (M⁺)

### Synthesis of (E)-1-(4-(2-(2-fluoroethoxy)ethoxy)phenyl)-3-(4-(methylamino)phenyl)prop-2-en-1-one (17)

Compound 16 (73 mg, 0.22 mmol) was dissolved in DMSO (4 mL). K₂CO₃ (152.0 mg, 1.1 mmol) was added, the mixture was stirred, and CH₃I (0.04 mL, 0.66 mmol) was slowly added dropwise. The mixture was stirred at room temperature for 3.5 h, followed by addition of purified water, and the mixture was extracted with ethyl acetate. The mixture was dehydrated by adding Na₂SO₄, then the solvent was evaporated under reduced pressure, and the residue was purified by preparative TLC using ethyl acetate:n-hexane = 1:1 as a developing solvent to obtain 22 mg (yield rate 29.1%) of Compound 17. ¹H-NMR (CDCl₃) δ: 2.90 (s, 3H), 3.78-3.95 (m, 4H), 3.99 (s, broad, 1H), 4.23 (t, J = 4.5 Hz, 2H), 4.53 (t, J = 4.5 Hz, 2H), 4.53 (t, J = 4.2 Hz, 1H), 4.69 (t, J = 4.2 Hz, 1H), 6.60 (d, J = 8.7 Hz, 2H), 6.99 (d, J = 8.7 Hz, 2H), 7.35 (d, J = 15.3 Hz, 1H), 7.51 (d, J = 8.7 Hz, 2H), 7.77 (d, J = 15.3 Hz, 1H), 8.02 (d, J = 8.7 Hz, 2H). EI-MS: m/z 343 (M⁺)

### Synthesis of 1-(4-(2-(2-(2-hydroxyethoxy)ethoxy)ethoxy)phenyl)ethanone (18)

4-Hydroxyacetophenone (1.36 g, 10 mmol) and 2-[2-(2-chloroethoxy)ethoxy]ethanol (1.45 mL, 10 mmol) were dissolved in DMF (3 mL), followed by addition of K₂CO₃ (4.14 g, 30 mmol), and the mixture was stirred at 100°C for 18 h. After 12 h, 2-[2-(2-chloroethoxy)ethoxy]ethanol (500 µL, 3.45 mmol) was further added, followed by addition of purified water, the mixture was extracted with chloroform and dehydrated by adding Na₂SO₄, and then the solvent was evaporated under reduced pressure to obtain Compound 18.

### Synthesis of 1-(4-(2-(2-(2-fluoroethoxy)ethoxy)ethoxy)phenyl)ethanone (19)

The whole amount of the above-obtained Compound 18 was dissolved in ethylene glycol dimethyl ether (4 mL), and the mixture was stirred with ice cooling. DAST (1 mL, 7.5 mmol) was slowly added while stirring with cooling. DAST (1.5 mL, 11.3 mmol) was further added, aqueous potassium carbonate was slowly added to terminate the reaction after 12 h, the mixture was extracted with chloroform and dehydrated by adding Na₂SO₄, and then the solvent was evaporated under reduced pressure. The residue was purified by silica gel column chromatography using ethyl acetate:n-hexane = 1:1 as an elution solvent to obtain 543 mg (yield rate 20%) of Compound 19. ¹H-NMR (CDCl₃) δ: 2.56 (s, 3H), 3.69-3.81 (m, 6H), 3.90 (t, J = 4.5 Hz, 2H), 4.21 (t, J = 5.1 Hz, 2H), 4.49 (t, J = 4.2 Hz, 1H), 4.65 (t, J = 4.2 Hz, 1H), 6.95 (d, J = 9.3 Hz, 2H), 7.92 (d, J = 9.0 Hz, 2H). EI-MS = m/z 270 (M⁺)

### Synthesis of (E)-1-(4-(2-(2-(2-fluoroethoxy)ethoxy)ethoxy)phenyl)-3-(4-nitrophenyl)prop-2-en-1-one (20)

Compound 19 (543 mg, 2.0 mmol) and 4-nitrobenzaldehyde (302 mg, 2.0 mmol) were dissolved in ethanol (5 mL). A small amount of 10% KOH was added in 5 drops with stirring, and the mixture was stirred at room temperature for 1 h. The precipitated crystals were collected by suction filtration to obtain 649 mg (yield rate 80.0%) of Compound 20. ¹H-NMR (CDCl₃) δ: 3.71-3.82 (m, 6H), 3.92 (t, J = 4.5 Hz, 2H), 4.24 (t, J = 4.8 Hz, 2H), 4.50 (t, J = 4.2 Hz, 1H), 4.66 (t, J = 4.5 Hz, 1H), 7.03 (d, J = 9.3 Hz, 2H), 7.66 (d, J = 15.6 Hz, 1H), 7.79 (d, J = 9.0 Hz, 2H), 7.81 (d, J = 15.6 Hz, 1H), 8.05 (d, J = 9.3 Hz, 2H), 8.28 (d, J = 8.7 Hz, 2H).

### Synthesis of (E)-1-(4-(2-(2-(2-fluoroethoxy)ethoxy)ethoxy)phenyl)-3-(4-aminophenyl)prop-2-en-1-one (21)

Compound 20 (649 mg, 1.60 mmol) and SnCl₂ (0.94 g, 8 mmol) were dissolved in ethanol (5 mL), and the mixture was stirred at 100°C for 30 min. 1 N NaOH was added, the mixture was extracted with ethyl acetate and dehydrated by adding Na₂SO₄, and then the solvent was evaporated under reduced pressure. The residue was purified by silica gel column chromatography using ethyl acetate:n-hexane = 2:1 as an elution solvent to obtain 206 mg (yield rate 34.5%) of Compound 21. ¹H-NMR (CDCl₃) δ: 3.70-3.83 (m, 6H), 3.89 (t, J = 4.5 Hz, 2H), 4.12 (s, broad, 2H), 4.21 (t, J = 4.8 Hz, 2H), 4.49 (t, J = 4.0 Hz, 1H), 4.65 (t, J = 3.9 Hz, 1H), 6.67 (d, J = 8.7 Hz, 2H), 6.98 (d, J = 8.7 Hz, 2H), 7.36 (d, J = 15.3 Hz, 1H), 7.47 (d, J = 8.4 Hz, 2H), 7.74 (d, J = 15.9 Hz, 1H), 8.01 (d, J = 9.0 Hz, 2H). EI-MS: m/z 373 (M⁺)

### Synthesis of (E)-1-(4-(2-(2-(2-fluoroethoxy)ethoxy)ethoxy)phenyl)-3-(4-methylamino)phenyl)prop-2-en-1-one (22)

Compound 21 (206 mg, 0.55 mmol) was dissolved in DMSO (6 mL). K₂CO₃ (380 mg, 2.75 mmol) was added, the mixture was stirred, and CH₃I (0.10 mL, 1.65 mmol) was slowly added dropwise. The mixture was stirred at room temperature for 2 h, followed by addition of purified water, and the mixture was extracted with ethyl acetate. The mixture was dehydrated by adding Na₂SO₄, then the solvent was evaporated under reduced pressure, and the residue was purified by silica gel column chromatography using ethyl acetate:n-hexane = 2:3 as an elution solvent to obtain 53 mg (yield rate 24.9%) of Compound 22. ¹H-NMR (CDCl₃) δ: 2.89 (s, 3H), 3.69-3.83 (m, 6H), 3.90 (t, J = 4.8 Hz, 2H), 4.12 (s, broad, 1H), 4.22 (t, J = 5.1 Hz, 2H), 4.49 (t, J = 4.2 Hz, 1H), 4.65 (t, J = 4.1 Hz, 1H), 6.60 (d, J = 8.7 Hz, 2H), 6.98 (d, J = 9.0 Hz, 2H), 7.35 (d, J = 15.3 Hz, 1H), 7.51 (d, J = 8.7 Hz, 2H), 7.76 (d, J = 15.3 Hz, 1H), 8.01 (d, J = 8.7 Hz, 2H). EI-MS: m/z 387 (M⁺)

### Synthesis of (E)-3-(4-(dimethylamino)phenyl)-1-(4-fluorophenyl)prop-2-en-1-one (23)

4-Fluoroacetophenone (283 mg, 2.1 mmol) and 4-dimethylaminobenzaldehyde (278 mg, 1.9 mmol) were dissolved in ethanol (10 mL). 10% KOH (5 mL) was gradually added with stirring, and the mixture was stirred at room temperature for 11 h. The precipitated crystals were washed with 50% aqueous ethanol to obtain 209 mg (yield rate 41.6%) of Compound 23. ¹H-NMR (CDCl₃) δ: 3.02 (s, 6H), 6.68 (d, J = 8.7 Hz, 2H), 7.15 (d, J = 9.0 Hz, 2H), 7.30 (d, J = 15.3 Hz, 1H), 7.54 (d, J = 9.0 Hz, 2H), 7.78 (d, J = 15.3 Hz, 1H), 8.02 (d, J = 8.7 Hz, 2H).

### Synthesis of (E)-1-(4-fluorophenyl)-3-(4-nitrophenyl)prop-2-en-1-one (24)

4-Fluoroacetophenone (305 mg, 2.2 mmol) and 4-nitrobenzaldehyde (301 mg, 2.0 mmol) were dissolved in ethanol (5 mL). 10% KOH (5 mL) was gradually added with stirring, and the mixture was stirred at room temperature for 13 h. The precipitated crystals were washed with 50% aqueous ethanol to obtain 490 mg (yield rate 90.7%) of Compound 24.

### Synthesis of (E)-3-(4-aminophenyl)-1-(4-fluorophenyl)prop-2-en-1-one (25)

Compound 24 (420 mg, 1.55 mmol) and SnCl₂ (863 mg) were dissolved in ethanol (5 mL), and the mixture was heated to reflux for 1 h. 1 N NaOH was added, the mixture was extracted with ethyl acetate and dehydrated by adding Na₂SO₄, and then the solvent was evaporated under reduced pressure. The residue was purified by silica gel column chromatography using ethyl acetate:n-hexane = 1:2 as an elution solvent to obtain 150 mg (yield rate 40.0%) of Compound 25. ¹H-NMR (CDCl₃) δ: 4.07 (s, 2H), 6.67 (d, J = 8.7 Hz, 2H), 6.98 (d, J = 8.7 Hz, 2H), 7.31 (d, J = 15.6 Hz, 1H), 7.47 (d, J = 8.4 Hz, 2H), 7.76 (d, J = 15.9 Hz, 1H), 8.03 (d, J = 9.0 Hz, 2H).

### Synthesis of (E)-1-(4-fluorophenyl)-3-(4-methylamino)phenyl)prop-2-en-1-one (26)

Compound 25 (70 mg) was dissolved in DMSO (2 mL). K₂CO₃ (120 mg) was added, the mixture was stirred, and CH₃I (140 mg) was slowly added dropwise. The mixture was stirred at room temperature for 3 h, followed by addition of purified water, and the mixture was extracted with ethyl acetate. The mixture was dehydrated by adding Na₂SO₄ then the solvent was evaporated under reduced pressure, and the residue was solvent purified by silica gel column chromatography using ethyl acetate:n-hexane = 1:1 as an elution to obtain 14 mg (yield rate 19%) of Compound 26. ¹H-NMR (CDCl₃) δ: 2.90 (s, 3H), 4, 20 (s, 1H), 6.59 (d, J = 8.7 Hz, 2H), 6.98 (d, J = 9.0 Hz, 2H), 7.35 (d, J = 15.3 Hz, 1H), 7.51 (d, J = 8.7 Hz, 2H), 7.77 (d, J = 15.3 Hz, 1H), 8.02 (d, J = 8.7 Hz, 2H).

### (2) Analyses of fluorine-containing chalcone derivatives by HPLC

The synthesized fluorine-containing chalcone derivatives were analyzed by reverse phase HPLC (column: COSMOSIL 5C18-AR-II (4.6 x 150 mm) manufactured by Nacalai Tesque Inc.; flow rate, 1.0 mL/min; mobile phase, a mixed solution of water and acetonitrile (1/1); detection by UV absorption at 254 nm).

### (3) Labeling of fluorine-containing chalcone derivatives with C-11

0.5 mg of Compound 26 or 22 was dissolved in methyl ethyl ketone (500 µL), and the mixture was reacted with [¹¹C]methyl triflate for 3 min. Then, the mixture was separated and purified by reverse phase HPLC to obtain target compounds, [¹¹C]Compounds 23 and 7. The radiochemical yields thereof were 35 and 28%, respectively, and it was confirmed that radiochemical purity was 95% or higher for both the compounds.

### (4) In-vitro binding inhibition experiment using Aβ(1-42) aggregate

The Aβ(1-42) aggregate was dissolved in a buffer containing 10 mM sodium phosphate and 1 mM EDTA (pH 7.4) at a concentration of 0.5 mg/mL, and the mixture was incubated at 37°C for 36 to 42 h. The binding inhibition experiment was performed using 12 x 75-mm borosilicate glass tubes. 850 µL of 10% ethanol solution, 50 µL of [¹²¹I]4-dimethylamino-4'-iodochalcone (in 10% aqueous ethanol), 50 µL (29 nM) of an Aβ(1-42) aggregate solution, and 50 µL of a solution of fluorine-containing chalcone derivatives in 10% aqueous ethanol prepared at various concentrations were mixed, and the mixture was allowed to stand at room temperature for 3 h. Further, nonspecific binding was calculated using 4-dimethylamino-4'-iodochalcone (400 nM), a nonradioactive compound. Aβ aggregate-bound chalcone derivatives and nonbound chalcone derivatives were separated with a Brandel M-24R cell harvester using Whatman GF/B filters. Radioactivities of substances remaining on the filter were measured with a γ counter. The 50% inhibitory concentrations were calculated using Graph Pad Prism (graph pad software), and inhibition constants (Kᵢ values) were calculated by Cheng-Prusoff equation, Kᵢ = IC50/(1+[L]/Kd). In this equation, the concentration of [¹²⁵I]4-dimethylamino-4'-iodochalcone used in the experiment was used for [L], and the dissociation constant of [¹²⁵I]4-dimethylamino-4'-iodochalcone against the Aβ(1-42) aggregate was used for Kd.

### (5) In-vivo radioactivity distribution experiment in normal mice

Labeled compounds, [¹¹C]Compounds 23 and 7, were diluted with physiological saline containing 10% ethanol. 100 µL of each of the labeled compounds was intravenously infused to five 5-week-old male ddY mice per group, the animals were decapitated at 2, 10, 30, or 60 min, blood was collected, and then weights and radioactivities thereof were measured.

### Experimental results

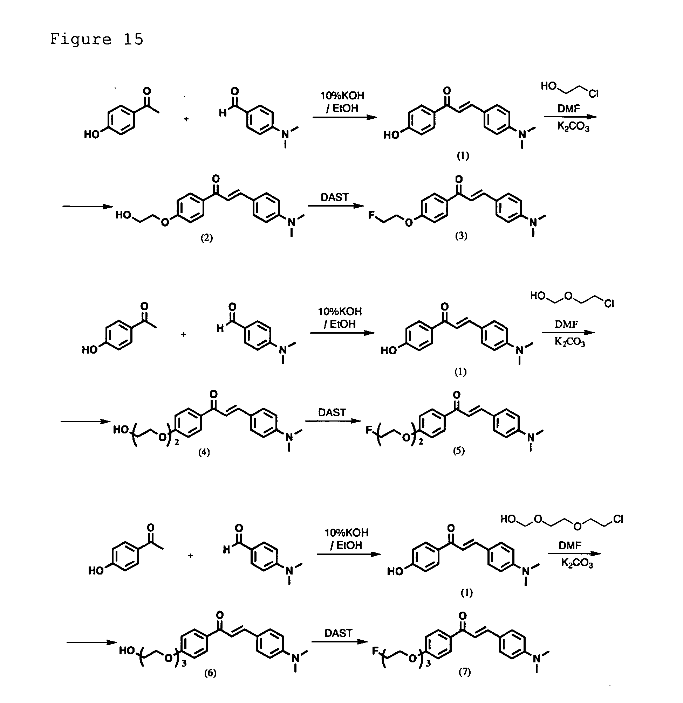

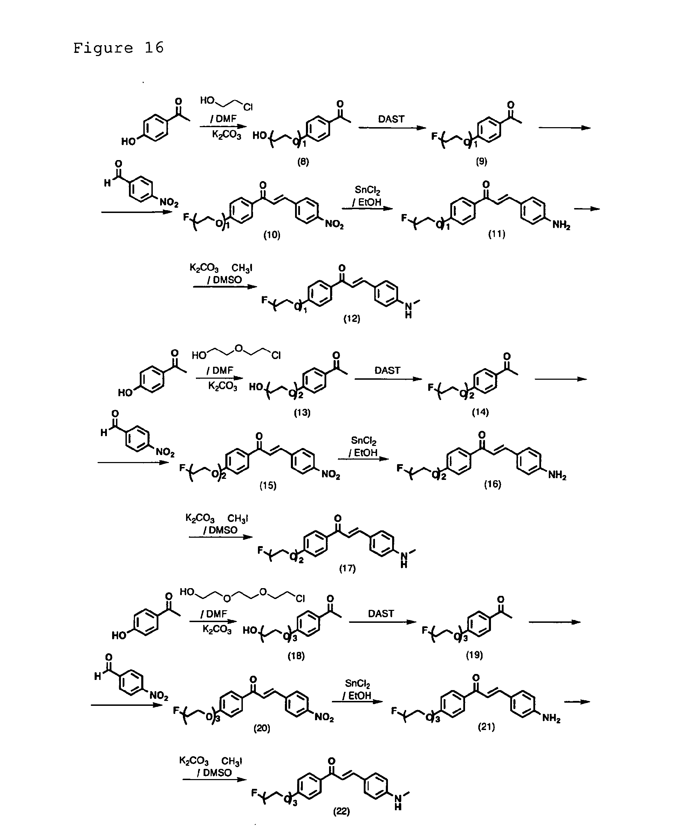

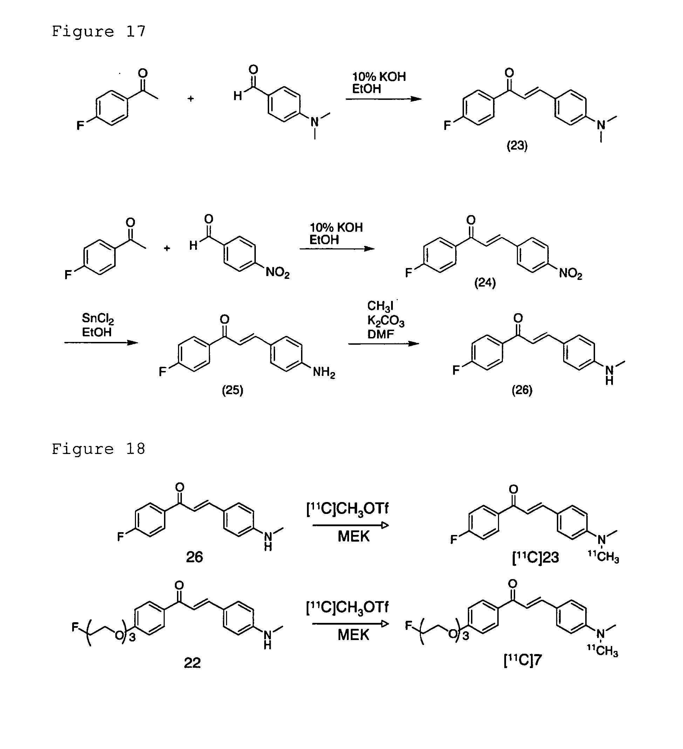

As shown in Figures 15, 16, and 17, fluorine-containing chalcone derivatives were synthesized by an aldol reaction. Compounds 26 and 22 were converted to [¹¹C]Compounds 23 and 7 by a reaction with [¹¹C]methyl triflate (Figure 18). The results of analyses of the synthesized fluorine-containing chalcone derivatives by reverse phase HPLC are shown in Table 16. Since the elution time was shortened by introduction of a fluoroethoxy group into the chalcone skeleton, it was suggested that the lipid solubility of the compounds was decreased. Furthermore, it was demonstrated that the lipid solubility of the compounds was decreased with the increase in the number of ethylene oxy groups.

To examine the binding property of fluorine-containing chalcone derivatives to amyloid, *in-vitro* inhibition experiment was performed using the Ab42 aggregate (Table 17). The results suggested that all the chalcone derivatives have a binding property to Aβ since they inhibited binding of [¹²⁵I]DAC to Aβ. No marked difference in the binding property to Aβ was observed that is dependent on the number of ethylene oxy groups introduced into the phenyl-Ring B. However, differences of the binding property depending on the types of substituents introduced into the phenyl-Ring A were observed, and the binding property improved in the order of NH₂ < NH(CH₃) < N(CH₃)₂ irrespective of the number of ethylene oxy groups.

To evaluate brain penetration and clearance of the fluorine-containing chalcone derivatives, in-vivo radioactivity distribution experiment was performed in normal mice (Table 18). As a result, [¹¹C]Compounds 23 and 7 showed high penetration into the brain at an early stage after administration (3.68, 4.31% ID/g at 2 min after administration) and rapid radioactivity clearance (1.04, 0.35% ID/g at 60 min after administration). Since the ratio of the radioactivity remaining in the brain at 30 min after administration to that at 2 min after administration was 28.3% for [¹¹C]Compound 23 and 14.8% for [¹¹C]Compound 7, and the ratio of the radioactivity remaining in the brain at 60 min after administration to that at 60 min after administration was 28.3% for [¹¹C]Compound 23 and 8.1% for [¹¹C]Compound 7, it was revealed that [¹¹C]Compound 7, into which three ethylene oxy groups were introduced, showed a favorable radioactive behavior in the brain of normal mice as compared with [¹¹C]Compound 23.

**[Table 16]**

| Compound | Retention time in HPLC (min) |
|---|---|
| 25 | 5. 23 |
| 26 | 8.92 |
| 23 | 24. 6 |
| 11 | 4.95 |
| 12 | 8.54 |
| 3 | 17.3 |
| 16 | 4.71 |
| 17 | 8.13 |
| 5 | 16. 2 |
| 21 | 4.44 |
| 22 | 7.76 |
| 7 | 15.3 |

**[Table 17]**

| Compound | Inhibition constant (Ki, nM) (mean±SE) |
|---|---|
| 25 | 617 ± 22 |
| 26 | 204 ±11 |
| 23 | 48 ± 2 |
| 11 | 553 ± 22 |
| 12 | 193 ± 15 |
| 3 | 29 ± 2 |
| 16 | 1003 ± 29 |
| 17 | 187 ± 3 |
| 5 | 19 ± 1 |
| 21 | 785 ± 33 |
| 22 | 353 ± 25 |
| 7 | 38 ± 1 |

**[Table 18]**

| | Radioactivity concentration in the brain of mice (% ID/g) | |
|---|---|---|
| Time after administration (min) | [¹¹C] 23 | [¹¹C]7 |
| 2 | 3. 68 ± 0.35 | 4.31 ± 0.33 |
| 10 | 1.53 ± 0.14 | 1.38 ± 0.16 |
| 30 | 1.04 ± 0.15 | 0.64 ± 0.07 |
| 60 | 1. 04 ± 0.20 | 0.35 ± 0.03 |

The contents in the specifications and/or the drawings of Japanese patent applications (Japanese Patent Application Nos. 2006-144024 and 2007-081637) on which the conventional priority of the present application is based are included in the scope of the present specification. Furthermore, all the publications, patents, and patent applications cited in the present invention are incorporated into the present specification.

## Claims

1. A composition for diagnosis of an amyloid-related disease, comprising a compound represented by general formula (I): 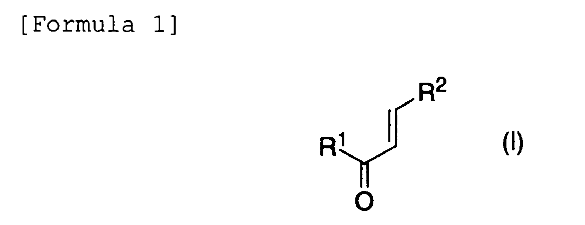 wherein R¹ represents an aromatic heterocyclic ring that may be substituted with one or more substituents selected from the following substituent group A, R² represents an aryl group that may be substituted with one or more substituents selected from the following substituent group A, or an aromatic heterocyclic ring that may be substituted with one or more substituents selected from the following substituent group A, and the substituent group A is a group consisting of a halogen atom, a hydroxyl group, a carboxyl group, a sulfone group, a dimethylamino group, a methylamino group, an amino group, a nitro group, an alkyl group having 1 to 4 carbon atoms that may be substituted with a halogen atom, an alkoxy group having 1 to 4 carbon atoms that may be substituted with a halogen atom, an alkoxy group having 2 to 8 carbon atoms that may be substituted with a halogen atom, an alkoxy group having 3 to 12 carbon atoms that may be substituted with a halogen atom, and a group represented by the formula: -(CH₂CH₂O)ₙ-F, wherein n is an integer of 4 to 10; or the compound labeled with a radionuclide, or a pharmaceutically acceptable salt thereof.

2. The composition for diagnosis of an amyloid-related disease according to claim 1, wherein R¹ in the general formula (I) represents a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or a pyridin-2-yl group.

3. The composition for diagnosis of an amyloid-related disease according to claim 1 or 2, wherein R² in the general formula (I) represents a 4-aminophenyl group, a 4-methylaminophenyl group, a 4-dimethylaminophenyl group, a 4-hydroxyphenyl group, a 4-methoxyphenyl group, a 6-iodopyridin-3-yl group, a 6-fluoropyridin-3-yl group, a 6-hydroxypyridin-3-yl group, a 5-iodopyridin-2-yl group, a 5-fluoropyridin-2-yl group, a 5-hydroxypyridin-2-yl group, a 5-iodopyridin-3-yl group, a 5-fluoropyridin-3-yl group, a 5-hydroxypyridin-3-yl group, a 4-iodopyridin-2-yl group, a 4-fluoropyridin-2-yl group, a 4-hydroxypyridin-2-yl group, a 5-iodothiophen-2-yl group, a 5-fluorothiophen-2-yl group, a 5-hydroxythiophen-2-yl group, a 5-iodofuran-2-yl group, a 5-fluorofuran-2-yl group, a 5-hydroxyfuran-2-yl group, a 5-aminothiophen-2-yl group, a 5 methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, a 5-{2-[2-(2-fluoroethoxy)ethoxy]ethoxy}furan-2-yl group, a pyridin-4-yl group, a pyridin-3-yl group, or a pyridin-2-yl group.

4. The composition for diagnosis of an amyloid-related disease according to claim 1, wherein R¹ in the general formula (I) represents a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group, and R² in the general formula (I) represents a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a 4-aminophenyl group, a 4-methylaminophenyl group, a 4-dimethylaminophenyl group, a 4-hydroxyphenyl group, a 4-methoxyphenyl group, a pyridin-4-yl group, a pyridin-3-yl group, or a pyridin-2-yl group.

5. The composition for diagnosis of an amyloid-related disease according to claim 1, wherein R¹ in the general formula (I) represents a 5-aminothiophen-2-yl group, a 5-methylaminothiophen-2-yl group, a 5-dimethylaminothiophen-2-yl group, a 5-aminofuran-2-yl group, a 5-methylaminofuran-2-yl group, a 5-dimethylaminofuran-2-yl group, a thiazol-2-yl group, a 1H-imidazol-5-yl group, a 1H-imidazol-2-yl group, a pyridin-4-yl group, a pyridin-3-yl group, or a pyridin-2-yl group, and R² in the general formula (I) represents a 6-iodopyridin-3-yl group, a 6-fluoropyridin-3-yl group, a 6-(2-fluoroethyl)pyridin-3-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-iodothiophen-2-yl group, a 5-fluorothiophen-2-yl group, a 5-(2-fluoroethyl)thiophen-2-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-iodofuran-2-yl group, a 5-fluorofuran-2-yl group, a 5-(2-fluoroethyl)furan-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group.

6. The composition for diagnosis of an amyloid-related disease according to claim 1, wherein R¹ in the general formula (I) represents a 6-hydroxypyridin-3-yl group, a 5-hydroxythiophen-2-yl group, a 5-hydroxyfuran-2-yl group, a 6-(2-fluoroethoxy)pyridin-3-yl group, a 6-[2-(2-fluoroethoxy)ethoxy]pyridin-3-yl group, a 6-{2-[2-(2-fluoroethoxy)ethoxy]ethoxy}pyridin-3-yl group, a 5-(2-fluoroethoxy)thiophen-2-yl group, a 5-[2-(2-fluoroethoxy)ethoxy]thiophen-2-yl group, a 5-{2-[2-(2-fluoroethoxy)ethoxy]ethoxy}thiophen-2-yl group, a 5-(2-fluoroethoxy)furan-2-yl group, a 5-[2-(2-fluoroethoxy)ethoxy]furan-2-yl group, or a 5-{2-[2-(2-fluoroethoxy)ethoxy]ethoxy}furan-2-yl group, and R² in the general formula (I) represents a 5-methylaminothiophen-2-yl group, a 5-dimethylaminothiophen-2-yl group, a 5-methylaminofuran-2-yl group, a 5-dimethylaminofuran-2-yl group, a 4-methylaminophenyl group, or a 4-dimethylaminophenyl group.

7. The composition for diagnosis of an amyloid-related disease according to claim 1, wherein R¹ in the general formula (I) represents a 5-methylaminothiophen-2-yl group, a 5-dimethylaminothiophen-2-yl group, a 5-methylaminofuran-2-yl group, or a 5-dimethylaminofuran-2-yl group, and R² in the general formula (I) represents a 6-hydroxypyridin-3-yl group, a 5-hydroxythiophen-2-yl group, or a 5-hydroxyfuran-2-yl group.

8. The composition for diagnosis of an amyloid-related disease according to claim 1, wherein R¹ in the general formula (I) represents a thienyl group in which one hydrogen atom is substituted with a halogen atom, and R² in the general formula (I) represents a phenyl group in which one hydrogen atom is substituted with a dimethylamino group or a methylamino group.

9. The composition for diagnosis of an amyloid-related disease according to claim 1, wherein R¹ in the general formula (I) represents a 5-iodothiophen-2-yl group, and R² in the general formula (I) represents a 4-dimethylaminophenyl group or a 4-methylaminophenyl group.

10. The composition for diagnosis of an amyloid-related disease according to any one of claims 1 to 9, wherein the radionuclide is a positron-emitting radionuclide.

11. The composition for diagnosis of an amyloid-related disease according to any one of claims 1 to 9, wherein the radionuclide is a γ-ray-emitting radionuclide.

12. The composition for diagnosis of an amyloid-related disease according to any one of claims 1 to 11, wherein the amyloid-related disease is Alzheimer's disease.

13. A method for screening for a therapeutic or prophylactic agent for an amyloid-related disease, comprising the steps of administering a test substance to an amyloid-related disease model animal, administering the composition for diagnosis of an amyloid-related disease according to any one of claims 1 to 12 to the model animal, and examining a distribution or a quantity of the compound represented by the general formula (I) contained in the brain of the model animal.

14. A method for evaluating a therapeutic or prophylactic agent for an amyloid-related disease, comprising the steps of administering the therapeutic or prophylactic agent for an amyloid-related disease to an amyloid-related disease model animal, administering the composition for diagnosis of an amyloid-related disease according to any one of claims 1 to 12 to the model animal, and examining a distribution or a quantity of the compound represented by the general formula (I) contained in the brain of the model animal.
